(19) Europäisches Patentamt
European Patent Office
Office européen des brevets

(11) Numéro de publication: **0 292 352**
**A2**

(12) **DEMANDE DE BREVET EUROPEEN**

(21) Numéro de dépôt: **88401106.5**

(22) Date de dépôt: **05.05.88**

(51) Int. Cl.[4]: **C 07 H 17/08**
A 61 K 31/70

(30) Priorité: **06.05.87 FR 8706361**
**07.01.88 FR 8800078**
**29.01.88 FR 8801031**

(43) Date de publication de la demande:
**23.11.88 Bulletin 88/47**

(84) Etats contractants désignés:
**AT BE CH DE ES FR GB GR IT LI LU NL SE**

(71) Demandeur: **ADIR ET COMPAGNIE**
**22, rue Garnier**
**F-92200 Neuilly-sur-Seine (FR)**

(72) Inventeur: **Lukacs, Gabor**
**22 Boulevard Kellermann**
**F-75013 Paris (FR)**

**Duchatelle-Ruggeri, Catherine**
**1 Place Jacques Froment**
**F-75018 Paris (FR)**

Revendications pour les Etats contractants suivants: ES + GR.

(54) **Nouveaux dérivés macrolides, leur procédé de préparation et les compositions pharmaceutiques les contenant.**

(57) Procédé de préparation de composés de formule générale
:

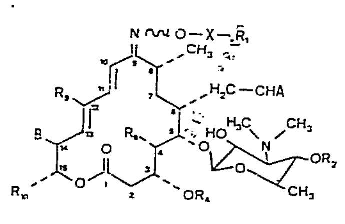

dans laquelle :
- A représente soit un atome d'oxygène,
soit un groupement de formule N ∾ O - Y - $R_5$ ∾ , B, X, Y, $R_1$, $R_2$, $R_3$, $R_4$, $R_5$, $R_8$, $R_9$, et $R_{10}$ étant définis dans la description.

EP 0 292 352 A2

## Description

**"NOUVEAUX DERIVES MACROLIDES, LEUR PROCEDE DE PREPARATION ET LES COMPOSITIONS PHARMACEUTIQUES LES CONTENANT"**

La présente invention concerne de nouveaux antibiotiques de la famille des macrolides, leur procédé de préparation et les compositions pharmaceutiques qui les contiennent.

Les besoins de la thérapeutique exigent le développement constant de nouveaux antibiotiques, à la fois en raison de la possibilité d'apparition de nouvelles souches résistantes, mais également dans le but de créer de nouvelles molécules possédant une activité améliorée aussi bien au niveau de leur seuil d'efficacité que de l'étendue de leur spectre d'action.

De nombreuses modifications du noyau des macrolides ont déjà été réalisées afin de produire de nouveaux antibiotiques intéressants. Parmi les plus récentes, citons les brevets US 4 528 369, 4 581 346 , 4 629 786 et les demandes de brevets européens 0 103 465, 0 104 028, 0 154 495 et 0 203 621.

Plus particulièrement, la présente invention a pour objet les dérivés macrolides de formule générale :

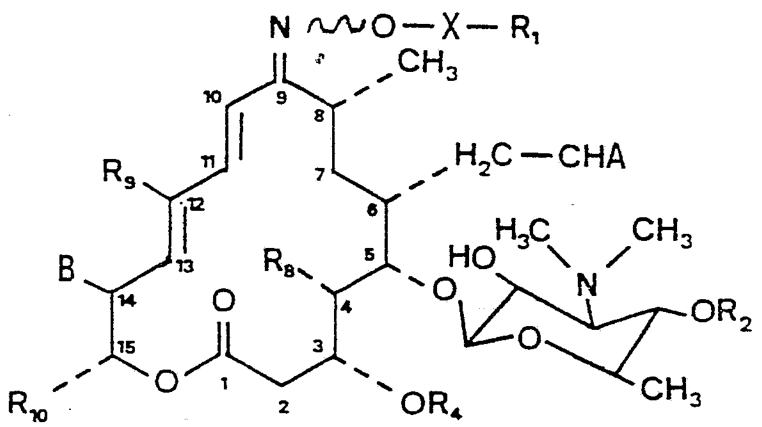

(I)

dans laquelle :

- A représente . soit un atome d'oxygène,

. soit un groupement de formule N ∿ O - Y - $R_5$, le signe ∿ existant sur le substituant du carbone 9 ainsi que dans la définition de A signifiant que l'une ou l'autre indépendamment des fonctions oxime ou éther d'oxime peut chacune se trouver sous la forme syn ou anti ou sous forme du mélange syn et anti,

- X et Y identiques ou différents représentent soit un radical alkyle comprenant au plus dix atomes de carbone, linéaire ou ramifié, soit un radical alkényle comprenant au plus dix atomes de carbone, linéaire ou ramifié, soit un radical alkynyle comprenant au plus dix atomes de carbone, linéaire ou ramifié, chacun des substituants alkyle, alkényle ou alkynyle pouvant être éventuellement substitué par un ou plusieurs groupements choisis parmi hydroxy, alkyle inférieur linéaire ou ramifié, alkényle inférieur linéaire ou ramifié, alkynyle inférieur linéaire ou ramifié, alkényloxy inférieur linéaire ou ramifié, alkénylthio inférieur linéaire ou ramifié, alkynyloxy inférieur linéaire ou ramifié, alkynylthio inférieur linéaire ou ramifié, fluoro, chloro, bromo, iodo, amino, dialkylamino inférieur,

- $R_1$ et $R_5$ identiques ou différents représentent :

. soit un atome d'hydrogène,

. soit un radical alkyloxy linéaire ou ramifié comprenant au plus dix atomes de carbone, un radical alkylthio linéaire ou ramifié comprenant au plus dix atomes de carbone, un radical alkényloxy linéaire ou ramifié comprenant au plus dix atomes de carbone, un radical alkénylthio linéaire ou ramifié comprenant au plus dix atomes de carbone, un radical alkynyloxy linéaire ou ramifié comprenant au plus dix atomes de carbone, un radical alkynylthio linéaire ou ramifié comprenant au plus dix atomes de carbone, un radical aryle (citons à titre d'exemple phényle, pyridyle, thiényle, furyle, benzothiényle, benzofuryle, indolyle, thiazolyle, oxazolyle, pyrrolyle, imidazolyle, pyrazolyle, isothiazolyle, isoxazolyle, benzimidazolyle), un radical aryloxy, arylthio, aralkyloxy, aralkylthio, chacun des radicaux alkyloxy, alkylthio, alkényloxy, alkénylthio, alkynyloxy, alkynylthio, aryle, aryloxy, arylthio, aralkyloxy, aralcoylthio étant éventuellement substitué par un ou plusieurs des radicaux choisis parmi hydroxy, alkyle inférieur, alkényle inférieur, alkynyle inférieur, alkyloxy inférieur, alkényloxy inférieur, alkynyloxy inférieur, alkylthio inférieur, alkénylthio inférieur, alkynylthio inférieur, fluoro, chloro, bromo, iodo, nitro, amino, dialkylamino inférieur, ou un radical N $R_6$ $R_7$ ci-dessous défini,

. soit un radical

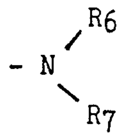

dans lequel $R_6$ et $R_7$ identiques ou différents représentent un atome d'hydrogène, un radical alkyle inférieur, ou alkényle inférieur ou alkynyle inférieur, ou forment avec l'azote un système hétérocyclique saturé ou non, comprenant éventuellement un autre hétéroatome et éventuellement substitué par un atome d'halogène ou un radical alkyle inférieur,

- un atome de chlore, de brome, d'iode ou de fluor,
- un groupement carboxylique libre ou salifié par une base minérale (KOH, NaOH, $Ca(OH)_2$) ou organique (triéthylamine, diéthylamine...) ou estérifié par un alcool aliphatique de formule $R_8OH$ où $R_8$ est un groupement alkyle inférieur,
- ou X - $R_1$ et Y - $R_5$ indépendamment l'un de l'autre représentent chacun un atome d'hydrogène,
- $R_2$ représente . soit un atome d'hydrogène,

. soit un radical de formule :

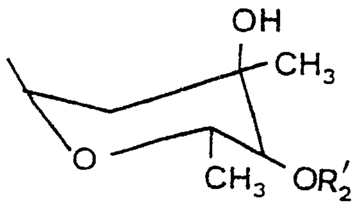

dans lequel $R'_2$ représente un atome d'hydrogène, un radical alkyle inférieur linéaire ou ramifié ou un radical acyle inférieur, linéaire ou ramifié,

- B représente . soit un atome d'hydrogène,

. soit un radical de formule : - $CH_2$ - O - B', où B' représente :

. soit un atome d'hydrogène,

. soit un radical de formule :

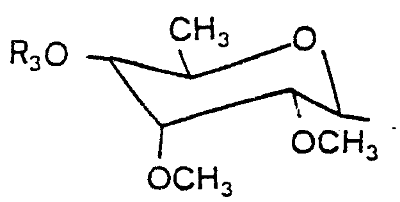

dans lequel $R_3$ représente un atome d'hydrogène ou un radical alkyle inférieur linéaire ou ramifié,

- $R_4$ représente . soit un atome d'hydrogène,

. soit un radical alkyle inférieur linéaire ou ramifié,

. soit un radical acyle inférieur linéaire ou ramifié,

- $R_8$ représente . soit un radical alkyloxy inférieur préférentiellement méthoxy lorsque B représente un atome hydrogène,

. soit un radical alkyl inférieur préférentiellement méthyle lorsque B représente un groupement - $CH_2$ - O - B',

- $R_9$ représente . soit un atome d'hydrogène lorsque B représente un atome d'hydrogène,

. soit un radical alkyle inférieur préférentiellement méthyle lorsque B représente un radical - $CH_2$ - O - B',

- $R_{10}$ représente un radical alkyle inférieur,

par radicaux alkyle inférieur, alkyloxy inférieur, alkylthio inférieur, alkényle inférieur, alkényloxy inférieur, alkénylthio inférieur, alkynyle inférieur, acyle inférieur, alkynyloxy inférieur, alkynylthio inférieur, on entend des groupements comprenant entre 1 et 6 atomes de carbone.

L'invention s'étend également aux sels des composés de formule (I). Parmi les acides que l'on peut ajouter aux composés de formule (I) pour former un sel d'addition, on peut citer, à titre d'exemple, les acides chlorhydrique, bromhydrique, iodhydrique, sulfurique, acétique, propionique, trifluoroacétique, maléique, malique, tartrique, méthanesulfonique, éthanesulfonique, benzènesulfonique, p-toluènesulfonique, phosphorique, fumarique, citrique, camphorique...

Plus particulièrement, la présente invention concerne les dérivés dans lesquels :

1/ - A est un atome d'oxygène,
- X est un radical alkyle inférieur linéaire ou ramifié,
- $R_1$ est . un atome d'hydrogène,
. un radical alkyle,
. un radical aryle éventuellement substitué par un groupement nitro,
. un radical alkényloxy
. un radical alkyloxy éventuellement substitué par un radical alkyloxy inférieur,
. un radical dialkylamino,
. un hétérocycle azoté comprenant éventuellement un autre hétéroatome,
. un radical aryloxy au aralkyle,
ou X - $R_1$ représente un atome d'hydrogène,
- B, $R_2$, $R_4$, $R_8$, $R_9$ et $R_{10}$ ont les mêmes valeurs que celles énoncées précédemment.

2/ - A est un radical de formule N ∿ O - Y - $R_5$ dans lequel le signe ∿ a la même signification que celle énoncée précédemment et,
- B, $R_1$, $R_2$, $R_3$, $R_4$, $R_8$, $R_9$, $R_{10}$ et X ont les valeurs indiquées en 1/,
- Y est un radical alkyle inférieur linéaire ou ramifié et,
- $R_5$ est . un atome d'hydrogène,
. un radical alcoyle,
. un radical alcoyloxy éventuellement substitué par un radical alcoyloxy inférieur,
ou Y - $R_5$ représente un atome d'hydrogène,
ou X - $R_1$ et Y - $R_5$ représentent chacun simultanément un atome d'hydrogène.

Des dérivés particulièrement intéressants de la présente invention sont ceux pour lesquels A, X, Y, $R_1$ et $R_5$ ont les valeurs mentionnées en 1/ et 2/ et pour lesquels,
d'une part B représente un atome d'hydrogène, tandis que $R_8$ représente un groupement méthoxy, $R_9$ un atome d'hydrogène et $R_{10}$ un groupement méthyle,
ou d'autre part B représente un groupement :

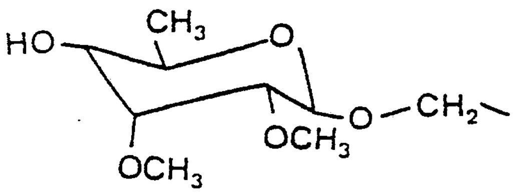

tandis que $R_8$ et $R_9$ représentent simultanément un groupement méthyle et $R_{10}$ un groupement éthyle.

La présente invention s'étend également à un procédé de préparation des dérivés de formule (I) caractérisé en ce que l'on utilise comme matière première,
- soit, lorsque B représente un atome d'hydrogène, un dérivé de formule (II/1),

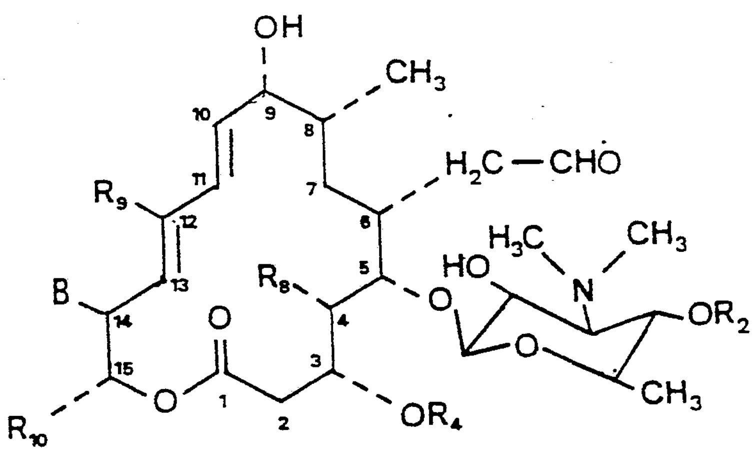

(II/1)

dans laquelle $R_2$, $R_4$, $R_8$ et $R_{10}$ ont la même définition que dans la formule (I), B et $R_9$ représentant simultanément chacun un atome d'hydrogène,
que l'on soumet à un agent d'oxydation comme par exemple le complexe trioxyde de soufre-pyridine en

présence d'un agent basique comme une amine organique, en milieu organique, préférentiellement choisi parmi diméthylsulfoxide, diméthylformamide, pyridine,
pour obtenir un dérivé de formule (II/1a) :

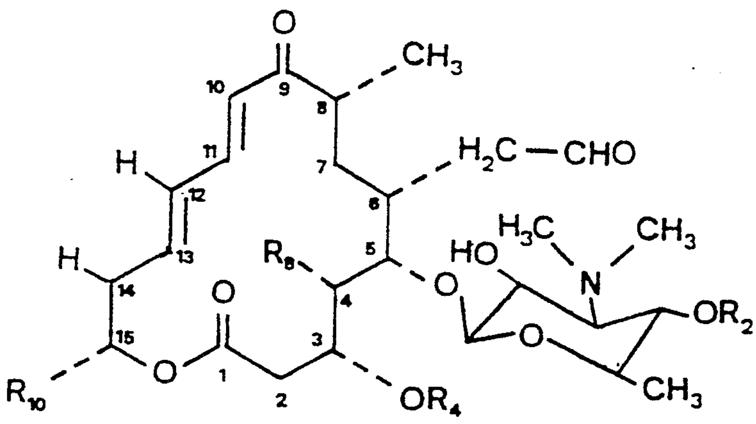

(II/1a)

dans laquelle $R_2$, $R_4$, $R_8$ et $R_{10}$ ont la même signification que dans la formule (I),
- soit, lorsque dans le composé de formule (I) que l'on souhaite obtenir B et $R_9$ ne représentent pas simultanément chacun un atome d'hydrogène,
un dérivé de formule (II/2) :

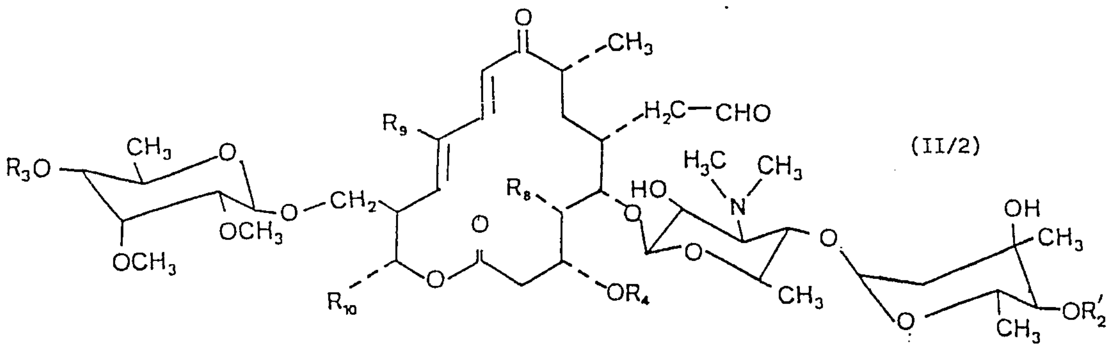

(II/2)

dans laquelle $R'_2$, $R_3$, $R_4$ et $R_{10}$ ont la même définition que dans la formule (I), et $R_8$ et $R_9$ représentent un radical alkyle inférieur préférentiellement méthyle,
qui, lorsque dans le composé de formule (I) que l'on désire obtenir B' et $R_2$ représentent simultanément un atome d'hydrogène,
est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

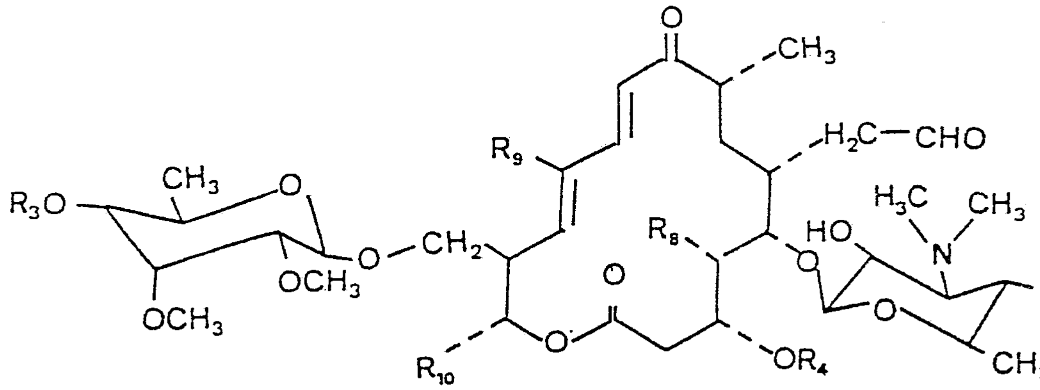

(II/2a)

dans laquelle $R_3$, $R_4$ et $R_{10}$ ont la même signification que dans la formule (I), et $R_8$ et $R_9$ la même signification que dans la formule (II/2),
que l'on fait réagir avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

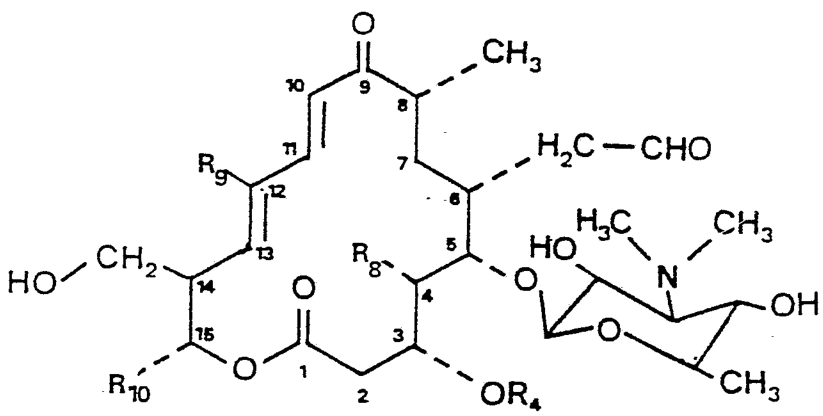

(II/2b)

dans laquelle $R_4$ et $R_{10}$ ont la même signification que dans la formule (I), et $R_8$ et $R_9$ la même signification que dans la formule (II/2), que l'on purifie éventuellement par chromatographie sur colonne de silice,
le dérivé alors choisi de formule générale (II/1a), ou (II/2) ou (II/2b) selon le composé de formule (I) que l'on souhaite obtenir étant alors condensé avec un alcool de formule Z - OH, dans lequel Z signifie un groupement alkyle inférieur, en présence d'un agent catalyseur acide comme l'acide paratoluène sulfonique, ou préférentiellement l'acide difluoroacétique lorsque le composé de formule (I) que l'on souhaite obtenir a un radical $R_2$ différent de H,
le milieu réactionnel étant ensuite soumis à l'action d'un agent basique, en particulier une amine comme la triéthylamine, pour obtenir, après extraction par un solvant organique, préférentiellement choisi parmi éther diéthylique, chloroforme, chlorure de méthylène et purification par chromatographie sur colonne de silice, un dérivé de formule (III) :

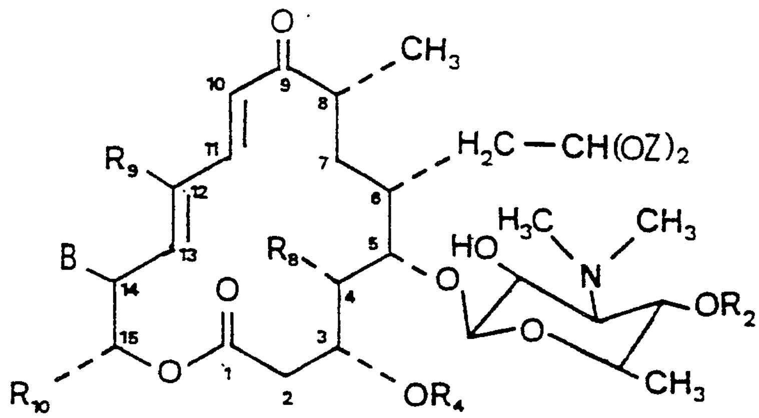

(III)

dans lequel B, $R_2$, $R_4$, $R_8$, $R_9$ et $R_{10}$ ont la même signification que dans la formule du dérivé choisi (II/1a), (II/2) ou (II/2b) et Z la même définition que précédemment,
que l'on condense en présence d'une base, comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N - O - X - R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V) :

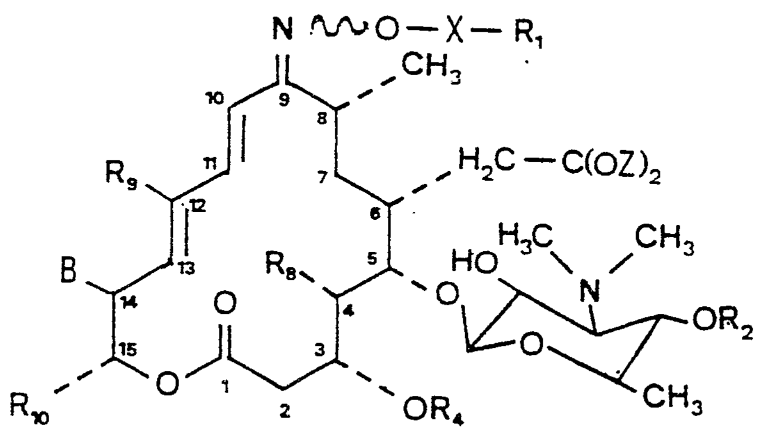

(V)

dans lequel le signe ∿∿ , X, $R_1$, $R_2$ ,$R_4$ ,$R_8$ , $R_9$ , $R_{10}$ et B ont la même signification que dans la formule (III) et Z la même définition que précédemment,

- soit avec un sel d'acide fort de l'hydroxylamine pour obtenir un dérivé de formule (V/a) :

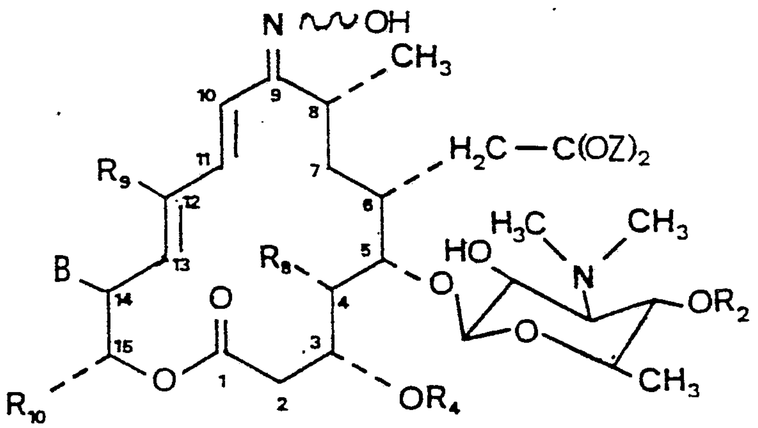

(V/a)

cas particulier de dérivés de formule (V), dans lequel le signe ∿ , B, $R_2$, $R_4$, $R_8$, $R_9$, $R_{10}$ et Z ont la même signification que dans la formule (V), X - $R_1$ signifiant ici un atome d'hydrogène,
que l'on traite le cas échéant en présence d'une base, comme par exemple la triéthylamine, ou la pyridine, ou un sel de métal alcalin comme le carbonate de sodium, ou le carbonate de potassium, ou le carbonate de calcium, ou l'hydrogéno carbonate de sodium, ou l'hydrogéno carbonate de potassium, ou un hydrure de métal alcalin tel que l'hydrure de sodium, dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V), dans lequel le signe ∿ , X, $R_1$, $R_2$, $R_4$, $R_8$, $R_9$, $R_{10}$ et B ont la même signification que dans la formule (I) et Z la même signification que dans la formule (III),
qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,1 N et 0,5 N, préférentiellement comprise entre 0,02 N et 0,2 N, préférentiellement comprise entre 0,05 N et 0,5 N, préférentiellement comprise 0,75 N et 0,25 N et à température ambiante, ou préférentiellement l'acide difluoroacétique en solution dans un mélange de solvants tel que le mélange acétonitrile - eau (1/1, v/v) lorsque le composé de formule (I) que l'on désire obtenir possède un radical $R_2$ différent de l'atome d'hydrogène,
pour obtenir un composé de formule (I/a) :

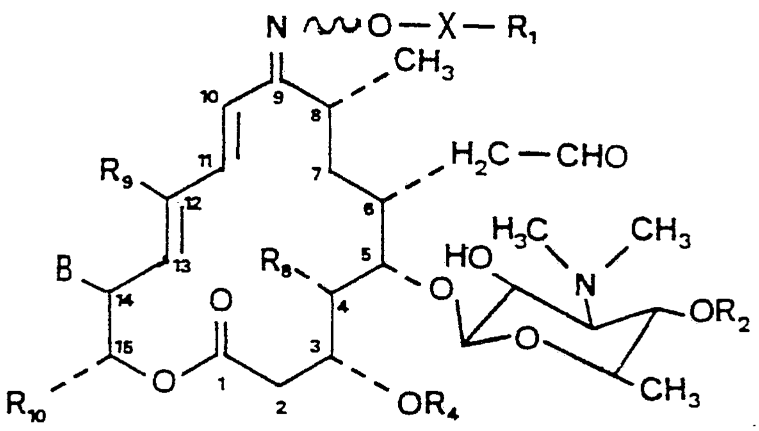

(I/a)

cas particulier des composés de formule (I), dans laquelle le signe ∿ X, $R_1$, $R_2$, $R_4$, $R_8$, $R_9$, $R_{10}$ et B ont la même définition que dans la formule (I), A représentant ici un atome d'oxygène,
lequel, dans le cas où dans la matière première utilisée $R_2$ ne signifie pas un atome d'hydrogène, peut au cours du processus de synthèse, en fonction des conditions opératoires sélectionnées ainsi que des valeurs des groupements X, $R_1$, $R_8$, $R_9$, $R_{10}$ et B, avoir été partiellement hydrolysé en un dérivé (I/a) démycarosylé pour lequel $R_2$ = H, les deux dérivés ainsi obtenus (c'est-à-dire celui pour lequel $R_2$ = H et celui pour lequel $R_2$ correspond à un résidu mycarosylé) étant alors aisément séparés par une technique classique de séparation

comme la chromatographie sur colonne de silice,

ce dérivé de formule (I/a) pouvant alors, le cas échéant, être traité en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium

- soit par un dérivé de formule (VII) :

**$H_2N-O-Y-R_5$ (VII)**

dans laquelle Y et $R_5$ ont la même définition que dans la formule (I), à l'exclusion du cas où $Y-R_5$ représente un atome d'hydrogène, ou par un sel d'acide fort d'un tel produit, pour obtenir après purification éventuelle par chromatographie sur colonne de silice un dérivé de formule (I),

- soit par un sel d'acide fort de l'hydroxylamine pour obtenir un produit de formule (I/b),

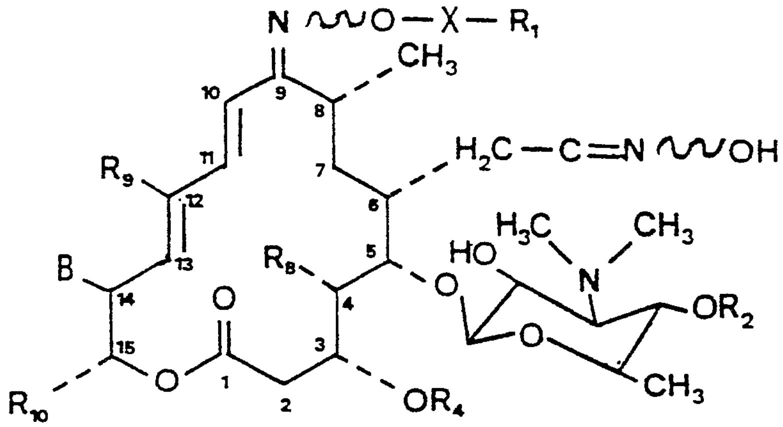

(I/b)

cas particulier de dérivés de formule (I) dans lequel le signe ∿ , X, $R_1$, $R_2$, $R_4$, $R_8$, $R_9$, $R_{10}$ et B ont la même signification que dans la formule (I), A représentant ici un groupement N ∿ $O-Y-R_5$ dans lequel $Y-R_5$ représente un atome d'hydrogène,

qui est traité le cas échéant, en présence d'une base, comme par exemple la triéthylamine, ou la pyridine, ou d'un sel de métal alcalin comme le carbonate de sodium ou de carbonate de potassium, ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5-Y-T'$ (VIII)**

dans laquelle T' représente un atome d'halogène et $R_5$ et Y ont la même signification que dans la formule (I), à l'exclusion du cas où $R_5-Y$ représentent ensemble un atome d'hydrogène, pour obtenir un composé de formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

Les dérivés de formule (V) sont nouveaux et font partie de l'invention au même titre que les dérivés de formule (I) dont ils constituent les intermédiaires de synthèse.

Un cas particulier de la présente invention concerne les dérivés de formule (I) dans lesquels A représente un groupement de formule :

N ∿ $O-Y-R_5$, $Y-R_5$ ayant la même signification que $X-R_1$.

De tels dérivés peuvent être obtenus par une variante simplifiée du procédé énoncé ci-dessus caractérisé en ce que l'on condense un dérivé de formule (II/1a) ou (II/2) ou (II/2b) selon le composé de formule (I) que l'on souhaite obtenir :

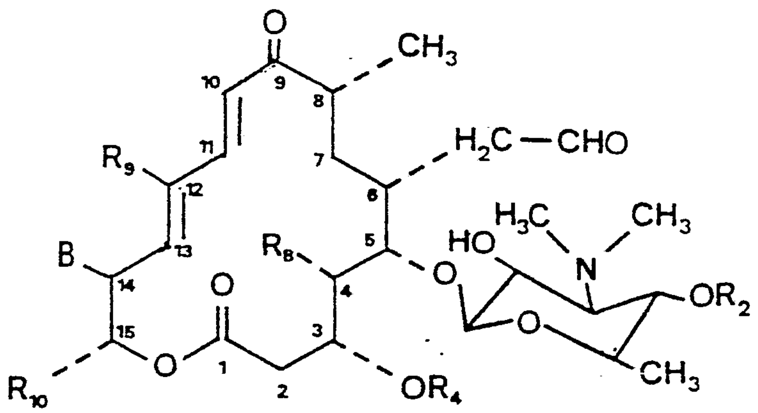

dans laquelle $R_2$, $R_4$, $R_8$, $R_9$, $R_{10}$ et B ont la même définition que dans celle indiqué précédemment pour chacune des formules (II/1) ou (II/2) ou (II/2b), en présence d'une base comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N$ - O - X - $R_1$ (IV)**

dans lequel X - $R_1$ a la même signification que dans la formule (I) à l'exception du cas où X - $R_1$ représente un atome d'hydrogène,

pour obtenir un produit de formule (IX) :

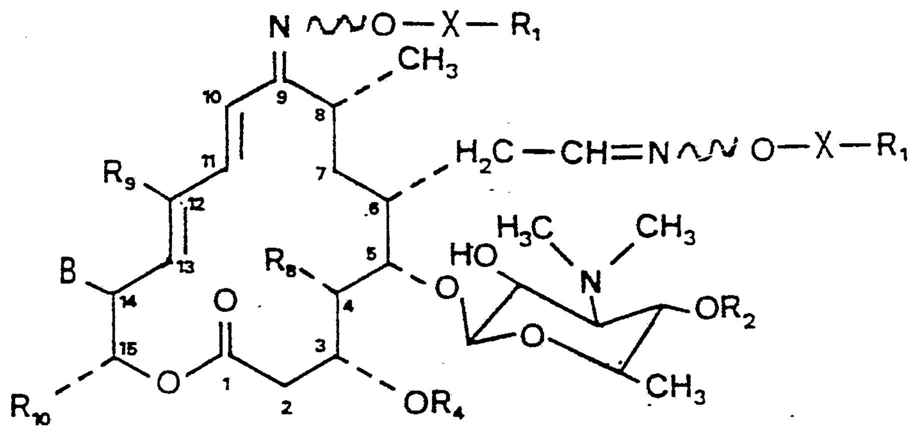

(IX)

cas particulier des dérivés de formule (I) dans laquelle le signe ∿ X, $R_1$, $R_2$, $R_4$, $R_8$, $R_9$, $R_{10}$ et B ont la même signification que dans la formule (I),

- soit avec un sel d'acide fort de l'hydroxylamine pour obtenir un dérivé de formule (IX/a) :

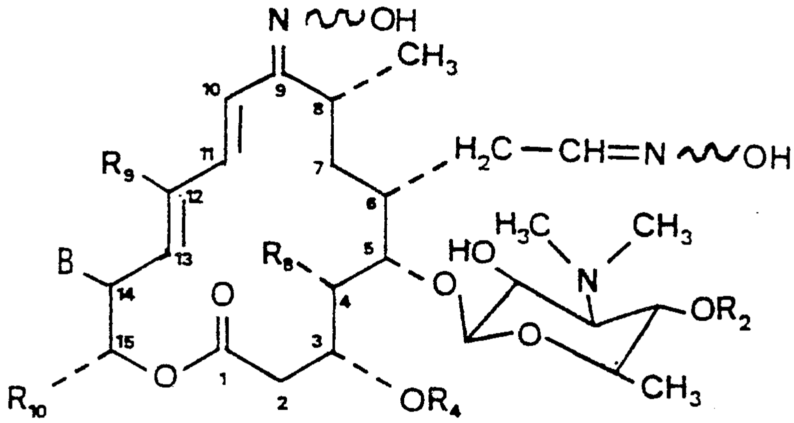

(IX/a)

cas particulier des dérivés de formule (I) et (IX), dans lequel le signe ∿∿ , $R_2$, $R_4$, $R_8$, $R_9$, $R_{10}$ et B ont la même signification que dans la formule (I), X - $R_1$ et Y - $R_5$ représentant ici ensemble un atome d'hydrogène, que l'on traite éventuellement en présence d'une base, comme par exemple la triéthylamine, la pyridine ou un sel de métal alcalin comme le carbonate de sodium ou le carbonate de potassium, ou l'hydrogéno carbonate de sodium ou l'hydrogéno carbonate de potassium, ou un hydrure de métal alcalin tel que l'hydrure de sodium, dans un solvant préférentiellement choisi parmi l'acétone ou le diméthyl formamide, le dioxanne, l'acétonitrile, le tétrahydrofuranne, l'éther diéthylique ou diisopropylique, par un dérivé de formule (X) :

**$R_1$ - X - T″ (X)**

dans lequel T″ représente un atome d'halogène et $R_1$ et X ont la même signification que dans la formule (I), à l'exclusion du cas où $R_1$ et X forment ensemble un atome d'hydrogène, pour obtenir un produit de formule (IX) cas particulier des dérivés de formule (I), dans lequel le signe ∿∿ $R_1$, $R_2$, $R_4$, $R_8$, $R_9$, $R_{10}$, B et X ont la même signification que dans la formule (I), que l'on purifie éventuellement par chromatographie sur colonne de silice à l'aide d'un mélange solvant approprié comme par exemple un mélange chlorure de méthylène / méthanol, et que l'on peut, si on le désire :

- soit salifier par un acide pharmaceutiquement acceptable,
- soit séparer en ses isomères puis, si nécessaire, salifier par un acide pharmaceutiquement acceptable.

Les composés de formule (I) possèdent des propriétés pharmacologiques intéressantes.

En particulier, ces composés sont actifs sur les cocci gram + et cocci gram -, les bacilles gram + (clostridies), certains bacilles gram -, hémophilus (ex : hémophilus influenzae), nesseiria gonorrhoeae, brucella, bordetella, les anaérobies, les mycoplasmes, les rickettsies et les myagawanelles (chlamidia), les spirochètes, les protozoaires et certains dermofongi.

Plus particulièrement, les composés de formule (I) possèdent une très bonne activité antibiotique sur les pneumocoques, les staphylocoques et les streptocoques. Ce spectre d'activité rend les composés de formule (I) particulièrement intéressants dans le traitement d'un grand nombre d'affections ; parmi celles-ci, il est possible de citer, à titre d'exemple, les pneumococcies telles que les bronchites, la brucellose, la diphtérie, la gonococcie, les pneumonies, les streptococcies telles que les angines aiguës, les otites, la scarlatine, les sinusites, les staphylococcies telles que septicémies à staphylocoques, anthrax, érésipèles, pyrodermites, staphylococcies aiguës, broncho pneumonies et suppurations pulmonaires.

De plus, les composés de la présente invention, de par leur structure, sont susceptibles de se révéler intéressants en raison de leur absence de toxicité hépatique ou gastrointestinale, ce qui les distingue avantageusement d'autres familles de composés antibiotiques.

La présente invention a également pour objet les compositions pharmaceutiques contenant les produits de formule (I) où un de leurs sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire, et notamment les préparations injectables ou buvables, les aérosols, les gouttes oculaires ou nasales, les comprimés simples ou dragéifiés, les comprimés sublinguaux, les sachets, les paquets, les gélules, les glossettes, les tablettes, les suppositoires, les crèmes, pommades, gels dermiques, etc...

Les compositions pharmaceutiques selon l'invention peuvent également se présenter sous forme d'une poudre lyophilisée qui est destinée à être dissoute au moment de l'emploi dans un solvant approprié notamment l'eau stérile apyrogène.

La posologie utile varie selon l'âge et le poids du patient, la voie d'administration, la nature de l'indication thérapeutique et des traitements éventuellement associés et s'échelonne entre 1 centigramme et 4 grammes par prise ou par application.

Les exemples suivants illustrent l'invention et ne la limitent en aucune façon.

Les spectres de résonance magnétique nucléaire du $^{13}C$ et du $^{1}H$ ont été enregistrés en utilisant le TMS comme référence interne.

Les matières premières utilisées dans la synthèse des composés de formule (I) sont la tylosine, ou la josamycine, connues dans la littérature.

**EXEMPLE 1 : OXIME (E + Z) - 9 DE LA DEMYCAROSYL TYLOSINE**

**STADE A :** Diéthylacétal - 20 de la démycarosyl tylosine

Dissoudre 907 mg (0,001 mmol) de tylosine base dans l'éthanol anhydre (8,3 ml) puis ajouter à température ambiante 260 mg (1,5 mmol) d'acide p-toluènesulfonique anhydre. Agiter pendant 2 heures puis ajouter 0,2 ml de triéthylamine. L'agitation est maintenue pendant 10 minutes supplémentaires puis la moitié du solvant est évaporée sous pression réduite. Extraire au dichlorométhane puis laver par une solution saturée de bicarbonate de sodium (2 x 30 ml) et d'eau (2 x 30 ml). La phase organique est séchée sur sulfate de sodium puis évaporée à sec. On obtient un résidu brut (1,150 g) qui est chromatographié (flash chromatographie) en utilisant le système $CH_2Cl_2$ : MeOH = 100 : 4. Le diéthylacétal - 20 de la démycarosyl tylosine (750 mg, 88 %) ainsi obtenu apparaît chromatographiquement pur.

Caractéristiques spectrales :

RMN $^{13}C$ 203,6 ppm : C (C = 0) (carbone 9)

Spectrométrie de masse : $[M - H]^+$ : M/Z : 846

**STADE B :** Diéthylacétal - 20 de l'oxime (E + Z) - 9 de la démycarosyl tylosine

Dissoudre 950 mg (1,12 mmol) du diéthylacétal - 20 de la démycarosyl tylosine, obtenu au stade A, dans 30 ml de pyridine anhydre. Ajouter 330 mg (4,74 mmol) de chlorhydrate d'hydroxylamine et porter le mélange à 80° C pendant 4 heures. Diluer dans l'eau puis extraire au dichlorométhane, laver et sécher comme indiqué au stade précédent. Le résidu brut (1,28 g) obtenu est purifié par flash chromatographie ($CH_2Cl_2$ : MeOH = 100 : 3) ce qui permet de séparer le produit attendu stéréochimiquement pur d'un mélange de ses deux isomères (Z) et (E).

Caractéristiques spectrales :

RMN $^{13}C$ (produit stéréochimiquement pur) 161,0 ppm : C (C = N) (carbone 9)

Spectrométrie de masse : $[M - H]^+$ : M/Z : 861

**STADE C :** Oxime (E + Z) - 9 de la démycarosyl tylosine

Dissoudre 600 mg (0,07 mmol) du diéthylacétal - 20 oxime (E + Z) - 9 de la démycarosyl tylosine stéréochimiquement pur, obtenu précédemment, dans 40 ml d'acétonitrile. Ajouter 40 ml d'une solution aqueuse d'acide chlorhydrique 0,1 N et agiter 4 heures à température ambiante. Ajouter ensuite 20 ml de solution de bicarbonate de sodium à 5 %, extraire au dichlorométhane, laver et sécher comme indiqué au Stade A. Le produit obtenu est pur.

Si on utilise comme produit de départ un mélange d'isomères (Z) et (E) du diéthylacétal - 20 oxime - 9 de la démycarosyl tylosine, on obtient un mélange d'isomères (Z) et (E) de l'oxime - 9 de la démycarosyl tylosine.

Caractéristiques spectrales :

RMN $^{13}C$ 159,8 ppm et 156,7 ppm : signaux caractéristiques du carbone 9 pour chacun des deux isomères (Z) et (E).

Spectrométrie de masse : $[M - H]^+$ : M/Z : 787

Point de fusion : 136 - 137° C

**EXEMPLE 2 : DIOXIME (E + Z) - 9, 20 DE LA TYLOSINE**

Dissoudre 1,8 g (1,96 mmol) de tylosine base dans 35 ml de pyridine anhydre puis ajouter 1,35 g (19 mmol) de chlorhydrate d'hydroxylamine. Agiter pendant 4 heures en maintenant une température de 80° C. Diluer le milieu réactionnel par l'eau, extraire au dichlorométhane puis laver par une solution saturée de bicarbonate de sodium (2 x 30 ml) puis par l'eau (2 x 30 ml). La phase organique est séchée sur sulfate de sodium puis évaporée à sec. On obtient un résidu brut (1,6 g) qui est purifié par chromatographie sur colonne de silice en utilisant un mélange acétate d'éthyle / méthanol (95/5) (Rf = 0,6).

Caractéristiques spectrales :

RMN $^{13}C$ 176,1 ppm ; 171,5 ppm et 160,3 ppm : pics caractéristiques des carbones 1,9,20

Spectrométrie de masse : $[M-H]^+$ : M/Z : 946

Point de fusion : 140 - 148 °C

**EXEMPLE 3 : DIOXIME (E + Z) - 9, 20 DE LA DEMYCAROSYL TYLOSINE**

En opérant comme dans l'exemple 2 , on obtient en fait, un mélange de la dioxime (E + Z) - 9, 20 de la tylosine et de la dioxime (E + Z) - 9, 20 de la démycarosyl tylosine, la séparation de ces deux composés étant réalisée par chromatographie sur colonne par un mélange acétate d'éthyle / méthanol 95/5 ; la dioxime (E + Z) - 9, 20 de la démycarosyl tylosine a un Rf. de 0,3.

Caractéristiques spectrales :

RMN $^{13}C$ 147,9 ; 171,0 et 159,5 ppm pics caractéristiques des carbones 1,9,20

Spectrométrie de masse : $[M-H]^+$ : M/Z : 802

Point de fusion : 128 - 134 °C

**EXEMPLE 4 : O - METHYLOXIME (E + Z) - 9 DE LA DEMYCAROSYL TYLOSINE**

**STADE A :** O - méthyloxime (E + Z) - 9 du diéthylacétal - 20 de la démycarosyl tylosine.

Dissoudre 950 mg (1,12 mmol) du diéthylacétal - 20 de la démycarosyl tylosine obtenu dans l'exemple 1 Stade A dans 30 ml de pyridine anhydre. Ajouter 235 mg (2,80 mmol) de chlorhydrate de méthoxylamine et agiter sous atmosphère d'azote à température ambiante pendant 48 heures. Ajouter un volume d'eau glacée. Agiter quelques minutes, extraire au dichlorométhane puis laver par une solution saturée de bicarbonate de sodium (2 x 30 ml) puis par l'eau (2 x 30 ml). La phase organique est séchée sur sulfate de sodium puis évaporée à sec. On obtient un résidu brut qui est purifié par chromatographie sur colonne de silice en utilisant le système $CH_2Cl_2$ / $CH_3$ OH 90/10. Le produit obtenu est utilisé tel quel dans le stade suivant.

**STADE B :** O - Méthyloxime (E + Z) - 9 de la démycarosyl tylosine

Procéder comme dans l'exemple 1, Stade C en remplaçant l'oxime (E + Z) - 9 diéthylacétal - 20 de la démycarosyl tylosine par l'O méthyloxime (E + Z) - 9 diéthylacétal - 20 de la démycarosyl tylosine.

Caractéristiques spectrales :

RMN $^{13}C$ 159,83 et 156,28 ppm signaux caractéristiques du carbone 9 pour chacun des isomères (Z) et (E).

Spectrométrie de masse : $[M-H]^+$ : M/Z : 801

**EXEMPLE 5 : O BENZYLOXIME (E + Z) - 9 DE LA DEMYCAROSYL TYLOSINE**

**STADE A :** O benzyloxime (E + Z) - 9 diéthylacétal - 20 de la démycarosyl tylosine

Dissoudre 2,31 g (2,73 mmol) du diéthylacétal - 20 de la démycarosyl tylosine obtenu dans l'exemple 1, Stade A, dans 40 ml de pyridine anhydre. Ajouter 1,7 g (10,92 mmol) de chlorhydrate de benzyloxyamine et agiter sous atmosphère d'azote à température ambiante pendant 96 heures en surveillant l'évolution de la

réaction par chromatographie sur couche mince (mélange solvant $CH_2Cl_2$ / MeOH : 9/1 v/v). Après disparition du produit de départ, extraire au dichlorométhane, laver par une solution saturée de bicarbonate de sodium (2 x 30 ml) puis par l'eau (2 x 30 ml). Les phases organiques sont réunies, séchées sur sulfate de sodium puis évaporées à sec. On obtient un résidu qui est purifié par chromatographie sur colonne de silice en utilisant un mélange dichlorométhane / méthanol (100 : 3) puis des quantités croissantes de méthanol.

**STADE B :** O benzyloxime (E + Z) - 9 de la démycarosyl tylosine

En opérant comme dans l'exemple 1, Stade C mais en remplaçant l'oxime (E + Z) - 9 diéthylacétal - 20 de la démycarosyl tylosine par l' O benzyloxime (E + Z) - 9 diéthylacétal - 20 de la démycarosyl tylosine obtenue au stade précédent, on obtient la O benzyloxime (E + Z) - 9 de la démycarosyl tylosine.

Caractéristiques spectrales :

Résonance magnétique nucléaire RMN $^1H$, 400 MHz δ = 7,35 à 7,50 ppm massif $^5H$, aromatiques.

Spectrométrie de masse : $[M - H]^+$ : M/Z : 877

**EXEMPLE 6 : O BENZYLOXIME (E + Z) - 9 DE LA TYLOSINE**

**STADE A :** Diméthylacétal en 20 de la tylosine

Dissoudre 6,0 g (65 mmol) de tylosine base dans 60 ml de méthanol anhydre puis ajouter 4,1 ml (6,5 mmol) d'acide difluoroacétique. Agiter à température ambiante pendant 120 heures. Ajouter 9 ml (65 mmol de triéthylamine et agiter pendant 1 heure à température ambiante. Evaporer à sec le méthanol. Ajouter 150 ml de dichlorométhane puis laver à l'eau. La phase organique est séchée sur sulfate de sodium puis évaporée à sec. On obtient un résidu brut qui est chromatographié (flash chromatographie) en utilisant le système $CH_2Cl_2$ : MeOH : $NH_4OH$ (20 : 1 : 0,05). Le diméthylacétal - 20 de la tylosine (2,6 g, 41,6 %) ainsi obtenu apparait chromatographiquement pur.

**STADE B :** Diméthyalcétal - 20 de l'O - benzyloxime de la tylosine

Dissoudre 2,6 (2,7 mmol) de diméthylacétal - 20 tylosine obtenu au stade A dans 20 ml de pyridine anhydre. Ajouter 1,68 g (10,8 mmol) de chlorhydrate de benzyloxyamine et agiter à température ambiante pendant 72 heures. Ajouter un volume d'eau glacée. Agiter quelques minutes, extraire au dichlorométhane ( 3 x 50 ml). La phase organique est évaporée à sec. On obtient un résidu brut qui est purifié par chromatographie comme indiqué au stade A. On obtient 1,95 g de produit chromatographiquement pur.

Rendement : 70 %

**STADE C :** O - benzyloxime de la tylosine

Dissoudre 1,5 g (1,4 mmol) du diméthylacétal - 20 de l'O -benzyloxime de la tylosine obtenu précédemment dans 100 ml d'un mélange acétronitrilé eau (1 - 1). Ajouter 0,46 ml (7 mmol) d'acide difluoroacétique et agiter à température ambiante pendant 37 heures. Ajouter 0,95 ml de triéthylamine et agiter pendant 1 heure. Evaporer l'acétonitrile et extraire au dichlorométhane ( 3 x 80 ml). La phase organique est séchée sur sulfate de sodium puis évaporée à sec. On obtient un résidu brut (3 g) qui est purifié par chromatographie comme indiqué au stade A. On obtient l'O - benzyloxime de la tylosine pure.

Rendement : 30 %

Si on utilise comme produit de départ un mélange d'isomères (Z) et (E) du diméthylacétal - 20 de la tylosine, on obtient un mélange d'isomères (Z) et (E) de l'oxime O - benzyl de la tylosine.

Caractéristiques spectrales :

RMN $^1H$ 200 MHz δ (ppm) δ = 2,45 ppm, singulet, 6H (2 x $CH_3$) : N $(CH_3)_2$
δ = 5,00 ppm, singulet, 2H, $CH_2$ (O - $CH_2$ -$C_6H_5$)
δ = 7,20 ppm, massif, 5H, aromatiques
δ = 9,40 ppm, massif, dédoublable, 1H, aldéhydique

Spectrométrie de masse : $[M - H]^+$ : M/Z : 1021

**EXEMPLE 7 : O [(METHOXY - 2 ETHOXY) - METHYL] OXYIMINO (E + Z) - 9 DESOXY - 9 TYLOSINE**

**STADE A :** Diméthylacétal en 20 de l'oxime (E + Z) - 9 de la tylosine

Dissoudre 2,0 g (2,08 mmol) de diméthylacétal - 20 de la tylosine obtenu dans l'exemple 6, stade A, dans 5 ml de pyridine anhydre. Ajouter 0,433 g (2,08 mmol) de chlorhydrate d'hydroxylamine et agiter à température ambiante pendant 12 heures. Ajouter un volume d'eau glacée. Agiter quelques minutes et extraire à l'acétate d'éthyle (3 x 50 ml). La phase organique est séchée sur sulfate de sodium puis évaporée à sec. On obtient 1,6 g (80 %) de produit dont la pureté est controlée par chromatographie sur colonne de silice en utilisant un mélange $CH_2Cl_2$ : MeOH : $NH_4OH$ = 10 : 1 : 0,05.

**STADE B :** Diméthylacétal - 20 de l'O [(méthoxy - 2 éthoxy) -methyl] oxyimino (E + Z) - 9 désoxy - 9 tylosine

Dissoudre 0,51 g (0,52 mmol) de diméthylacétal - 20 de l'oxime (E + Z) - 9 de la tylosine obtenu au stade A dans 10 ml de tétrahydrofuranne anhydre. Ajouter 27 mg d'une suspension à 50 % dans l'huile d'hydrure de sodium (0,57 mmol) et agiter à température ambiante pendant 15 minutes. Ajouter 64 µl (0,62 mmol) de chlorure de méthoxy-éthoxy méthyle et agiter à température ambiante pendant 30 minutes. Après addition de 50 mg de trisilicate de magnésium, évaporer à sec le tétrahydrofuranne. Ajouter 200 ml de dichlorométhane et filtrer. La phase organique est évaporée à sec et purifiée par flash chromatographie ($CH_2Cl_2$ : MeOH, $NH_4OH$ - 20 : 1 : 0,05). On obtient l'O [(méthoxy - 2 éthoxy) - methyl] oxyimino (E + Z) - 9 désoxy - 9 diméthylacétal - 20 de la tylosine chromatographiquement pur.

Rendement : 55 %

**STADE C :** O [(méthoxy - 2 éthoxy) - méthyl] oxyimino (E + Z) - 9 désoxy - 9 tylosine

Dissoudre 0,28 g (2,6 mmol) de diméthylacétal en 20 de l'O [(méthoxy - 2 éthoxy) - méthyl] oxyimino (E + Z) - 9 désoxy - 9 de la tylosine obtenu précédemment dans 20 ml d'un mélange solvant acétronitrile : eau (1 : 1). Ajouter 82 µl (13 mmol) d'acide trifluoroacétique et agiter 37 heures à température ambiante. Ajouter 50 µl (2,6 mmol) de triéthylamine et agiter pendant 1 heure. Evaporer l'acétonitrile et extraire au dichlorométhane (3 x 30 ml). La phase organique est séchée sur sulfate de sodium puis évaporée à sec. On obtient un résidu brut qui est purifié et séparé du dérivé démycarosylé par flash chromatographie comme indiqué au stade B. On obtient l'O [(méthoxy - 2 éthoxy) - méthyl] oxyimino (E + Z) -9 désoxy - 9 tylosine.

Rendement : 28%

Caractéristiques spectrales :

RMN $^1H$ 200 MHz δ(ppm) δ = 2,50 ppm, singulet, 6H(2 x $CH_3$) : N - $(CH_3)_2$
δ = 3,36 ppm, singulet, 3H ($CH_3$) ($OCH_2$ - O $(CH_2)_2$ - $OCH_3$)
δ = 9,70 ppm, singulet, 1H, (CH = O)

Spectrométrie de masse : $[M - H]^+$ : M/Z : 1019

**EXEMPLE 8 : O [(METHOXY - 2 ETHOXY) - METHYL] OXYIMINO (E + Z) - 9 DESOXY - 9 DEMYCAROSYL TYLOSINE**

Lors de la purification du résidu brut obtenu de la même façon que dans l'exemple 7, stade C, après séparation du dérivé mycarosylé par flash chromatographie on obtient l'O [(méthoxy - 2 éthoxy) - méthyl] oxyimino (E + Z) - 9 désoxy - 9 démycarosyl tylosine.

Rendement : 40 %

Caractéristiques spectrales :

RMN $^1H$ 200 MHz δ(ppm) δ = 2,45 ppm, singulet, 6H(2 x $CH_3$) : N - $(CH_3)_2$
δ = 3,35 ppm, singulet, 3H ($CH_3$) ($OCH_2$ - O $(CH_2)_2$ - $OCH_3$)
δ = 9,80 ppm, singulet, 1H, (CH = O)

Spectrométrie de masse : $[M - H]^+$ : M/Z : 875

**EXEMPLES 9 ET 10 :**

De la même façon que dans l'exemple 5, en remplaçant le chlorhydrate de benzyloxylamine par :

. le chlorhydrate d'O allylhydroxylamine **(EXEMPLE 9),**

. le chlorhydrate d'O éthylhydroxylamnine **(EXEMPLE 10).**

On obtient :

**EXEMPLE 9 : ALLYLOXYIMINO (E + Z) - 9 DESOXY - 9 DEMYCAROSYL TYLOSINE**

**EXEMPLE 10 : ETHYLOXYIMINO (E + Z) - 9 DESOXY - 9 DEMYCAROSYL TYLOSINE**

**EXEMPLE 11 : ALLYLOXYIMINO (E + Z) - 9 DESOXY - 9 TYLOSINE**

En procédant de la même façon que dans l'exemple 6, mais en remplaçant au stade B le chlorhydrate de benzyloxyamine par le chlorhydrate d'allyl hydroxylamine, on obtient le produit attendu.

**EXEMPLE 12 : ETHYLOXYIMINO (E + Z) - 9 DESOXY - 9 TYLOSINE**

En procédant de la même façon que dans l'exemple 6, mais en remplaçant au stade B le chlorhydrate de benzyloxyamine par le chlorhydrate d'O éthyl hydroxylamine, on obtient le produit attendu.

**EXEMPLES 13 ET 14 : O - [(VINYLOXY) - 2 ETHYL] OXYIMINO (E + Z) - 9 DESOXY - 9 TYLOSINE ET O - [(VINYLOXY) - 2 ETHYL] OXYIMINO (E + Z) - 9 DESOXY - 9 DEMYCAROSYL TYLOSINE**

En opérant comme dans les exemples 7 et 8, mais en remplaçant au stade B, le chlorure de (méthoxy - 2 éthoxy) méthyle par le chloro - 2 vinyloxy - 1 éthane, on obtient les produits attendus.

**EXEMPLE 15 : O - (DIMETHYLAMINO - 2 ETHYL) OXIME (E + Z) - 9 DE LA TYLOSINE**

Procéder comme dans l'exemple 7 en remplaçant au stade B, le chlorure de (méthoxy - 2 éthoxy) méthyle par une quantité double de chlorhydrate de chloro -1 diméthylamino - 2 éthane.

**EXEMPLE 16 : O - (DIMETHYLAMINO - 2 ETHYL) OXIME (E + Z) - 9 DE LA DEMYCAROSYL TYLOSINE**

Lors de la purification finale du composé de l'exemple 15, on obtient le composé attendu.

Caractéristiques spectrales :

Spectrométrie de masse : $[M - H]^+$ : M/Z : 857

**EXEMPLES 17 ET 18 : (DIMETHYLAMINO - 2 METHYL - 1 ETHYL) OXYIMINO (E + Z) - 9 DESOXY - 9 TYLOSINE ET (DIMETHYLAMINO - 2 METHYL - 1 ETHYL) OXYIMINO (E + Z) - 9 DESOXY - 9 DEMYCAROSYL TYLOSINE**

En procédant comme dans les exemples 15 et 16, mais en remplaçant le chlorhydrate de chloro - 1 diméthylamino - 2 éthane par le chlorhydrate de chloro - 2 diméthylamino - 1 propane, on obtient les deux produits du titre.

**EXEMPLE 19 : [(MORPHOLINYL - 4) - 2 ETHYL] OXYIMINO (E + Z) - 9 DESOXY - 9 TYLOSINE**

En procédant comme dans l'exemple 7, mais en remplaçant le chlorure de (méthoxy - 2 éthoxy) méthyle par le chlorhydrate de (chloro - 2 éthyl) - 4 morpholine, on obtient le produit attendu.

**EXEMPLE 20 : [(MORPHOLINYL - 4) - 2 ETHYL] OXYIMINO (E + Z) - 9 DESOXY - 9 DEMYCAROSYL TYLOSINE**

Lors de la purification finale du composé de l'exemple 19, on obtient le composé attendu.

**EXEMPLES 21 ET 22 : [(PYRROLIDINYL - 1) - 2 ETHYL] OXYIMINO (E + Z) - 9 DESOXY - 9 TYLOSINE ET [(PYRROLIDINYL - 1 ) - 2 ETHYL] OXYIMINO (E + Z) - 9 DESOXY - 9 DEMYCAROSYL TYLOSINE**

En remplaçant dans les exemples 19 et 20, le chlorhydrate de (chloro - 2 éthyl) - 4 morpholine par le chlorhydrate de (chloro - 2 éthyl) - 1 pyrrolidine, on obtient les produits attendus.

**EXEMPLES 23 ET 24 : [(PIPERIDINYL - 1) - 2 ETHYL] OXYIMINO (E + Z) - 9 DESOXY - 9 TYLOSINE ET [(PIPERIDINYL - 1) - 2 ETHYL] OXYIMINO (E + Z) - 9 DESOXY - 9 DEMYCAROSYL TYLOSINE**

En remplaçant dans les exemples 19 et 20, le chlorhydrate de (chloro - 2 éthyl) - 4 morpholine par le chlorhydrate de (chloro - 2 éthyl) - 1 pyrrolidine, on obtient les produits attendus.

**EXEMPLES 25 ET 26 :**

Bis [O - (méthyloxime) (E + Z)] - 9, 20 de la tylosine,

Bis [O - (méthyloxime) (E + Z)] - 9, 20 de la démycarosyl tylosine.

En opérant comme dans les exemples 2 et 3, mais en remplaçant le chlorhydrate d'hydroxylamine par le chlorhydrate d'O méthylhydroxylamine, on obtient :

**EXEMPLE 25 : BIS [O - (METHYLOXIME) (E + Z)] - 9, 20 DE LA TYLOSINE**

**EXEMPLE 26 : BIS [O - (METHYLOXIME) (E + Z)] - 9, 20 DE LA DEMYCAROSYLTYLOSINE**

**EXEMPLE 27 : BIS [O - (METHOXY -2 ETHOXY) METHYLOXIME] - 9, 20 DE LA TYLOSINE**

Dissoudre 2,2 g (2,32 mmol) de la dioxime (E + Z) - 9, 20 de la tylosine dans 40 $cm^3$ de tétrahydrofuranne. Ajouter 236 mg (5 mmol) d'une suspension à 50 % dans l'huile d'hydrure de sodium et agiter à température ambiante pendant 15 minutes. Ajouter 0,6 g (4,8 mmol) de chlorure de (méthoxy - 2 éthoxy) méthyle et agiter à température ambiante pendant 2 heures Après addition de 500 mg de trisilicate de magnésium, évaporer à sec le tétrahydrofuranne. Ajouter 300 ml de dichlorométhane, filter. La phase organique est évaporée à sec, et purifiée par chromatographie sur silice.

Caractéristiques spectrales :

Spectrométrie de masse : $[M - H]^+$ : M/Z : 1122

**EXEMPLE 28 : OXIME (E + Z) - 9 DE LA DEMYCAROSYL DEMYCINOSYL TYLOSINE**

**STADE A :** Démycarosyl tylosine

Agiter 4 g (0,004 mmol) de tylosine base dans 80 ml d'acide chlorhydrique 0,2N pendant 4 heures à la température ambiante. Laver le milieu réactionnel obtenu au dichlorométhane, séparer la phase aqueuse et l'ajuster à PH 8,0. Extraire à deux reprises par 120 ml de dichlorométhane, réunir les phases organiques, sécher sur sulfate de sodium et évaporer. Le résidu est constitué de démycarosyl tylosine.

Rendement : 94 %

Caratéristiques spectrales :

Spectrométrie de masse : $[M - H]^+$ : M/Z : 772

**STADE B :** Démycarosyl démycinosyl tylosine

Dissoudre 5 g (0,0065 mmol) de démycarosyl tylosine obtenu au stade A dans 110 ml d'acide chlorhydrique 0,5 N et agiter pendant 27 heures environ à 75 °C. Laver le milieu réactionnel au dichlorométhane. Récupérer la phase aqueuse, l'ajuster à PH 8 et extraire à deux reprises par 120 ml de dichlorométhane. Sécher la phase organique sur sulfate de sodium et évaporer. Le résidu est chromatographie sur gel de silice (éluant $CH_2Cl_2$ ; MEOH ; $NH_4OH$ ; 10 ; 1 ; 0,05) pour obtenir le produit attendu.

Rendement : 20 %

Caractéristiques spectrales :

Spectrométrie de masse : $[M - H]^+$ : M/Z : 598

**STADE C :** Diéthylacétal - 20 de la démycarosyl démycinosyl tylosine

Dissoudre 540 mg (0,9 mmol) de démycarosyl démycinosyl tylosine dans 10 ml d'éthanol anhydre. Ajouter à température ambiante 400 mg d'acide p -toluène sulfonique anhydre. Agiter pendant 4 heures à température ambiante et ajouter 0,3 ml de triéthylamine. Maintenir l'agitation pendant 10 minutes supplémentaires et évaporer la moitié du solvant sous pression réduite. Diluer à l'eau, extraire au dichlorométhane, laver à deux reprises par 30 ml d'une solution de bicarbonate de sodium puis à deux reprises par 30 ml d'eau. Sécher la phase organique sur sulfate de sodium et évaporer à sec. On obtient le produit attendu.

Rendement : 80 %

Caractéristiques spectrales :

Spectrométrie de masse : [M - H]+ : M/Z : 672

**STADE D :** Diéthylacétal - 20 de l'oxime (E + Z) - 9 de la démycarosyl démycinosyl tylosine

Dissoudre 100 mg (0,15 mmol) du diéthylacétal - 20 de la démycarosyl démycinosyl tylosine, obtenu au stade C, dans 4,5 ml de pyridine anhydre. Ajouter 45 mg (0,65 mmol) de chlorhydrate d'hydroxylamine et porter le mélange à 80°C pendant 4 heures. Diluer dans l'eau puis extraire au dichlorométhane, laver et sécher comme indiqué au stade précédent. Le résidu brut obtenu, est purifié par flash chromatographie.

Caractéristiques spectrales :

Spectrométrie de masse : [M - H]+ : M/Z : 687

**STADE E :** Oxime (E + Z) - 9 de la démycarosyl démycinosyl tylosine

Dissoudre 500 mg (0,73 mmol) du diéthylacétal - 20 oxime (E + Z) - 9 de la démycarosyl démycinosyl tylosine obtenu précédemment dans 40 ml d'acétonitrile. Ajouter 40 ml d'une solution aqueuse d'acide chlorhydrique 0,2 N et agiter 4h30 à température ambiante. Ajouter ensuite 20 ml de solution de bicarbonate de sodium à 5 %, extraire au dichlorométhane, laver et sécher comme indiqué au Stade C. Le produit obtenu est purifié par chromatographie préparative.

Caractéristiques spectrales :

Spectrométrie de masse : [M - H]+ : M/Z ; 613

**EXEMPLE 29 : DIOXIME (E + Z) - 9, 20 DE LA DEMYCAROSYL DEMYCINOSYL TYLOSINE**

Dissoudre 250 mg (0,42 mmol) de démycarosyl démycinosyl tylosine base obtenue dans l'exemple 1, stade B dans 30 ml de pyridine anhydre puis ajouter 0,30 g (4,3 mmol) de chlorhydrate d'hydroxylamine. Agiter pendant 4 heures en maintenant une température de 80°C. Diluer le milieu réactionnel par l'eau, extraire au dichlorométhane puis laver par une solution saturée de bicarbonate de sodium (2 x 10 ml) puis par l'eau (2 x 10 ml). La phase organique est séchée sur sulfate de sodium puis évaporée à sec. On obtient un résidu brut qui est purifié par chromatographie sur colonne de silice.

Caractéristiques spectrales :

Spectrométrie de masse : [M - H]+ : M/Z : 628

**EXEMPLE 30 : O METHYLOXIME (E + Z) - 9 DE LA DEMYCAROSYL DEMYCINOSYL TYLOSINE**

**STADE A :** O - méthyloxime (E + Z) - 9 du diéthylacétal - 20 de la démycarosyl démycinosyl tylosine.

Dissoudre 100 mg (0,15 mmol) du diéthylacétal - 20 de la démycarosyl démycinosyl tylosine obtenue dans l'exemple 28, Stade C dans 4,5 ml de pyridine anhydre. Ajouter 33 mg (0,40 mmol) de chlorhydrate de méthoxylamine et agiter sous atmosphère d'azote à température ambiante pendant 48 heures. Ajouter un volume d'eau glacée. Agiter quelques minutes, extraire au dichlorométhane puis laver par une solution saturée de bicarbonate de sodium (2 x 30 ml) puis par l'eau (2 x 30 ml). La phase organique est séchée sur sulfate de sodium puis évaporée à sec. On obtient un résidu brut qui est purifié par chromatographie sur colonne de silice. Le produit obtenu est utilisé tel quel dans l stade suivant.

**STADE B :** O - Méthyloxime (E + Z) - 9 de la démycarosyl démycinosyl tylosine

Procéder comme dans l'exemple 28, Stade E en remplaçant l'oxime (E + Z) - 9 diéthylacétal - 20 de la démycarosyl démycinosyl tylosine par l'O méthyloxime (E + Z) - 9 diéthylacétal - 20 de la démycarosyl démycinosyl tylosine.

Caractéristiques spectrales :

Spectrométrie de masse : [M - H]+ : M/Z : 627

**EXEMPLE 31 : O BENZYLOXIME (E + Z) - 9 DE LA DEMYCAROSYL DEMYCINOSYL TYLOSINE**

**STADE A :** O benzyloxime (E + Z) - 9 diéthylacétal - 20 de la démycarosyl démycinosyl tylosine

Dissoudre 100 mg (0,15 mmol) du diéthylacétal - 20 de la démycarosyl démycinosyl tylosine obtenue dans l'exemple 28, Stade C, dans 4,5 ml de pyridine anhydre. Ajouter 96 mg (0,6 mmol) de chlorhydrate de benzyloxyamine et agiter sous atmosphère d'azote à température ambiante pendant 72 heures. Extraire au

dichlorométhane, laver par une solution saturée de bicarbonate de sodium (2 x 30 ml) puis par l'eau (2 x 30 ml). Les phases organiques sont réunies, séchées sur sulfate de sodium puis évaporées à sec. On obtient un résidu qui est purifié par chromatographie sur colonne de silice en utilisant comme solvant d'élution le mélange dichlorométhane / méthanol / ammoniaque (20/1/0,05).

Rendement : 86 %

Caractéristiques spectrales :

Spectrométrie de masse : $[M-H]^+$ : M/Z : 777

**STADE B :** O benzyloxime (E + Z) - 9 de la démycarosyl démycinosyl tylosine

En opérant comme dans l'exemple 28, Stade E mais en remplaçant l'oxime (E + Z) - 9 diéthylacétal - 20 de la démycarosyl démycinosyl tylosine par l' O benzyloxime (E + Z) - 9 diéthylacétal - 20 de la démycarosyl démycinosyl tylosine obtenue au stade précédent, on obtient la O benzyloxime (E + Z) - 9 de la démycarosyl démycinosyl tylosine, purifiée par chromatographie préparative : (Solvant d'élution dichlorométhane / méthanol / ammoniaque (10/1/0,05)).

Spectrométrie de résonance magnétique nucléaire $^1H$ (400 MHz) : $\delta = 0,96$ ppm 3H, $CH_3$, $C_{17}$
$\delta = 1,00$ ppm 3H, $CH_3$, $C_{18}$
$\delta = 5,12$ ppm 2H, $CH_2$ $\underline{CH_2}$ - $C_6H_5$
$\delta = 7,38$ pm aromatiques

Caractéristiques spectrales :

Spectrométrie de masse : $[M-H]^+$ : M/Z : 703

**EXEMPLES 32 ET 33 :**

De la même façon que dans l'exemple 31, en remplaçant le chlorhydrate de benzyloxyamine par :
. le chlorhydrate d'O allylhydroxylamine **(EXEMPLE 32),**
. le chlorhydrate d'O éthylhydroxylamine **(EXEMPLE 33).**
On obtient :

**EXEMPLE 32 : 0 ALLYLOXIME (E + Z) - 9 DE LA DEMYCAROSYL DEMYCINOSYL TYLOSINE**

**EXEMPLE 33 : 0 ETHYLOXIME (E + Z) - 9 DE LA DEMYCAROSYL DEMYCINOSYL TYLOSINE**

**EXEMPLE 34 : 0 [(METTHOXY - 2 ETHOXY) - METHYL] OXYIMINO - 9 DESOXY - 9 DEMYCAROSYL DEMYCINOSYL TYLOSINE**

Dissoudre 2 g (2,92 mmol) du diéthylacétal - 20 de l'oxime (E + Z) -9 de la démycarosyl démycinosyl tylosine, obtenu au stade D de l'exemple 28, dans du tétrahydrofuranne et procéder ensuite comme dans l'exemple 7, stade B, on obtient le diéthylacétal - 20 de l'O [(méthoxy - 2 éthoxy) -méthyl] oxyimino (E + Z) - 9 désoxy - 9 démycarosyl démycinosyl tylosine que l'on dissout ensuite dans 40 ml d'acétonitrile pour le traiter selon le protocole explicité dans l'exemple 28, stade E.

Caractéristiques spectrales :

Spectrométrie de masse : $[M-H]^+$ : M/Z : 701

**EXEMPLE 35 : 0 - [(VINYLOXY) - 2 ETHYL] OXIME (E + Z) - 9 DE LA DEMYCAROSYL DEMYCINOSYL TYLOSINE**

En opérant comme dans l'exemple précédent mais en remplaçant le chlorure de (méthoxy - 2 éthoxy) méthyle par le chloro - 2 vinyloxy - 1 éthane, on obtient le produit attendu.

Caractéristiques spectrales :

Spectrométrie de masse : $[M-H]^+$ : M/Z : 683

**EXEMPLE 36 : 0 - (DIMETHYLAMINO - 2 ETHYL) OXIME (E + Z) 9 DE LA DEMYCAROSYL DEMYCINOSYL TYLOSINE**

Dissoudre 1,8 g (2,94 mmol) du diméthylacétal en 20 de l'oxime (E +Z) - 9 de la démycarosyl démycinosyl tylosine obtenu dans l'exemple 28, stade D dans 50 $cm^3$ de tétrahydrofuranne. Ajouter 142 mg (3 mmol) d'une suspension à 50 % dans l'huile d'hydrure de sodium et agiter à température ambiante pendant 15 minutes. Ajouter 0,44 g (3 mmol) de chlorhydrate de chloro - 1 diméthylamino - 2 éthane et agiter à température ambiante pendant 1 heure. Après addition de 300 mg de silicate de magnésium, évaporer le tétrahydrofuranne. Ajouter 200 ml de dichlorométhane, filtrer et évaporer à sec. Le résidu est purifié par chromatographie sur colonne de silice puis dissous dans 150 ml d'acétonitrile. On ajoute 150 ml d'une solution aqueuse d'acide chlorhydrique 0,2N et on agite 4h30 à température ambiante. On ajoute ensuite 20 ml de solution de bicarbonate de sodium à 5 % et on extrait au dichlorométhane. Laver, sécher et purifier par chromatographie sur colonne de silice.

Caractéristiques spectrales :

Spectrométrie de masse : $[M - H]^+$ : M/Z : 684

**EXEMPLE 37 : O - (DIMETHYLAMINO - 2 METHYL - 1 ETHYL) OXIME (E + Z) - 9 DE LA DEMYCAROSYL DEMYCINOSYL TYLOSINE**

En procédant comme dans l'exemple 36, mais en remplaçant le chlorhydrate de chloro - 1 diméthylamino - 2 éthane par le chlorhydrate de chloro - 2 diméthylamino - 1 propane, on obtient l'O - (diméthylamino - 2 méthyl - 1 éthyl) oxime (E + Z) - 9 de la démycarosyl démycinosyl tylosine.

Caractéristiques spectrales :

Spectrométrie de masse : $[M - H]^+$ : M/Z : 698

**EXEMPLE 38 : 0 - [(MORPHOLINYLE - 4) - 2 ETHYL] OXIME (E + Z) - 9 DE LA DEMYCAROSYL DEMYCINOSYL TYLOSINE**

Dissoudre 1,8 g (2,94 mmol) du diméthylacétal en 20 de l'oxime (E + Z) - 9 de la démycarosyl démycinosyl tylosine obtenue dans l'exemple 28 dans 50 $cm^3$ de tétrahydrofuranne et procéder comme dans l'exemple précédent en remplaçant le chlorhydrate de chloro - 1 diméthylamino - 2 éthane par le chlorhydrate de (chloro - 2 éthyl) - 4 morpholine.

Caractéristiques spectrales :

Spectrométrie de masse : $[M - H]^+$ : M/Z : 726

**EXEMPLE 39 : 0 - [(PYRROLIDINYL - 1) - 2 ETHYL] OXIME (E + Z) - 9 DE LA DEMYCAROSYL DEMYCINOSYL TYLOSINE**

En remplaçant dans l'exemple précédent le chlorhydrate de (chloro -2 éthyl) - 4 morpholine par le chlorhydrate de (chloro - 2 éthyl) - 1 pyrrolidine, on obtient le produit attendu.

Caractéristiques spectrales :

Spectrométrie de masse : $[M - H]^+$ : M/Z : 710

**EXEMPLE 40 : 0 - [(PIPERIDINYL - 1) - 2 ETHYL] OXIME (E + Z) - 9 DE LA DEMYCAROSYL DEMYCINOSYL TYLOSINE**

En remplaçant dans l'exemple 39, le chlorhydrate de (chloro - 2 éthyl) - 4 morpholine par le chlorhydrate de (chloro - 2 éthyl) - 1 pipéridine, on obtient le produit attendu.

Caractéristiques spectrales :

Spectrométrie de masse : $[M - H]^+$ : M/Z : 724

**EXEMPLE 41 : BIS [0 - (METHYLOXIME) (E + Z)] - 9, 20 DE LA DEMYCAROSYL DEMYCINOSYL TYLOSINE**

En opérant comme dans l'exemple 29, mais en remplaçant le chlorhydrate d'hydroxylamine par le chlorhydrate d'O méthylhydroxylamine, on obtient le produit attendu.

Caractéristiques spectrales :

Spectrométrie de masse : $[M - H]^+$ : M/Z : 656

**EXEMPLE 42 : BIS [0 - (METHOXY - 2 ETHOXY) METHYLOXIME] - 9, 20 DE LA DEMYCAROSYL DEMYCINOSYL TYLOSINE**

Procéder comme dans l'exemple 27, en remplaçant la dioxime (E + Z) -9, 20 de la tylosine par la dioxime (E + Z) - 9, 20 de la démycarosyl démycinosyl tylosine obtenue dans l'exemple 29.

Caractéristiques spectrales :

Spectrométrie de masse : $[M - H]^+$ : M/Z : 948

**EXEMPLE 43 : OXIME (E + Z) - 9 DE LA CARBOMYCINE B OU HYDROXYIMINO (E + Z) - 9 DESOXY - 9 CARBOMYCINE B**

**STADE A :** Carbomycine B ou dihydro - leucomycine $A_3$

Dissoudre 10,0 g (12,1 mmol) de leucomycine $A_3$ ou encore appelée Josamycine, dans 30 ml de DMSO et 15 ml de triéthylamine. A la solution ainsi obtenue, ajouter, à température ambiante et sous atmosphère d'azote 5,6 g de complexe trioxyde de soufre - pyridine (36,3 mmole) mis en solution dans 40 ml de DMSO. Agiter le milieu réactionnel à température ambiante pendant 3 heures. Ajouter environ 200 g de glace, filtrer ; reprendre le produit ainsi obtenu par le chlorure de méthylène, laver la phase organique à trois reprises par l'eau ; sécher la phase organique sur sulfate de sodium, filtrer et évaporer à sec. Purifier le résidu ainsi obtenu par flash chromatographie sur colonne de silice ; éluant $CH_2Cl_2$ -MeOH - $NH_4OH$ (300/5/0,25). On obtient 3,6 g de carbomycine B.

Rendement : 36,4 %.

**STADE B :** Diméthylacétal en 18 de la carbomycine B ou diméthoxy -18 désoxy - 18 carbomycine B

Dissoudre 2,8 g (3,4 mmole) de carbomycine B précédemment obtenue dans 30 ml de méthanol. Ajouter 0,65 g ( 3,4 mmole) d'acide paratoluène sulfonique monohydrate. Agiter la solution obtenue pendant 50 minutes ; puis ajouter 1,7 ml de triéthylamine et maintenir l'agitation pendant 30 minutes. Evaporer le méthanol, reprendre le résidu par 80 ml de dichlorométhane et laver à l'eau. Sécher la phase organique sur sulfate de sodium, filtrer et évaporer à sec. Purifier le résidu obtenu par flash chromatographie sur colonne de silice en utilisant comme éluant le mélange $CH_2Cl_2/CH_3OH/NH_4OH$ : 25/1/0,05. On obtient 2,0 g de diméthylacétal en 18 de la carbomycine B, ou diméthoxy - 18 désoxy - 18 carbomycine B.

Rendement : 68 %

**STADE C :** Oxime (E + Z) - 9 de la carbomycine B diméthylacétal en 18 ou didésoxy - 9,18 hydroxyimino (E + Z) - 9 diméthoxy - 18 carbomycine B

Dissoudre 1,5 g (17 mmol) de diméthylacétal en 18 de la carbomycine B dans 10 ml de pyridine. Ajouter 354 mg (51 mmole) de chlorhydrate d'hydroxylamine et agiter la solution obtenue pendant 24 heures à température ambiante. Ajouter de la glace et extraire au dichlorométhane. Sécher sur sulfate de sodium, filtrer et évaporer à sec. Le résidu obtenu est purifié par flash chromatographie en utilisant comme éluant le mélange $CH_2Cl_2/MeOH/NH_4OH$ : 300/5/0,25. On obtient 1,2 g d'oxime (E + Z) - 9 de la carbomycine B diméthylacétal.

Rendement : 71 %.

**STADE D :** Oxime (E + Z) - 9 de la carbomycine B ou hydroxyimino - 9 désoxy - 9 carbomycine B

Dissoudre 1,2 g d'oxime (E + Z) - 9 de la carbomycine B diméthylacétal en 18 (1,35 mmol) dans 100 ml d'un mélange acétonitrile -eau (1/1, v/v). Ajouter cinq équivalents (0,47 ml) d'acide difluoroacétique et agiter 3 jours à température ambiante. Ajouter 3 ml de triéthylamine et maintenir l'agitation 1 heure. Evaporer l'acétonitrile au bain marie sous vide et extraire à trois reprises par 80 ml de dichlorométhane. Sécher sur sulfate de sodium, filtrer et évaporer à sec le solvant. Le résidu obtenu est purifié par flash chromatographie sur colonne de silice ; éluant : chlorure de méthylène/méthanol/ammoniaque (300/5/0,25). On obtient 600 mg d'oxime.

Rendement : 53 %.

Formule brute : $C_{42}H_{68}N_2O_{15}$

Poids moléculaire : 840,98

Caractéristiques spectrales :

RMN $1_H$ Solvant $CDCl_3$ 200 MHz δ = 3,40 singulet, 3H $CH_3$ substituant $R_4$
δ = 3,71 singulet, 1H $H_5$
δ = 9,20 singulet, 1H aldéhydique $C_{18}$

RMN $^{13}C$ Solvant $CDCl_3$ δ = 84,9 ppm, $C_5$
δ = 201,4 ppm, $C_{18}$

Spectrométrie de masse : $[M-H]^+$ : M/Z : 841

**EXEMPLE 44 : O - BENZYLOXIME (E + Z) - 9 DE LA CARBOMYCINE B OU BENZYLOXYIMINO (E + Z) - 9 DESOXY - 9 CARBOMYCINE B**

**STADE A :** O benzyloxime (E + Z) - 9 du diméthylacétal - 18 de la carbomycine B

Dissoudre 1,5 g du diméthylacétal - 18 de la carbomycine B (17 mmol) obtenue dans l'exemple 43 stade B, dans 10 ml de pyridine. Ajouter 820 mg (51 mmol) de chlorhydrate de benzyloxyamine. Agiter 3 jours à température ambiante. Ajouter de la glace et extraire au $CH_2Cl_2$. Sécher sur $Na_2SO_4$, filtrer et évaporer à sec. Le produit obtenu est purifié par flash chromatographie sur colonne de silice en utilisant comme éluant le mélange $CH_2Cl_2$/méthanol/ammonique : 50 - 1 - 0,02). On obtient 1,2 g de produit attendu.

Rendement : 70%.

**STADE B :** O - Benzyloxime (E + Z) - 9·de la carbomycine B

Dissoudre 1,2 g (1,23 mmol) de l'O benzyloxime (E + Z) 9 diméthylacétal - 18 de la carbomycine B dans 100 ml d'un mélange acétonitrile eau (1/1, v/v). Ajouter 0,5 ml d'acide difluoroacétique. Agiter 4 jours à température ambiante puis ajouter 15 équivalents de triéthylamine (3 ml) et agiter une heure. Evaporer l'acétonitrile au bain marie sous vide et extraire au chlorure de méthylène. Sécher sur sulfate de sodium, filtrer, et évaporer à sec. Le résidu obtenu est purifié par flash chromatographie en utilisant comme éluant le mélange chlorure de méthylène/méthanol/ammoniaque (200/2,5/0,15). On obtient 500 mg du produit attendu.

Rendement : 45 %.

Formule brute : $C_{49}H_{74}N_2O_{15}$

Poids moléculaire : 931,1

Caractéristiques spectrales :

RMN $^1H$ Solvant $CDCl_3$ 200 MHz δ = 3,55 singulet, 3H, $\underline{COCH_3}$ ($R_4$)
δ = 5,10 singulet, 2H, $\underline{CH_2}$ - $C_6H_5$
δ = 7,20 singulet, 5H, noyau benzénique
δ = 9,30 singulet, 1H, aldéhydique.

RMN $^{13}C$ Solvant $CDCl_3$ δ = 155,8 ppm $C_9$
δ = 169,8 ppm $C_1$
δ = 201,1 ppm $C_{18}$

**EXEMPLE 45 : BENZYLOXYIMINO - 9 DESOXY - 9 DE - (ISOVALERYL - 4" MYCAROSYL) CARBOMYCINE B**

En opérant comme dans l'exemple 44, on obtient au stade B, après élution de la benzyloxyimino - 9 (E + Z) désoxy - 9 carbomycine B, la benzyloxyimino - 9 (E + Z) désoxy - 9 dé - (isovaléryl - 4" mycarosyl) carbomycine B.

Rendement : 20 %

Formule brute : $C_{37}H_{54}N_2O_{11}$

Poids moléculaire : 702,8

Caractéristiques spectrales :

RMN $^1H$ Solvant $CDCl_3$ 200 MHz δ = 3,53 ppm, singulet, 3H, CO - $CH_3$ ($R_4$)
δ = 7,30 ppm, singulet, 5H, noyau benzénique
δ = 9.40 ppm, singulet, H, aldéhydique.

RMN $^{13}C$ Solvant $CDCl_3$ δ = 156 ppm, $C_9$
δ = 170 ppm, $C_1$
δ = 201,3 ppm, $C_{18}$

Spectrométrie de masse (F.A.B) : $[M + H]^+$ : M/Z : 703

**EXEMPLE 46 : METHYLOXYIMINO (E + Z) - 9 DESOXY - 9 CARBOMYCINE B**

En opérant comme dans l'exemple 44 et en remplaçant le chlorhydrate de benzyloxyamine par le chlorhydrate de méthyloxyamine, on obtient le produit attendu.

**EXEMPLE 47 : METHYLOXYIMINO (E + Z) - 9 DESOXY - 9 DE -(ISOVALERYL - 4" MYCAROSYL) CARBOMYCINE B**

En opérant comme dans l'exemple 45 et en remplaçant le chlorhydrate de benzyloxyamine par le chlorhydrate de méthyloxyamine, on obtient le produit attendu.

Caratéristiques spectrales :

Spectrométrie de masse : $[M + H]^+$ : 627

**EXEMPLES 48 ET 49 :**

De la même façon que dans l'exemple 46, en remplaçant le chlorhydrate de méthyloxyamine par :
. le chlorhydrate d'O allylhydroxylamine **(EXEMPLE 48),**
. le chlorhydrate d'O éthylhydroxylamine **(EXEMPLE 49),**
on obtient :

**EXEMPLE 48 : ALLYLOXYIMINO (E + Z) - 9 DESOXY - 9 CARBOMYCINE B**

**EXEMPLE 49 : ETHYLOXYIMINO (E + Z) - 9 DESOXY - 9 CARBOMYCINE B**

**EXEMPLES 50 ET 51 :**

De la même façon que dans l'exemple 47, en remplaçant le chlorhydrate de méthyloxyamine par :
. le chlorhydrate d'O allyl hydroxylamine **(EXEMPLE 50),**
. le chlorhydrate d'O éthyl hydroxylamine **(EXEMPLE 51),**
on obtient :

**EXEMPLE 50 : ALLYLOXYIMINO (E + Z) - 9 DESOXY - 9 DE - (ISOVALERYL - 4" MYCAROSYL) CARBOMYCINE B**

**EXEMPLE 51 : ETHYLOXYIMINO (E + Z) - 9 DESOXY - 9 DE - (ISOVALERYL - 4" MYCAROSYL) CARBOMYCINE B**

**EXEMPLE 52 : 0 [(METHOXY - 2 ETHOXY) - METHYL] OXIME (E + Z) - 9 DE LA CARBOMYCINE B OU [(METHOXY - 2 ETHOXY) - METHYL] OXYIMINO (E + Z) - 9 DESOXY - 9 CARBOMYCINE B**

Dissoudre 0,46 g (0,52 mmol) du diméthylacétal de 18 en l'oxime (E + Z) - 9 de la carbomycine B obtenu dans l'exemple 43, stade C dans 10 ml de tétrahydrofuranne. Procéder ensuite comme dans l'exemple 7, stades B et C pour obtenir le produit attendu.

**EXEMPLE 53 : 0[(VINYLOXY) - 2 ETHYL] OXIME (E + Z) - 9 DE LA CARBOMYCINE B**

En opérant comme dans l'exemple précédent mais en remplaçant le chlorure de (méthoxy - 2 éthoxy) méthyle par le chloro - 2 vinyloxy - 1 éthane, on obtient le produit attendu.

**EXEMPLE 54 : 0 (DIMETHYLAMINO - 2 ETHYL) OXIME (E + Z) - 9 DE LA CARBOMYCINE B**

Dissoudre 2,0 g (2,25 mmol) du diméthylacétal en 18 de l'oxime (E + Z) - 9 de la carbomycine B obtenu dans l'exemple 43 dans 45 $cm^3$ de tétrahydrofuranne et procéder de la même façon que dans l'exemple 15.

**EXEMPLE 55 : 0 - (DIMETHYLAMINO - 2 METHYL - 1 ETHYL) OXIME (E + Z) - 9 DE LA CARBOMYCINE B**

En procédant comme dans l'exemple 54, mais en remplaçant le chlorhydrate de chloro - 1 diméthylamino - 2 éthane par le chlorhydrate de chloro - 2 diméthylamino - 1 propane, on obtient la 0 - (diméthylamino - 2 méthyl - 1 éthyl) oxime (E + Z) - 9 de la carbomycine B.

**EXEMPLE 56 : 0 [(MORPHOLINYL - 4) - 2 ETHYL] OXIME (E + Z) - 9 DE LA CARBOMYCINE B**

Dissoudre 2,0 g (2,25 mmol) du diméthylacétal de l'oxime (E + Z) - 9 de la carbomycine B obtenu dans l'exemple 43 dans 45 $cm^3$ de tétrahydrofuranne et procéder de la même façon que dans l'exemple 19.

**EXEMPLE 57 : 0 [(PYRROLIDINYL - 1) - 2 ETHYL) OXIME (E + Z) - 9 DE LA CARBOMYCINE B**

En remplaçant dans l'exemple précédent le chlorhydrate de (chloro -2 éthyl) - 4 morpholine par le chlorhydrate de (chloro - 2 éthyl) - 1 pyrrolidine, on obtient le produit attendu.

**EXEMPLE 58 : 0 [(PIPERIDINYL - 1) - 2 ETHYL] OXIME (E + Z) - 9 DE LA CARBOMYCINE B**

En remplaçant dans l'exemple 56, le chlorhydrate de (chloro - 2 éthyl) - 4 morpholine par le chlorhydrate de (chloro - 2 éthyl) - 1 pipéridine, on obtient le produit attendu.

**EXEMPLE 59 : DIOXIME (E + Z) - 9, 18 DE LA CARBOMYCINE B**

Dissoudre 2,8 g (3,4 mmol) de la carbomycine B obtenu dans l'exemple 43, Stade A dans 20 ml de pyridine. Ajouter 2,36 g (34 mmol) de chlorhydrate d'hydroxylamine et agiter la solution obtenue pendant 48 heures à température ambiante. Diluer le milieu réactionnel par l'eau, extraire au dichlorométhane. Sécher sur sulfate de sodium, filtrer et évaporer à sec. Le résidu obtenu est purifié par flash chromatographie sur colonne de silice en utilisant comme éluant un mélange $CH_2Cl_2/MeOH/NH_4OH$.

Caractéristiques spectrales :

Spectrométrie de masse : $[M-H]^+$ : 856

**EXEMPLE 60 : BIS [METHOXYIMINO (E + Z)] - 9,18 DIDESOXY - 9,18 CARBOMYCINE B**

En opérant comme dans l'exemple 59, mais en remplaçant le chlorhydrate d'hydroxylamine par le chlorhydrate de méthoxyamine, on obtient le produit attendu.

**EXEMPLE 61 : BIS [0 (METHOXY - 2 ETHOXY) METHYLOXIME (E + Z)] -9,18 DE LA CARBOMYCINE B**

Dissoudre 2 g (2,34 mmol) de la dioxime (E + Z) - 9,18 de la carbomycine B dans 40 $cm^3$ de tétrahydrofuranne, et procéder ensuite comme dans l'exemple 27. On obtient le produit attendu.

**EXEMPLE 62 : [(METHOXY - 2 ETHOXY) METHOXYIMINO (E + Z)] - 9 METHOXYIMINO - 18 DIDESOXY - 9,18 CARBOMYCINE B**

Dissoudre 2 g (2,15 mmol) de [(méthoxy - 2 éthoxy) - méthoxyimino] (E + Z) - 9 désoxy - 9 carbomycine B, obtenu dans l'exemple 52, dans 5 ml de pyridine. Ajouter 650 mg (65 mmole) de chlorhydrate de méthoxyamine. Agiter 3 jours à température ambiante. Diluer le milieu réactionnel à l'aide d'eau glacée et extraire au chlorure de méthylène. Sécher sur sulfate de sodium, filtrer et évaporer à sec. Purifier le résidu par flash chromatographie sur colonne de silice en utilisant comme éluant un mélange $CH_2Cl_2$/méthanol/ammoniaque. On obtient le produit attendu.

**EXEMPLE 63 : PARANITROBENZYLOXYIMINO (E + Z) - 9 DESOXY - 9 CARBOMYCINE B**

En remplaçant dans l'exemple 44, le chlorhydrate de benzyloxyamine par le chlorhydrate paranitrobenzyloxyamine, on obtient le produit attendu.

**EXEMPLE 64 : PARANITROBENZYLOXYIMINO (E + Z) - 9 TYLOSINE**

En remplaçant dans l'exemple 6, le chlorhydrate de benzyloxyamine par le chlorhydrate de paranitrobenzyloxyamine, on obtient le produit attendu.

**EXEMPLE 65 : ETUDE DE L'ACTIVITE DES PRODUITS DE FORMULE (I) SUR DIVERSES SOUCHES BACTERIENNES**

La détermination des concentrations minimales inhibitrices (CMI) est effectuée :

- pour les Staphylocoques et les Entérocoques (Streptocoques D) en milieu gélosé ou liquide de MUELLER HINTON ;
- pour les Hémophilus, Streptocoques non D et Neisseria Gonorrhoeae, la détermination des CMI est effectuée suivant la méthode de dilution en milieu gélosé au sang cuit enrichi du mélange Polyvitex*. La culture est faite en atmosphère enrichie en $CO_2$.

La lecture des CMI est effectuée après 18 heures d'incubation à 37° C.

Les produits sont testés dans la gamme de concentrations de 0,125 à 256 mg/l (dilutions successives de raison 2).

Les concentrations minimales inhibitrices sont de l'ordre :

- du $mg.ml^{-1}$ pour le Staphylocoque doré, les Streptocoques B, C, D, G les Pneumocoques ;
- de 0,5 $mg.ml^{-1}$ pour les Streptocoques A ;
- de 0,1 $mg.ml^{-1}$ pour Neisseria Gonorrhoeae.

L'ensemble de ces études montre l'activité antibiotique intéressante du produit de l'invention à la fois par l'intensité de son activité ainsi que par l'étendue de son spectre d'action.

**EXEMPLE 66 : COMPOSITION PHARMACEUTIQUE : COMPRIME**

Comprimés dosés à 100 mg de [(méthoxy - 2 éthoxy) méthoxyimino] (E + Z) - 9 désoxy - 9 tylosine

[(Méthoxy - 2 éthoxy) méthoxyimino] (E + Z) - 9 désoxy - 9 carbomycine B 100 g
Amidon de blé 70 g
Amidon de maïs 60 g
Lactose 60 g
Stéarate de magnésium 9 g
Silice 4 g
Hydroxy propylcellulose 7 g
Formule de préparation pour 1000 comprimés.

**Revendications**

1/ Composés de formule générale :

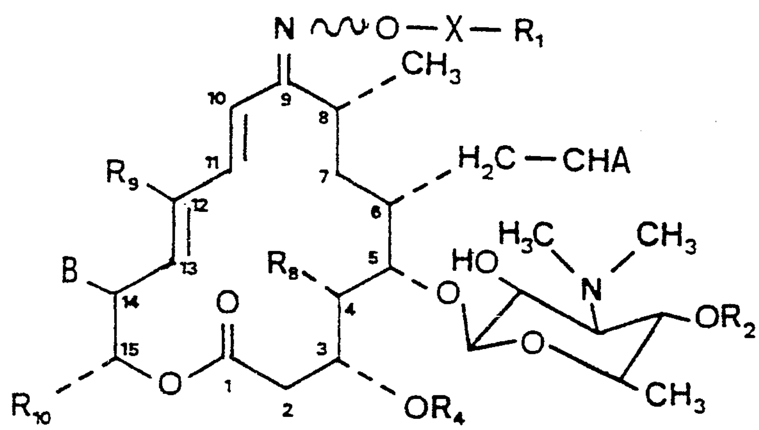

(I)

dans laquelle :

- A représente . soit un atome d'oxygène,

. soit un groupement de formule N ∿ O - Y - $R_5$, le signe ∿ existant sur le substituant du carbone 9 ainsi que dans la définition de A signifiant que l'une ou l'autre indépendamment des fonctions oxime ou éther d'oxime peut chacune se trouver sous la forme syn ou anti ou sous forme du mélange syn et anti,

- X et Y identiques ou différents représentent soit un radical alkyle comprenant au plus dix atomes de carbone, linéaire ou ramifié, soit un radical alkényle comprenant au plus dix atomes de carbone, linéaire ou ramifié, soit un radical alkynyle comprenant au plus dix atomes de carbone, linéaire ou ramifié, chacun des substituants alkyle, alkényle ou alkynyle pouvant être éventuellement substitué par un ou plusieurs groupements choisis parmi hydroxy, alkyle inférieur linéaire ou ramifié, alkényle inférieur linéaire ou ramifié, alkynyle inférieur linéaire ou ramifié, alkényloxy inférieur linéaire ou ramifié, alkénylthio inférieur linéaire ou ramifié, alkynyloxy inférieur linéaire ou ramifié, alkynylthio inférieur linéaire ou ramifié, fluoro, chloro, bromo, iodo, amino, dialkylamino inférieur,

- $R_1$ et $R_5$ identiques ou différents représentent :

- soit un atome d'hydrogène,

- soit un radical alkyloxy linéaire ou ramifié comprenant au plus dix atomes de carbone, un radical alkylthio linéaire ou ramifié comprenant au plus dix atomes de carbone, ou radical alkényloxy linéaire ou ramifié comprenant au plus dix atomes de carbone, ou radical alkénylthio linéaire ou ramifié comprenant au plus dix atomes de carbone, un radical alkynyloxy linéaire ou ramifié comprenant au plus dix atomes de carbone, un radical alkynylthio linéaire ou ramifié comprenant au plus dix atomes de carbone, un radical aryle (citons à titre d'exemple phényle, pyridyle, thiényle, furyle, benzothiényle, benzofuryle, indolyle, thiazolyle, oxazolyle, pyrrolyle, imidazolyle, pyrazolyle, isothiazolyle, isoxazolyle, benzimidazolyle), un radical aryloxy, arylthio, aralkyloxy, aralkylthio, chacun des radicaux alkyloxy, alkylthio, alkényloxy, alkénylthio, alkynyloxy, alkynylthio, aryle, aryloxy, arylthio, aralkyloxy, aralkylthio étant éventuellement substitué par un ou plusieurs des radicaux choisis parmi hydroxy, alkyle inférieur, alkényle inférieur, alkynyle inférieur, alkyloxy inférieur, alkényloxy inférieur, alkynyloxy inférieur, alkylthio inférieur, alkénylthio inférieur, alkynylthio inférieur, fluoro, chloro, bromo, iodo, nitro, amino, dialkkylamino inférieur, ou un radical N $R_6$ $R_7$ ci-dessous défini,

- soit un radical

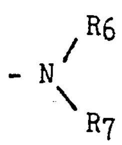

dans lequel $R_6$ et $R_7$ identiques ou différents représentent un atome d'hydrogène, un radical alkyle inférieur, ou alkényle inférieur ou alkynyle inférieur, ou forment avec l'azote un système hétérocyclique saturé ou non, comprenant éventuellement un autre hétéroatome et éventuellement substitué par un atome d'halogène ou un radical alkyle inférieur,

- un atome de chlore, de brome, d'iode ou de fluor,

- un groupement carboxylique libre ou salifié par une base minérale (KOH, NaOH, $Ca(OH)_2$) ou organique (triéthylamine, diéthylamine...) ou estérifié par un alcool aliphatique de formule $R_8OH$ où $R_8$ est un groupement alcoyle inférieur,

- ou X - $R_1$ et Y - $R_5$ indépendamment l'un de l'autre représentent chacun un atome d'hydrogène,

- $R_2$ représente . soit un atome d'hydrogène,

. soit un radical de formule :

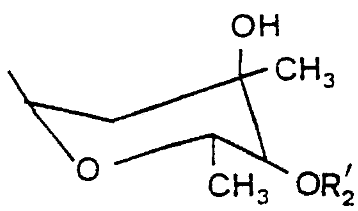

dans lequel $R'_2$ représente un atome d'hydrogène, un radical alkyle ou un radical acyle inférieur, linéaire ou ramifié,

- B représente . soit un atome d'hydrogène,

. soit un radical de formule : - $CH_2$ - O - B', où B' représente :

. soit un atome d'hydrogène,

. soit un radical de formule :

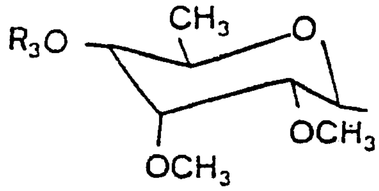

dans lequel $R_3$ représente un atome d'hydrogène ou un radical alkyle inférieur linéaire ou ramifié,

- $R_4$ représente . soit un atome d'hydrogène,

. soit un radical alkyle inférieur linéaire ou ramifié,

. soit un radical acyle inférieur linéaire ou ramifié,

- $R_8$ représente . soit un radical alkyle inférieur préférentiellement méthyle lorsque B représente un groupement - $CH_2$ - O - B′,

. soit un radical alkyloxy inférieur préférentiellement méthoxy lorsque B représente un atome hydrogène,

- $R_9$ représente . soit un atome d'hydrogène lorsque B représente un atome d'hydrogène,

. soit un radical alkyle inférieur préférentiellement méthyle lorsque B représente un radical - $CH_2$ - O - B′,

- $R_{10}$ représente un radical alkyle inférieur,

le terme inférieur concernant les radicaux alkyle inférieur, alkyloxy inférieur, alkylthio inférieur, alkényle inférieur, alkényloxy inférieur, alkénylthio inférieur, alkynyle inférieur, alkynyloxy inférieur, alkynylthio inférieur, signifiant qu'il s'agit de groupements comprenant entre 1 et 6 atomes de carbone, et leurs sels d'addition avec un acide.

2/ Composés selon la revendication 1 caractérisés en ce que A représente un atome d'oxygène, leurs isomères, (Z) ou (E) isolés ou sous forme de mélange ainsi que leurs sels d'addition à un acide pharmaceutiquement acceptable.

3/ Composés selon la revendication 1 caractérisés en ce que A représente un groupement de formule N ∿ O - Y - $R_5$, leurs isomères (Z) ou (E) isolés ou sous forme de mélange ainsi que leurs sels d'addition à un acide pharmaceutiquement acceptable.

4/ Composés selon l'une quelconque des revendications 1 ou 3 caractérisés en ce que Y - $R_5$ a la même signification que X - $R_1$, ainsi que leurs isomères (Z) ou (E) isolés ou sous forme de mélange ainsi que leurs sels d'addition à un acide pharmaceutiquement acceptable.

5/ Composés selon l'une quelconque des revendications 1 ou 2 caractérisé en ce que :

- A représente un atome d'oxygène,
- X est un radical alcoyle inférieur linéaire ou ramifié,
- $R_1$ est . un atome d'hydrogène,

. un radical alkyle,

. un atome aryle éventuellement substitué par un groupement nitro,

. un radical alkényloxy,

. un radical alkyloxy éventuellement substitué par un radical alkyloxy inférieur,

. un radical dialkylamino,

. un hetérocycle azoté comprenant éventuellement un autre hétéroatome,

. un radical aryloxy ou aralkyle,

ou bien X - $R_1$ représente un atome d'hydrogène, leurs isomères (Z) ou (E) isolés ou sous forme de mélange ainsi que leurs sels d'addition à un acide pharmaceutiquement acceptable.

6/ Composés selon l'une quelconque des revendications 1 ou 3 dans lesquels :

- Y est un radical alkyle inférieur,
- $R_5$ est un atome d'hydrogène ou radical alkyle ou un radical alkyloxy éventuellement substitué par un radical alkyloxy inférieur, ou Y - $R_5$ représente un atome d'hydrogène, leurs isomères (Z) ou (E) isolés ou sous forme de mélange ainsi que leurs sels d'addition à un acide pharmaceutiquement acceptable.

7/ Composés selon les revendications 1 à 6, dans lesquels B′ et $R_2$ représentent simultanément un atome d'hydrogène, leurs isomères (Z) ou (E) isolés ou sous forme de mélange ainsi que leurs sels d'addition à un acide pharmaceutiquement acceptable.

8/ Composés selon les revendications 1 à 6, dans lesquels B et $R_9$ représentent simultanément un atome d'hydrogène, $R_8$ un groupement méthoxy et $R_{10}$ un groupement méthyle, leurs isomères (Z) ou (E) isolés ou sous forme de mélange ainsi que leurs sels d'addition à un acide pharmaceutiquement acceptable.

9/ Composés selon les revendications 1 à 6 dans lesquels B représente un groupe de formule :

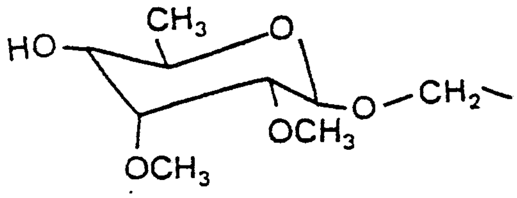

et $R_8$ et $R_9$ représentent simultanément un groupement méthyle et $R_{10}$ un groupement éthyle, leurs isomères (Z) ou (E) isolés ou sous forme de mélange ainsi que leurs sels d'addition à un acide pharmaceutiquement acceptable.

10/ Composé selon l'une quelconque des revendications 1 ou 9 qui est l'oxime - 9 de la tylosine, ses isomères (Z) et (E) isolés ou sous forme de mélange ainsi que ses sels d'addition à un acide pharmaceutiquement acceptable.

11/ Composé selon l'une quelconque des revendications 1 ou 9 qui est l'O - méthyloxime - 9 de la tylosine, ses isomères (Z) et (E) isolés ou sous forme de mélange ainsi que ses sels d'addition à un acide pharmaceutiquement acceptable.

12/ Composé selon l'une quelconque des revendications 1 ou 9 qui est l'O benzyloxime - 9 de la tylosine, ses isomères (Z) et (E) isolés ou sous forme de mélange ainsi que ses sels d'addition à un acide pharmaceutiquement acceptable.

13/ Composé selon l'une quelconque des revendications 1 ou 9 qui est l'O [(méthoxy - 2 éthoxy) - méthyl] oxime - 9 de la tylosine, ses isomères (Z) et (E) isolés ou sous forme de mélange ainsi que ses sels d'addition à un acide pharmaceutiquement acceptable.

14/ Composé selon l'une quelconque des revendications 1 ou 9 qui est la dioxime - 9, 20 de la tylosine, ses isomères (Z) et (E) isolés ou sous forme de mélange ainsi que ses sels d'addition à un acide pharmaceutiquement acceptable.

15/ Composé selon l'une quelconque des revendications 1 ou 9 qui est l'oxime - 9 de la démycarosyl tylosine, ses isomères (Z) et (E) isolés ou sous forme de mélange ainsi que ses sels d'addition à un acide pharmaceutiquement acceptable.

16/ Composé selon l'une quelconque des revendications 1 ou 9 qui est l'O - méthyloxime - 9 de la démycarosyl tylosine, ses isomères (Z) et (E) isolés ou sous forme de mélange ainsi que ses sels d'addition à un acide pharmaceutiquement acceptable.

17/ Composé selon l'une quelconque des revendications 1 ou 9 qui est l'O - benzyloxyme - 9 de la démycarosyl tylosine, ses isomères (Z) et (E) isolés ou sous forme de mélange ainsi que ses sels d'addition à un acide pharmaceutiquement acceptable.

18/ Composé selon l'une quelconque des revendications 1 ou 9 qui est 1'O [méthoxy - 2 éthoxy) - méthyl] oxime - 9 de la démycaroxyl tylosine, ses isomères (Z) et (E) isolés ou sous forme de mélange ainsi que ses sels d'addition à un acide pharmaceutiquement acceptable.

19/ Composé selon l'une quelconque des revendications 1 ou 9 qui est la dioxime - 9, 20 de la démycarosyl tylosine, ses isomères (Z) et (E) isolés ou sous forme de mélange ainsi que ses sels d'addition à un acide pharmaceutiquement acceptable.

20/ Composé selon l'une quelconque des revendications 1 ou 8 qui est l'hydroxyimino (E + Z) - 9 carbomycine B, ses isomères (Z) et (E) isolés ou sous forme de mélange ainsi que ses sels d'addition à un acide pharmaceutiquement acceptable.

21/ Composé selon l'une quelconque des revendications 1 ou 8 qui est la méthoxyimino (E + Z) - 9 désoxy - 9 carbomycine B, ses isomères (Z) et (E) isolés ou sous forme de mélange ainsi que ses sels d'addition à un acide pharmaceutiquement acceptable.

22/ Composé selon l'une quelconque des revendications 1 ou 8 qui est la benzyloxyimino (E + Z) - 9 désoxy - 9 carbomycine B, ses isomères (Z) et (E) isolés ou sous forme de mélange ainsi que ses sels d'addition à un acide pharmaceutiquement acceptable.

23/ Composé selon l'une quelconque des revendications 1 ou 8 qui est la paranitrobenzyloxyimino (E + Z) - 9 désoxy - 9 carbomycine B, ses isomères (Z) et (E) isolés ou sous forme de mélange ainsi que ses sels d'addition à un acide pharmaceutiquement acceptable.

24/ Composé selon l'une quelconque des revendications 1 ou 8 qui est l'O [(méthoxy - 2 éthoxy) - méthyl] oxyimino - 9 désoxy - 9 carbomycine B, ses isomères (Z) et (E) isolés ou sous forme de mélange ains que ses sels d'additions à un acide pharmaceutiquement acceptable.

25/ Procédé de préparation des composés de formule (I) caractérisé en ce que l'on utilise comme matière première :

- soit lorsque B représente un atome d'hydrogène, un dérivé de formule (II/1) :

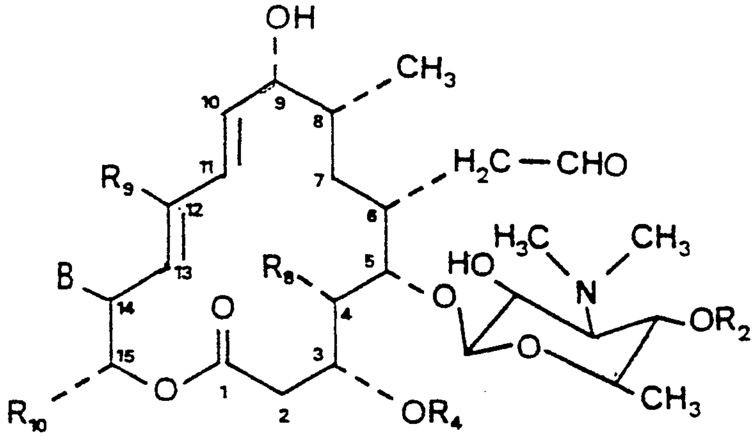

(II/1)

dans laquelle $R_2$, $R_4$, $R_8$, $R_{10}$ ont la même signification que dans la formule (I),
B et $R_9$ représentant simultanément un atome d'hydrogène,
que l'on soumet à un agent d'oxydation comme par exemple le complexe trioxyde de soufre-pyridine en présence d'un agent basique comme une amine organique,
en milieu organique, préférentiellement choisi parmi diméthylsulfoxide, diméthylformamide, pyridine,
pour obtenir un dérivé de formule (II/1a) :

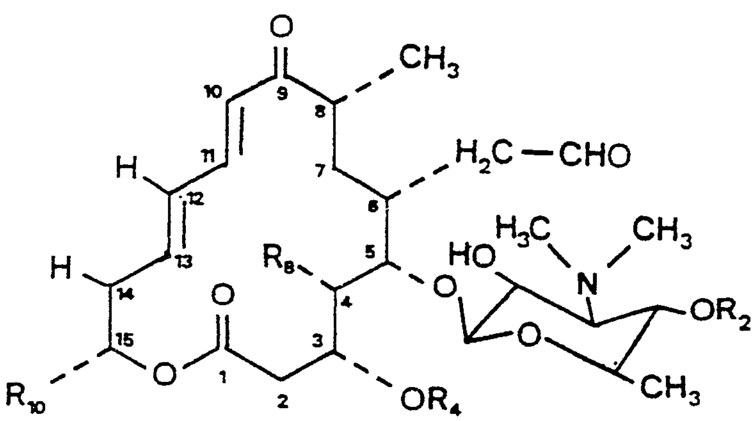

(II/1a)

dans laquelle $R_2$, $R_4$, $R_8$ et $R_{10}$ ont la même signification que dans la formule (I),

- soit, lorsque dans le composé de formule (I) que l'on souhaite obtenir, B et $R_9$ ne représentent pas simultanément chacun un atome d'hydrogène
un dérivé de formule (II/2) :

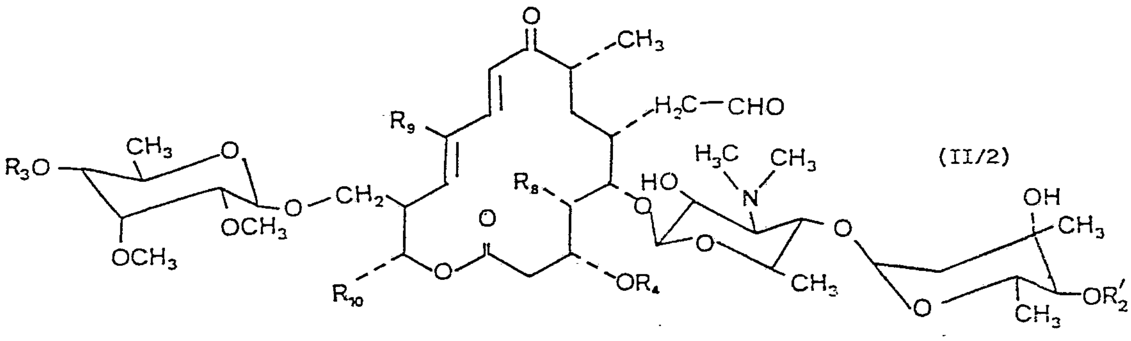

(II/2)

dans laquelle $R'_2$, $R_3$, $R_4$ et $R_{10}$ ont la même définition que dans la formule (I), et $R_8$ et $R_9$ signifient un radical alkyle inférieur préférentiellement méthyle,

qui,

lorsque dans le composé de formule (I) que l'on souhaite obtenir B' et $R_2$ représentent simultanément un atome d'hydrogène est tout d'abord soumis à l'action de l'acide chlorhydrique dilué de normalité comprise entr 0,05 et 0,4 préférentiellement comprise entre 0,1 et 0,30, préférentiellement comprise entre 0,15 et 0,25 à température ambiante pour conduire après lavage par un solvant organique approprié, alcalinisation et extraction par un solvant organique approprié à un dérivé de formule (II/2a) :

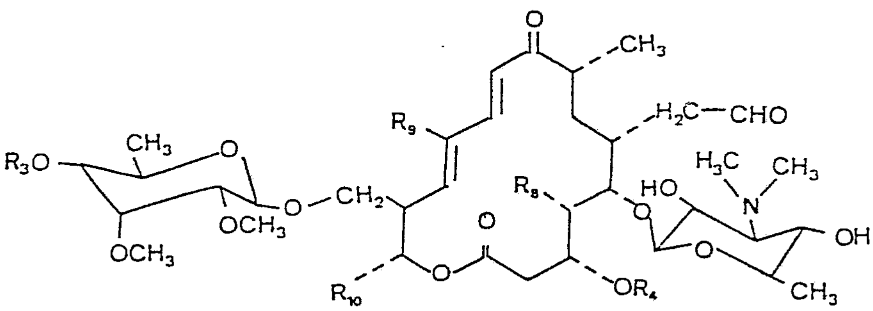

(II/2a)

dans laquelle $R_3$, $R_4$ et $R_{10}$ ont la même signification que dans la formule (I), et $R_8$ et $R_9$ la même signification que dans la formule (II/2),

que l'on fait réagir avec l'acide chlorhydrique dilué de normalité comprise entre 0,25 et 0,75 ; préférentiellement entre 0,3 et 0,7 ; préférentiellement entre 0,4 et 0,6 ; à une température comprise préférentiellement entre 30 et 100 °C ; préférentiellement comprise entre 50 et 90 °C ; preférentiellement entre 70 et 80 °C, pour conduire, après lavage par un solvant organique approprié, alcalinisation et extraction par un solvant organique approprié à un dérivé de formule (II/2b) :

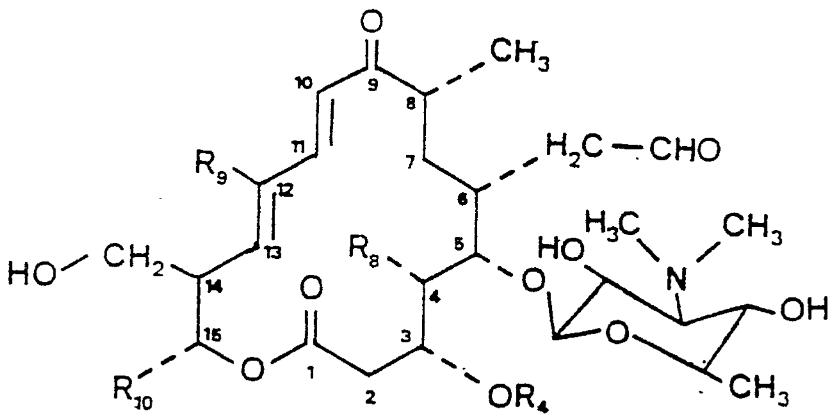

(II/2b)

dans laquelle $R_4$ et $R_{10}$ ont la même signification que dans la formule (I), et $R_8$ et $R_9$ la même signification que dans la formule (II/2)
que l'on purifie éventuellement par chromatographie sur colonne de silice,
le dérivé alors choisi de formule générale (II/1a), (II/2), ou (II/2b) selon le composé de formule (I) que l'on souhaite obtenir, étant alors condensé avec un alcool de formule Z - OH, dans lequel Z signifie un groupement alkoyle inférieur, en présence d'un agent catalyseur acide comme l'acide paratoluène sulfonique ou préférentiellement l'acide difluoroacétique lorsque le composé de formule (I) que l'on souhaite obtenir a un radical $R_2$ différent H,
le milieu réactionnel étant ensuite soumis à l'action d'un agent basique, en particulier une amine comme la triéthylamine, pour obtenir, après extraction par un solvant organique préférentiellement choisi parmi éther diéthylique, chloroforme, chlorure de méthylène et purification par chromatographie sur colonne de silice, un dérivé de formule (III) :

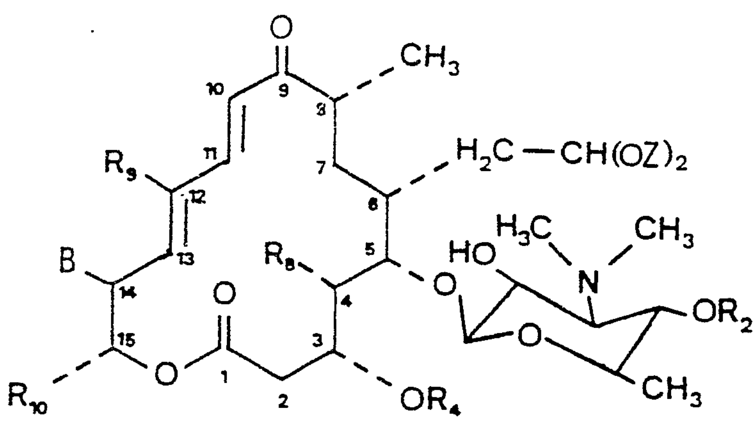

(III)

dans lequel B, $R_2$, $R_4$, $R_8$, $R_9$ et $R_{10}$ ont la même signification que dans la formule de dérivé choisi (II/1a), (II/2) ou (II/2b) et Z la même définition que précédemment,
que l'on condense en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium,

- soit avec un dérivé de formule (IV) :

**$H_2N$ - O - X - $R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V) :

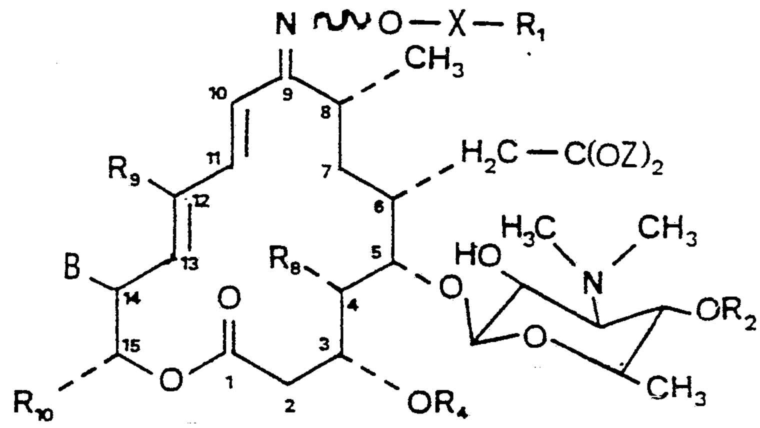

(V)

dans lequel le signe ∼ , X, $R_1$, $R_2$, $R_4$, $R_8$, $R_9$, $R_{10}$ et B ont la même signification que dans la formule (III) et Z la même définition que précédemment,

- soit avec un sel d'acide fort de l'hydroxylamine pour obtenir un dérivé de formule (V/a) :

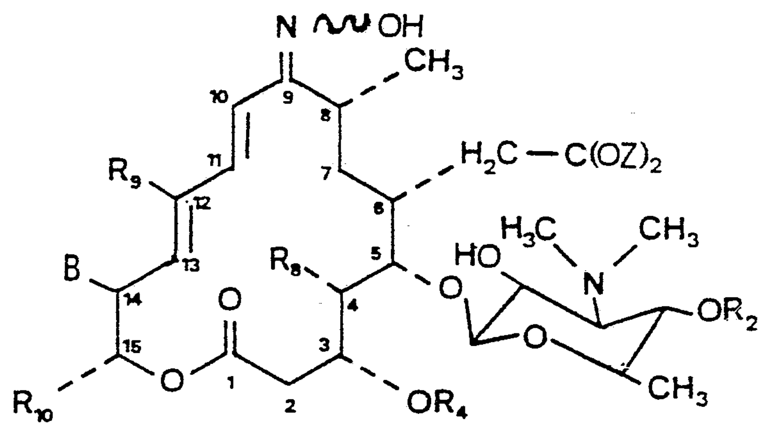

(V/a)

cas particulier de dérivés de formule (V), dans lequel le signe ∼ , B, $R_2$, $R_4$, $R_8$, $R_9$, $R_{10}$ et Z ont la même signification que dans la formule (V), X - R1 significant ici un atome d'hydrogène,
que l'on traite le cas échéant en présence d'une base, comme par exemple la triéthylamine ou la pyridine ou un sel de métal alcalin comme le carbonate de sodium ou le carbonate de potassium ou le carbonate de calcium ou l'hydrogéno carbonate de sodium ou l'hydrogéno carbonate de potassium, ou un hydrure de métal alcalin tel que l'hydrure de sodium dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuellement par chromatographie sur colonne de silice un produit de formule (V), dans lequel le signe ∼ , X, $R_1$, $R_2$, $R_4$, $R_8$, $R_9$, $R_{10}$ et B ont la même signification que dans la formule (I) et Z la même signification que dans la formule (III),
qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,01N et 0,5N, préférentiellement comprise entre 0,02N et 0,2N, préférentiellement comprise entre 0,05N et 0,5N, préférentiellement comprise entre 0,075N et 0,25N à température ambiante,
ou préférentiellement l'acide difluoroacétique en solution dans un mélange de solvants tel que le mélange acétonitrile - eau (1/1, v/v) lorsque le composé de formule (I) que le composé de formule (I) que l'on désire obtenir, possède un radical $R_2$ différent de l'atome d'hydrogène,
pour obtenir un composé de formule (I/a), cas particulier des composés de formule (I) :

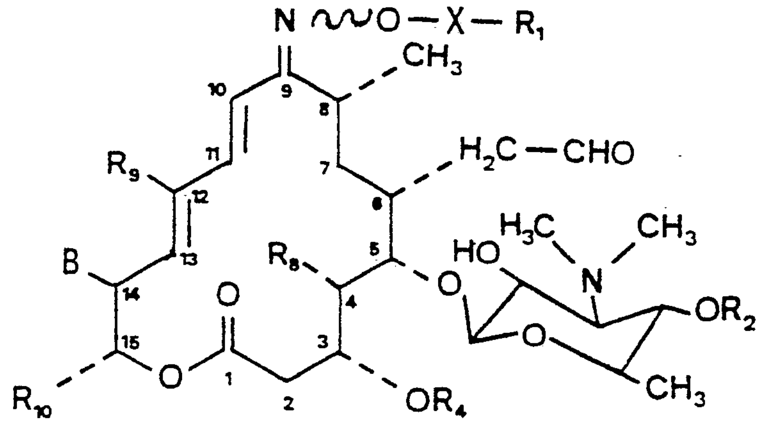

(I/a)

dans lequel le signe ∿ , X, $R_1$, $R_2$, $R_4$, $R_8$, $R_9$, $R_{10}$ et B ont la même définition que dans la formule (I), A représentant ici un atome d'oxygène,

lequel, dans le cas où dans la matière première utiisée $R_2$ ne signifie pas un atome d'hydrogène, peut au cours du processus de synthèse, en fonction des conditions opératoires sélectionnées ainsi que des valeurs des groupements X, $R_1$, $R_8$, $R_9$, $R_{10}$ et B, avoir été partiellement hydrolysé en un dérivé (I/a) démycarosylé pour lequel $R_2$ = H, les deux dérivés ainsi obtenus (c'est-à-dire celui pour lequel $R_2$ = H et celui pour lequel $R_2$ correspond à un résidu mycarosé) étant alors aisément séparés par une technique classique de séparation comme la chromatographie sur colonne de silice,

ce dérivé de formule (I/a) peut, le cas échéant, être traité en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuellement par chromatographie sur colonne de silice un dérivé de formule (I),

- soit par un sel d'acide fort de l'hydroxylamine pour obtenir un produit de formule (I/b),

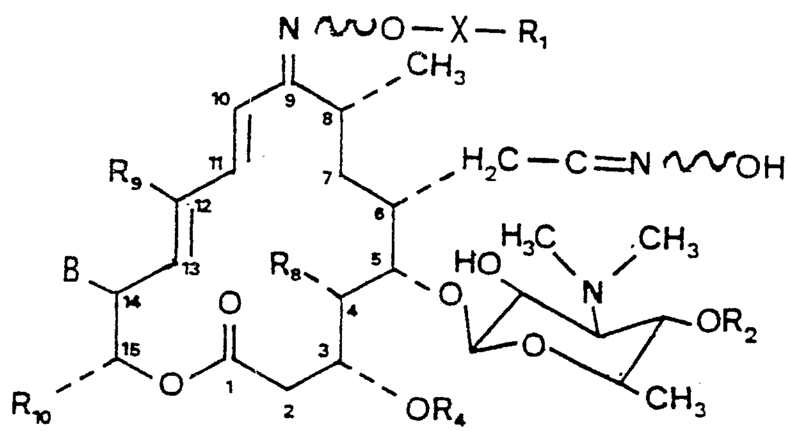

(I/b)

cas particulier de dérivés de formule (I) dans lequel le signe ∿ , X, $R_1$, $R_2$, $R_4$, $R_8$, $R_9$, $R_{10}$ et B ont la même signification que dans la formule (I), A représentant ici un groupement N ∿ O - Y - $R_5$ dans lequel Y - $R_5$ représente un atome d'hydrogène,

qui est traité le cas échéant, en présence d'une base, comme par exemple la triéthylamine, ou la pyridine, ou d'un sel de métal alcalin comme le carbonate de sodium ou de carbonate de potassium, ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T′ (VIII)**

dans laquelle T′ représente un atome d'halogène et $R_5$ et Y ont la même signification que dans la formule

(I), à l'exclusion du cas où $R_5$ - Y représentent ensemble un atome d'hydrogène, pour obtenir un composé de formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

26/ Procédé de préparation selon la revendication 25 des dérivés de formule (I) dans lesquels Y - $R_5$ a la même signification que X - $R_1$ ou dérivés (IX) caractérisé en ce que l'on condense un dérivé de formule (II/1a) ou (II/2) ou (II/2b) selon la formule de composé de formule (I) que l'on souhaite obtenir:

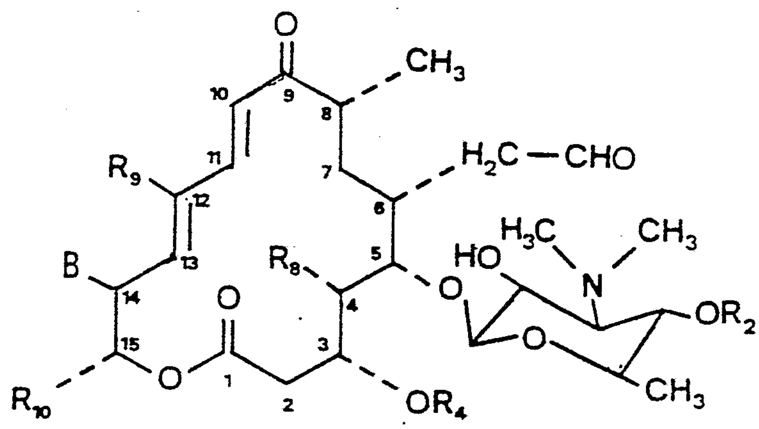

(II)

dans laquelle $R_2$, $R_4$, $R_8$, $R_9$, $R_{10}$ et B ont la même définition que celle correspondant à chacune des formules (II/1a), (II/2) ou (II/2b), selon la revendication 25 en présence d'une base comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium

- soit avec un dérivé de formule (IV) :

**$H_2N$ - O - X - $R_1$ (IV)**

dans lequel X - $R_1$ a la même signification que dans la formule (I) à l'exception du cas où X - $R_1$ représente un atome d'hydrogène.

pour obtenir un produit de formule (IX) :

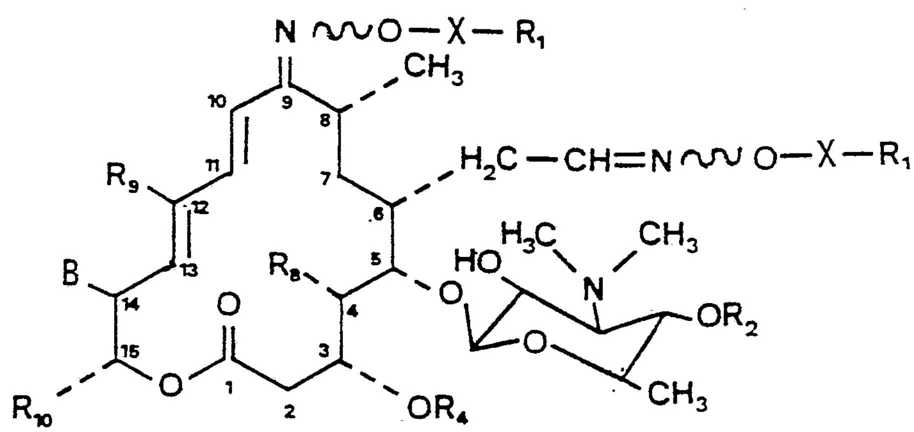

(IX)

cas particulier des dérivés de formule (I) dans laquelle le signe ∿ X, $R_1$, $R_2$, $R_4$, $R_8$, $R_9$, $R_{10}$ et B ont la même signification que dans la formule (I),

- soit avec un sel d'acide fort de l'hydroxylamine pour obtenir un dérivé de formule (IX/a) :

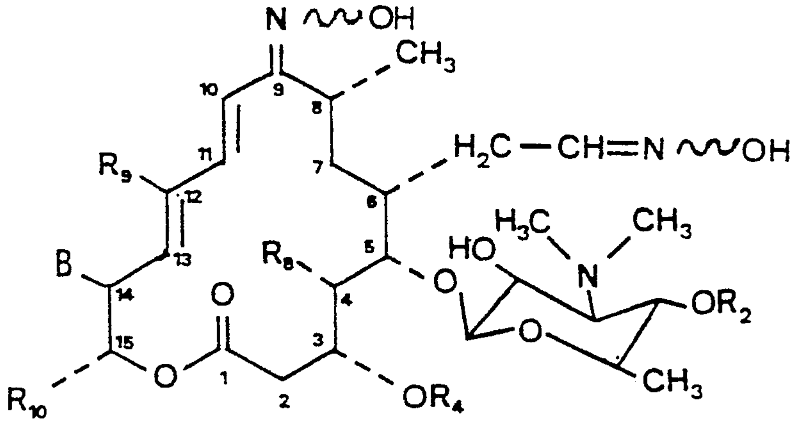

(IX/a)

cas particulier de dérivés de formules (I) et (IX),
dans lequel le signe ∿∿ , $R_2$, $R_4$, $R_8$, $R_9$, $R_{10}$ et B ont la même signification que dans la formule I, X - $R_1$ et Y - $R_5$ représentant ici ensemble un atome d'hydrogène,
que l'on traite éventuellement en présence d'une base, comme par exemple la triéthylamine, la pyridine ou un sel de métal alcalin comme le carbonate de sodium ou le carbonate de potassium ou l'hydrogéno carbonate de sodium ou l'hydrogéno carbonate de potassium, ou un hydrure de métal alcalin tel que l'hydrure de sodium, dans un solvant préférentiellement choisi parmi l'acétone ou le diméthyl formamide, le dioxanne, l'acétonitrile, le tétrahydrofuranne, l'éther diéthylique ou diisopropylique, par un dérivé de formule (X) :

**$R_1$ - X - T″ (X)**

dans lequel T″ représente un atome d'halogène et $R_1$ et X ont la même signification que dans la formule (I), à l'exclusion du cas où $R_1$ et X forment ensemble un atome d'hydrogène, pour obtenir un produit de formule (IX) cas particulier des dérivés de formule (I), dans lequel le signe ∿∿ $R_1$, $R_2$, $R_4$, $R_8$, $R_9$, $R_{10}$, B et X ont la même signification que dans la formule (I),
que l'on purifie éventuellement par chromatographie sur colonne de silice à l'aide d'un mélange solvant approprié comme par exemple un mélange chlorure de méthylène / méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

27/ Procédé de préparation selon la revendication 25, de composés de formule (I/f), cas particulier des dérivés de formule (I), dans lesquels :

B représente un groupement - $CH_2$ - O - B′ où B′ représente un groupement de formule :

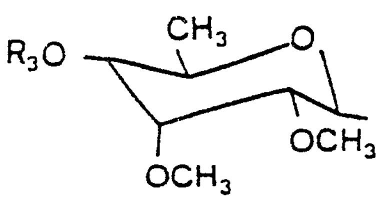

$R_2$ un groupe de formule :

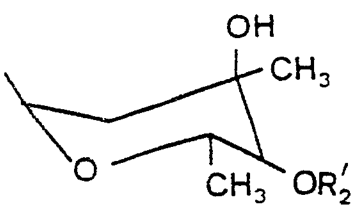

cas particulier où $R'_2$ et $R_3$ représentent un atome d'hydrogène, et A, X, $R_1$, $R_4$, $R_8$, $R_9$ et $R_{10}$ ont la même signification que dans la formule (I),

caractérisé en ce que l'on fait agir sur la tylosine, connue de la littérature, un alcool aliphatique inférieur Z - OH, préférentiellement le méthanol, en présence d'un agent acide comme par exemple l'acide difluoroacétique,
le milieu réactionnel étant ensuite soumis à l'action d'un agent basique, en particulier une amine organique approprié (préférentiellement le dichlorométhane) et purification par chromatographie sur colonne de silice un dérivé de formule (III/b) :

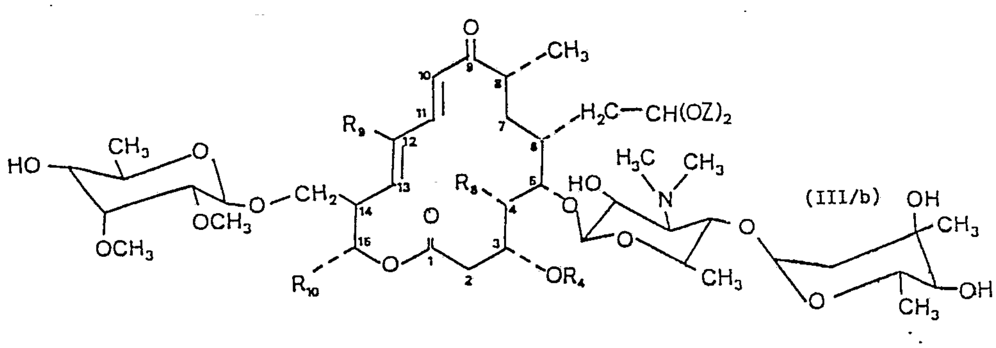
(III/b)

dans lequel $R_4$, $R_8$, $R_9$ et $R_{10}$ ont la même signification que dans la formule (I),
et Z représente un groupement alkyl inférieur,
que l'on condense en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium,

- soit avec un dérivé de formule (IV) :

**$H_2N$ - O - X - $R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuellement par chromatographie sur colonne de silice un dérivé de formule (V/b) :

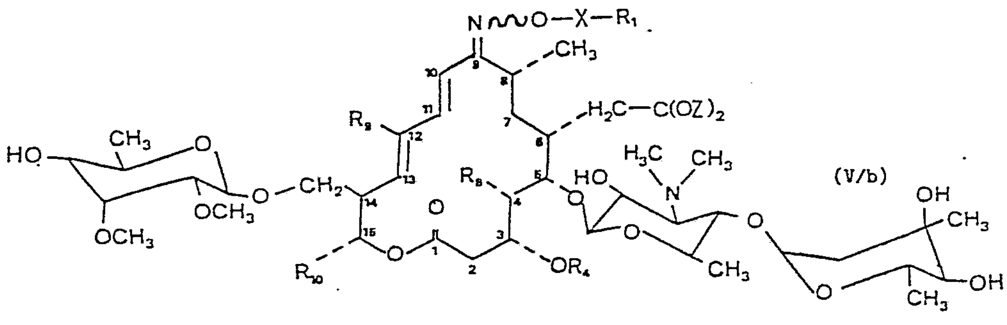
(V/b)

dans lequel le signe ∿∿ , X, $R_1$, $R_4$, $R_8$, $R_9$ et $R_{10}$ ont la même signification que dans la formule (I) et Z la même définition que précédemment,

- soit avec un sel d'acide fort de l'hydroxylamine pour obtenir un dérivé de formule (V/c) :

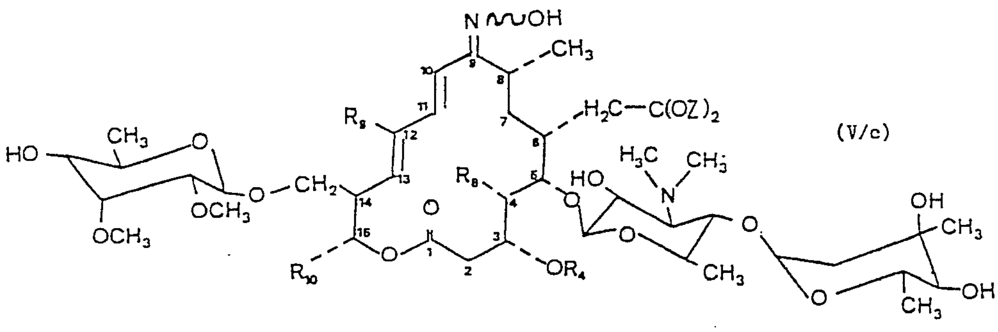

(V/c)

cas particulier de dérivés de formule (V/b), dans lequel le signe ∿ , $R_4$, $R_8$, $R_9$, $R_{10}$ et Z ont la même signification que dans la formule (V/b), X - R1 significant ici un atome d'hydrogène,
que l'on traite le cas échéant en présence d'une base, comme par exemple la triéthylamine ou la pyridine ou un sel de métal alcalin comme le carbonate de sodium ou le carbonate de potassium ou le carbonate de ou l'hydrogéno carbonate de sodium ou l'hydrogéno carbonate de potassium, ou un hydrure de métal alcalin tel que l'hydrure de sodium dans un solvant préférentiellement choisi parmi la pyridine, l'acétone ou le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V), dans lequel le signe ∿ , X, $R_1$, $R_4$, $R_8$, $R_9$ et $R_{10}$ ont la même signification que dans la formule (I) et Z la même signification que dans la formule (III/b),
qui, quel que soit le procédé selon lequel il a été obtenu, est soumis à un procédé de déprotection habituel de la fonction aldéhydique portée par le carbone hors cycle en β du carbone 6, comme par exemple, l'action d'une solution acide préférentiellement l'acide difluoracétique en solution dans un mélange de solvants tel que le mélange acétonitrile - eau (1/1, v/v) à température ambiante pour obtenir un composé de formule (I/g), cas particulier des composés de formule (I) :

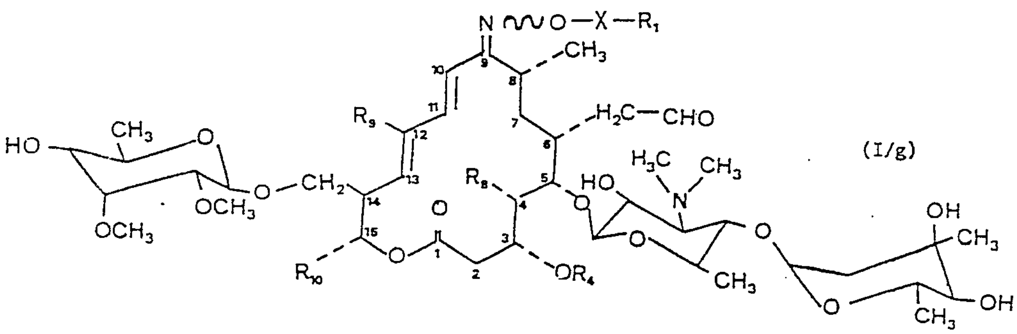

(I/g)

dans lequel le signe ∿ , X, $R_1$, $R_4$, $R_8$, $R_9$ et $R_{10}$ ont la même définition que dans la formule (I), A représentant ici un atome d'oxygène,
lequel, puisque $R_2$ ne signifie pas un atome d'hydrogène, peut au cours du processus de synthèse, en fonction des conditions opératoires sélectionnées ainsi que des valeurs des groupements X et $R_1$, avoir été partiellement hydrolysé en un dérivé (I/g) démycarosylé pour lequel $R_2$ = H, les deux dérivés ainsi obtenus (c'est-à-dire celui pour lequel $R_2$ = H et celui pour lequel $R_2$ correspond à un résidu mycarosé) étant alors aisément séparés par une technique classique de séparation classique de séparation comme

la chromatographie sur colonne de silice,
dérivé de formule (I/g),
que l'on peut, le cas échéant, traiter en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I),

- soit par un sel d'acide fort de l'hydroxylamine pour obtenir un produit de formule (I/h),

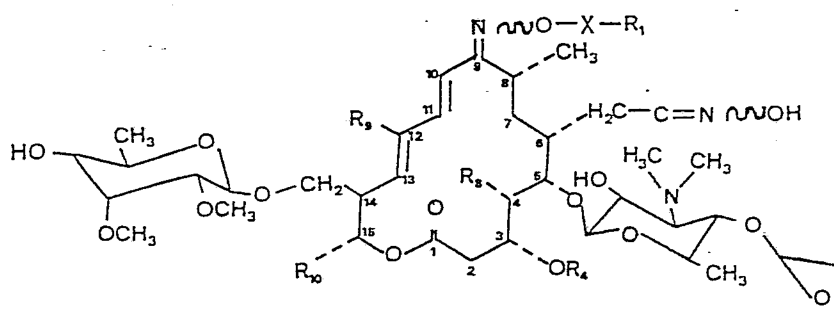
(I/h)

cas particulier de dérivés de formule (I) dans lequel le signe ∿ , X, $R_1$, $R_4$, $R_8$, $R_9$ et $R_{10}$ ont la même signification que dans la formule (I), A représentant ici un groupement N ∿ O - Y - $R_5$ dans lequel Y -$R_5$ représente un atome d'hydrogène,
qui est traité le cas échéant, en présence d'une base, comme par exemple la triéthylamine ou la pyridine ou d'un sel de métal alcalin comme le carbonate de sodium ou de carbonate de potassium ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T' (VIII)**

dans laquelle T' représente un atome d'halogène et $R_5$ et Y ont la même signification que dans la formule (I), à l'exclusion du cas où $R_5$ - Y représentent ensemble un atome d'hydrogène, pour obtenir un composé de formule (I/f),

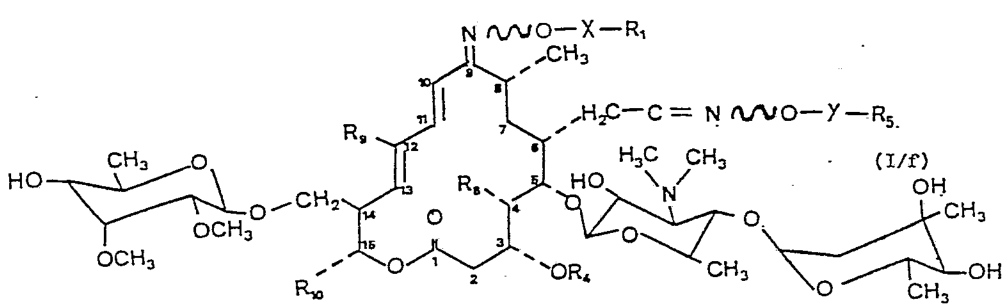
(I/f)

cas particulier de dérivés de formule (I) dans lequel le signe ∿ , X, $R_1$, $R_4$, $R_5$, $R_8$, $R_9$ et $R_{10}$ ont la

même signification que dans la formule (I),

- que l'on purifie éventuellement par chromatographie sur colonne de silice à l'aide d'un mélange solvent approprié comme par exemple un mélange chlorure de méthylène / méthanol,

et que l'on peut, si on le désire :

- soit salifier par un acide pharmaceutiquement acceptable,

- soit séparer en ses isomères puis, si nécessaire, salifier par un acide pharmaceutiquement acceptable.

28/ Procédé de préparation selon la revendication1, de composés de formule (I) dans lesquelles B et $R_9$ représentent simultanément un atome d'hydrogène appelés dérivés (I/k), caractérisé en ce que l'on utilise comme matière première un dérivé de formule (II/1) :

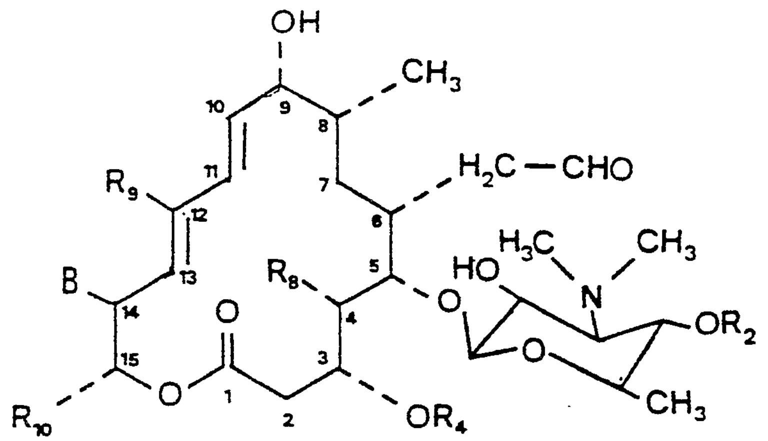

(II/1)

dans laquelle $R_2$, $R_4$, $R_8$ et $R_{10}$ ont la même définition que dans la formule (I), B et $R_9$ représentant simultanément chacun un atome d'hydrogène,

que l'on soumet à un agent d'oxydation comme par exemple le complexe trioxyde de soufre-pyridine en présence d'un agent basique comme une amine organique,

en milieu organique, préférentiellement choisi parmi diméthylsulfoxide, diméthylformamide, pyridine, pour obtenir un dérivé de formule (II/1a) :

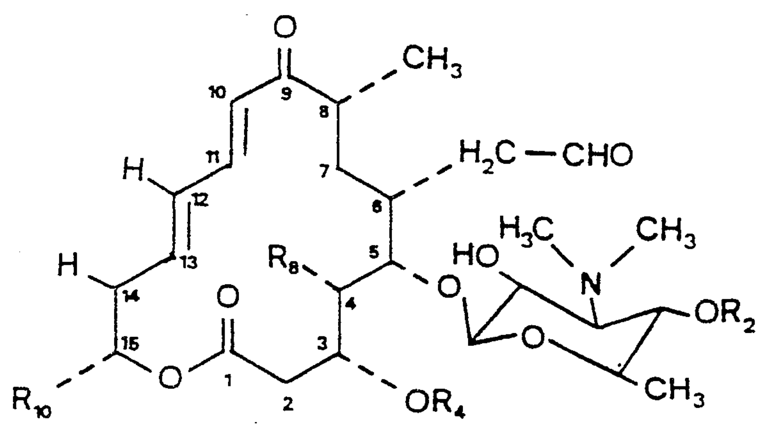

(II/1a)

dans laquelle $R_2$, $R_4$ et $R_{10}$ ont la même signification que dans la formule (II/1),

qui est alors condensé avec un alcool de formule Z - OH, dans lequel Z signifie un groupement alkyle inférieur, en présence d'un agent catalyseur acide comme l'acide paratoluène sulfonique, ou préférentiellement l'acide difluoroacétique lorsque le composé de formule (I/k) que l'on souhaite obtenir a un radial $R_2$ différent de H,

le milieu réactionnel étant ensuite soumis à l'action d'un agent basique, en particulier une amine comme la triéthylamine, pour obtenir, après extraction par un solvant organique, préférentiellement choisi parmi éther diéthylique, chloroforme, chlorure de méthylène et purification par chromatographie sur colonne de silice, un dérivé de formule (III/k) :

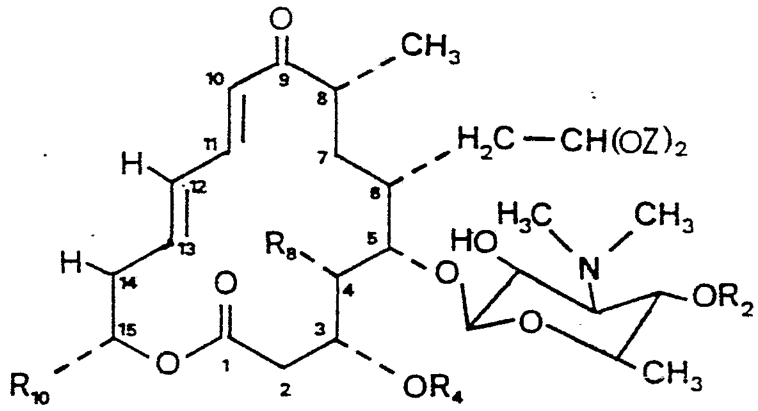

(III/k)

dans lequel $R_2$, $R_4$, $R_8$ et $R_{10}$ ont la même signification que dans la formule du dérivé (II/1a) et Z la même définition que précédemment,

que l'on condense en présence d'une base, comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N - O - X - R_1$ (IV)**

dans lequel X et $R_1$ ont la même signification que dans la formule (I) à l'exclusion du cas où $X - R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V/k) :

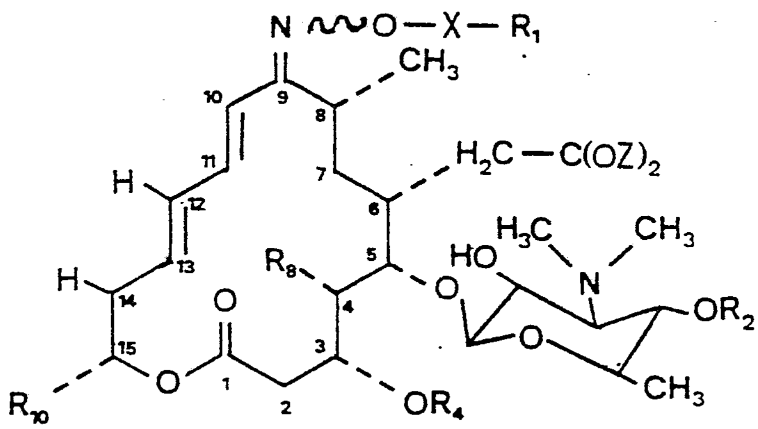

(V/k)

dans lequel le signe ∿∿ , X, $R_1$, $R_2$, $R_4$, $R_8$ et $R_{10}$ ont la même signification que dans la formule (I) et Z la même définition que précédemment,

- soit avec un sel d'acide fort de l'hydroxylamine pour obtenir un dérivé de formule (V/ak) :

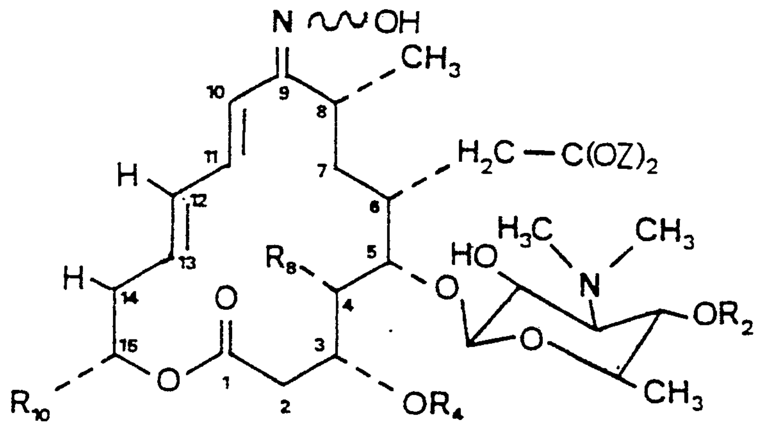

(V/ak)

cas particulier de dérivés de formule (V/k), dans lequel le signe ∿ , $R_2$, $R_4$, $R_8$, $R_{10}$ et Z ont la même signification que dans la formule (V/k), X - $R_1$ significant ici un atome d'hydrogène,
que l'on traite le cas échéant en présence d'une base, comme par exemple la triéthylamine, ou la pyridine, ou un sel de métal alcalin comme le carbonate de sodium, ou le carbonate de potassium, ou le carbonate de calcium, ou l'hydrogéno carbonate de sodium, ou l'hydrogéno carbonate de potassium, ou un hydrure de métal alcalin tel que l'hydrure de sodium, dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V/k), dans lequel le signe ∿ , X, $R_1$, $R_2$, $R_4$, $R_8$ et $R_{10}$ ont la même signification que dans la formule (II/1) et Z la même signification que dans la formule (III/k),
qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,1 N et 0,5 N, préférentiellement comprise entre 0,02 N et 0,2 N, préférentiellement comprise entre 0,05 N et 0,5 N, préférentiellenment comprise 0,75 N et 0,25 N et à température ambiante, ou préférentiellement l'acide difluoroacétique en solution dans un mélange de solvants tel que le mélange acétonitrile - eau (1/1, v/v) lorsque le composé de formule (I) que l'on désire obtenir possède un radical $R_2$ différente de l'atome d'hydrogène,
pour obtenir un composé de formule (I/ak) :

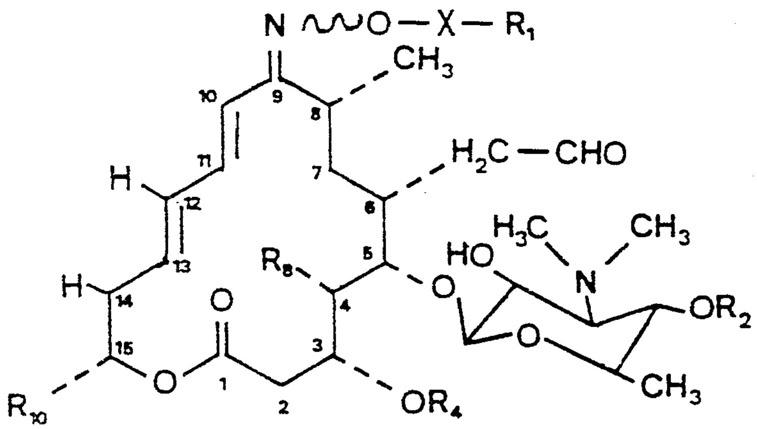

(I/ak)

cas particulier des composés de formule (I) dans laquelle le signe ∿ , X, $R_1$, $R_2$, $R_4$, $R_8$ et $R_{10}$ ont la même définition que dans la formule (I), A représentant ici un atome d'oxygène,
lequel, dans le cas où dans la matière première utilisée $R_2$ ne signifie pas un atome d'hydrogène, peut au cours du processus de synthèse, en fonction des conditions opératoires sélectionnées ainsi que des valeurs des groupements X, $R_1$, $R_8$ et $R_{10}$, avoir été partiellement hydrolysé en un dérivé (I/ak) démycarosylé pour lequel $R_2$ = H, les deux dérivés ainsi obtenus (c'est-à-dire celui pour lequel $R_2$ = H

et celui pour lequel $R_2$ correspond à un résidu mycarosylé) étant alors aisément séparés par une technique classique de séparation comme la chromatographie sur colonne de silice,
ce dérivé de formule (I/ak) pouvant alors, le cas échéant, être traité en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium,
- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I/k),
- soit par un sel d'acide fort de l'hydroxylamine pour obtenir un produit de formule (I/bk),

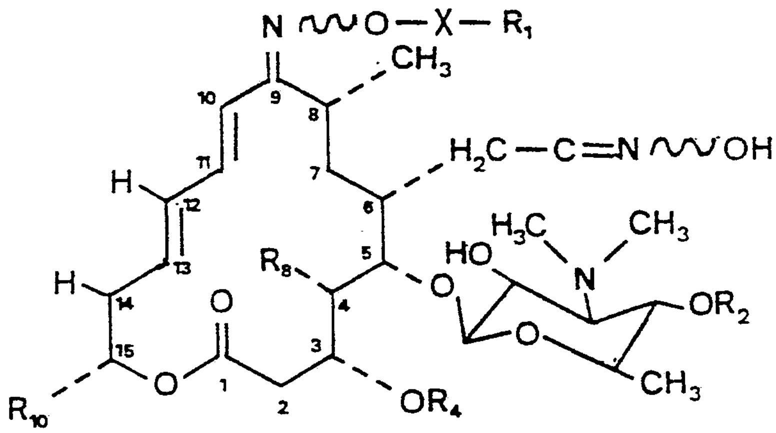

(I/bk)

cas particulier de dérivés de formule (I/k) dans lequel le signe ∿ , X, $R_1$, $R_2$, $R_4$, $R_8$ et $R_{10}$ ont la même signification que dans la formule (I), A représentant ici un groupement N ∿ O - Y - $R_5$ dans lequel Y -$R_5$ représente un atome d'hydrogène,
qui est traité le cas échéant, en présence d'une base, comme par exemple la triéthylamine, ou la pyridine, ou d'un sel de métal alcalin comme le carbonate de sodium ou de carbonate de potassium, ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T' (VIII)**

dans laquelle T' représente un atome d'halogène et $R_5$ et Y ont la même signification que dans la formule (I), à l'exclusion du cas où $R_5$ - Y représentent ensemble un atome d'hydrogène, pour obtenir un composé de formule (I),
- que l'on purifie éventuellement par chromatographie sur colonne de silice à l'aide d'un mélange solvant approprié comme par exemple un mélange chlorure de méthylène / méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

29/ Composés de structure générale (V) selon la revendication 25 utiles pour la préparation des composés selon la revendication 1.

30/ Composition pharmaceutique contenant comme principe actif au moins un composé selon l'une des revendications 1 à 24 en combinaison avec un ou plusieurs excipients ou véhicules inertes, non-toxiques, pharmaceutiquement acceptables.

31/ Composition pharmaceutique selon la revendication 30, contenant au moins un principe actif selon l'une des revendications 1 à 24 utilisables dans le traitement des maladies justifiant une antibiothérapie à large spectre.

**Revendications pour l'Etat contractant suivant: ES**

1/ Procédé de préparation des composés de formule générale :

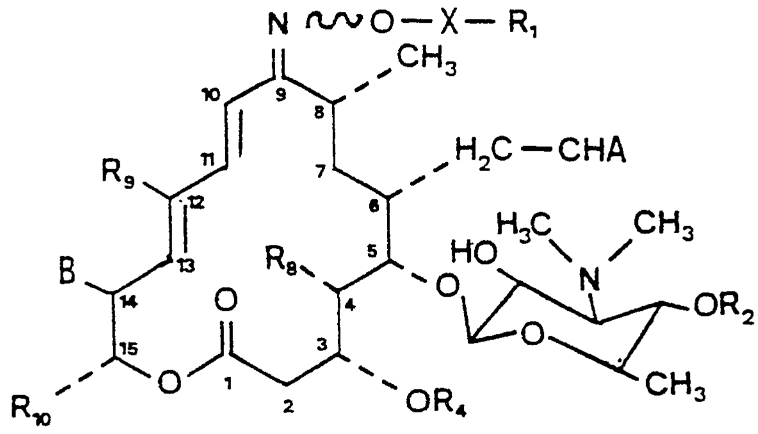

(I)

dans laquelle :

- A représente . soit un atome d'oxygène,

. soit un groupement de formule N ∿ O - Y - $R_5$, le signe ∿ existant sur le substituant du carbone 9 ainsi que dans la définition de A signifiant que l'une ou l'autre indépendamment des fonctions oxime ou éther d'oxime peut chacune se trouver sous la forme syn ou anti ou sous forme du mélange syn et anti,

- X et Y identiques ou différents représentent soit un radical alkyle comprenant au plus dix atomes de carbone, linéaire ou ramifié, soit un radical alkényle comprenant au plus dix atomes de carbone, linéaire ou ramifié, soit un radical alkynyle comprenant au plus dix atomes de carbone, linéaire ou ramifié, chacun des substituants alkyle, alkényle ou alkynyle pouvant être éventuellement substitué par un ou plusieurs groupements choisis parmi hydroxy, alkyle inférieure linéair ou ramifié, alkényle inférieur linéaire ou ramifié, alkynyle inférieur linéaire ou ramifié, alkényloxy inférieur linéaire ou ramifié, alkénylthio inférieur linéaire ou ramifié, alkynyloxy inférieur linéaire ou ramifié, alkynylthio inférieur linéaire ou ramifié, fluoro, chloro, bromo, iodo, amino, dialkylamino inférieur,

- $R_1$ et $R_5$ identiques ou différents représentent :

- soit un atome d'hydrogène.

- soit un radical alkyloxy linéaire ou ramifié comprenant au plus dix atomes de carbone, un radical alkylthio linéaire ou ramifié comprenant au plus dix atomes de carbone, un radical alkényloxy linéaire ou ramifié comprenant au plus dix atomes de carbone, un radical alkénylthio linéaire ou ramifié comprenant au plus dix atomes de carbone, un radical alkynyloxy linéaire ou ramifié comprenant au plus dix atomes de carbone, un radical alkynylthio linéaire ou ramifié comprenant au plus dix atomes de carbone, un radical aryle (citons à titre d'exemple phényle, pyridyle, thiényle, furyle, benzothiényle, benzofuryle, indolyle, thiazolyle, oxazolyle, pyrrolyle, imidazolyle, pyrazolyle, isothiazolyle, isoxazolyle, benzimidazolyle), un radical aryloxy, arylthio, aralkyloxy, aralkylthio, chacun des radicaux alkyloxy, alkylthio, alkényloxy, alkénylthio, alkynyloxy, alkynylthio, aryle, aryloxy, arylthio, aralkyloxy, aralkylthio étant éventuellement substitué par un ou plusieurs des radicaux choisis parmi hydroxy, alkyle inférieur, alkényle inférieur, alkynyle inférieur, alkyloxy inférieur, alkényloxy inférieur, alkynyloxy inférieur, alkylthio inférieur, alkénylthio inférieur, alkynylthio inférieur, fluoro, chloro, bromo, iodo, nitro, amino, dialkkylamino inférieur, ou un radical N $R_6$ $R_7$ ci-dessous défini,

- soit un radical

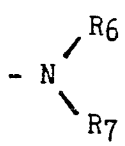

dans lequel $R_6$ et $R_7$ identiques ou différents représentent un atome d'hydrogène, un radical alkyle inférieur, ou alkényle inférieur ou alkynyle inférieur, ou forment avec l'azote un système hétérocyclique saturé ou non, comprenant éventuellement un autre hétéroatome et éventuellement substitué par un atome d'halogène ou un radical alkyle inférieur,

- un atome de chlore, de brome, d'iode ou de fluor,

- un groupement carboxylique libre ou salifié par une base minérale (KOH, NaOH, $Ca(OH)_2$) ou organique (triéthylamine, diéthylamine...) ou estérifié par un alcool aliphatique de formule $R_8OH$ où $R_8$ est un groupement alcoyle inférieur,

- ou X - $R_1$ et Y - $R_5$ indépendamment l'un de l'autre représentent chacun un atome d'hydrogène,

- $R_2$ représente . soit un atome d'hydrogène,
. soit un radical de formule :

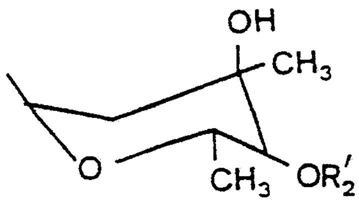

dans lequel $R'_2$ représente un atome d'hydrogène, un radical alkyle ou un radical acyle inférieur, linéaire ou ramifié,

- B représente . soit un atome d'hydrogène,
. soit un radical de formule : - $CH_2$ - O - B', où B' représente :
. soit un atome d'hydrogène,
. soit un radical de formule :

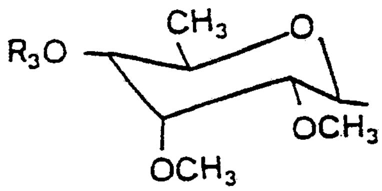

dans lequel $R_3$ représente un atome d'hydrogène ou un radical alkyle inférieur linéaire ou ramifié,

- $R_4$ représente . soit un atome d'hydrogène,
. soit un radical alkyle inférieur linéaire ou ramifié,
. soit un radical acyle inférieur linéaire ou ramifié,

- $R_8$ représente . soit un radical alkyle inférieur préférentiellement méthyle lorsque B représente un groupement $CH_2$ - O - B',
. soit un radical alkyloxy inférieur préférentiellement méthoxy lorsque B représente un atome d'hydrogène,

- $R_9$ représente . soit un atome d'hydrogène lorsque B représente un atome d'hydrogène,
. soit un radical alkyle inférieur préférentiellement méthyle lorsque B représente un radical $CH_2$ - O - B',

- $R_{10}$ représente un radical alkyle inférieur,

le terme inférieur concernant les radicaux alkyle inférieur, alkyloxy inférieur, alkylthio inférieur, alkényle inférieur, alkényloxy inférieur, alkénylthio inférieur, alkynyle inférieur, alkynyloxy inférieur, alkynylthio inférieur, signifiant qu'il s'agit de groupements comprenant entre 1 et 6 atomes de carbone,
et leurs sels d'addition avec un acide,
caractérisé en ce que l'on utilise comme matière première :

- soit lorsque B représente un atome d'hydrogène, un dérivé de formule (II/1) :

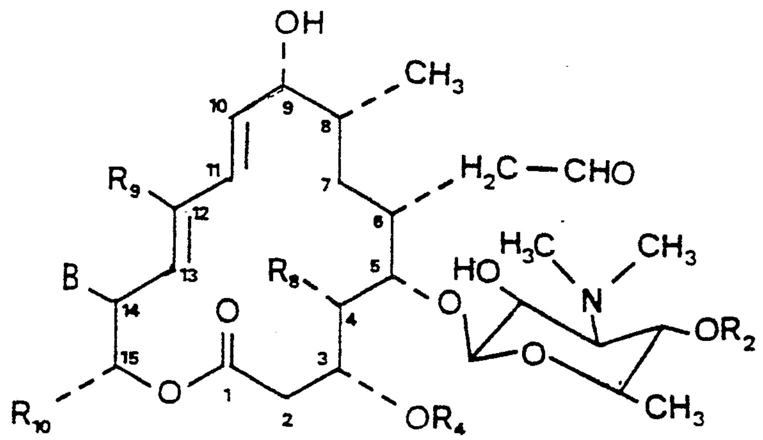

(II/1)

dans laquelle $R_2$, $R_4$, $R_8$ et $R_{10}$ ont la même signification que dans la formule (I), B et $R_9$ représentent simultanément un atome d'hydrogène, que l'on soumet à un agent d'oxydation comme par exemple le complexe trioxyde de soufre-pyridine en présence d'un agent basique comme une amine organique, en milieu organique, préférentiellement choisi parmi diméthylsulfoxide, diméthylformamide, pyridine, pour obtenir un dérivé de formule (II/1a) :

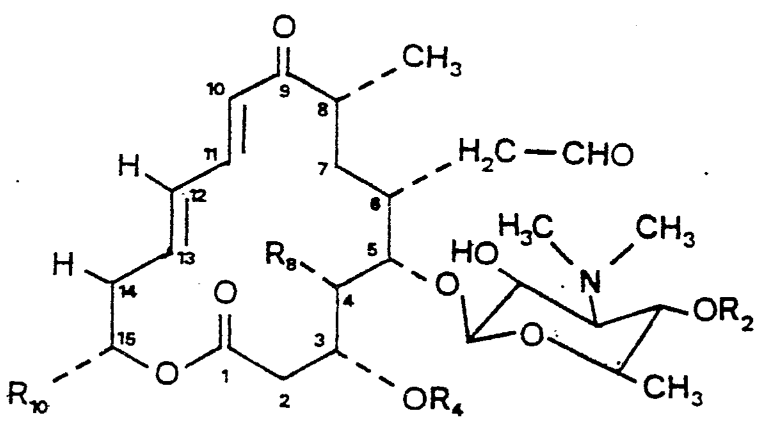

(II/1a)

dans laquelle $R_2$, $R_4$, $R_8$ et $R_{10}$ ont la même signification que dans la formule (I), soit, lorsque dans le composé de formule (I) que l'on souhaite obtenir, B et $R_9$ ne représentent pas simultanément chacun un atome d'hydrogène, un dérivé de formule (II/2) :

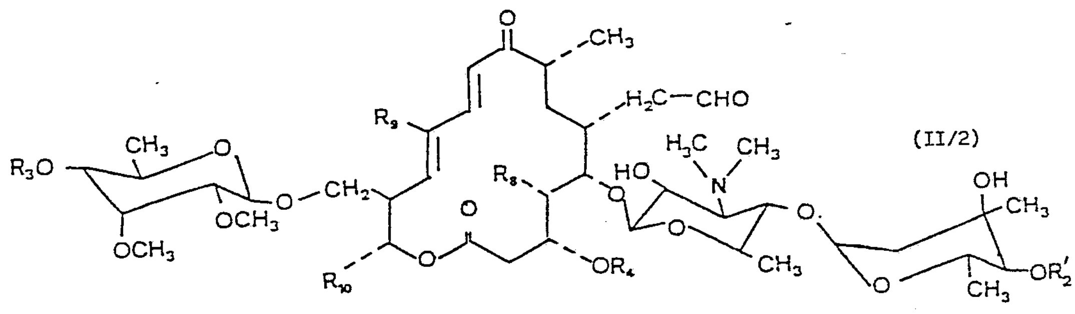

(II/2)

dans laquelle $R'_2$, $R_3$, $R_4$ et $R_{10}$ ont la même définition que dans la formule (I), et $R_8$ et $R_9$ signifient un radical alkyle inférieur préférentiellement méthyle,
qui, lorsque dans le composé de formule (I) que l'on souhaite obtenir B' et $R_2$ représentent simultanément un atome d'hydrogène est tout d'abord soumis à l'action de l'acide chlorhydrique dilué de normalité comprise entr 0,05 et 0,4 préférentiellement comprise entre 0,1 et 0,30, préférentiellement comprise entre 0,15 et 0,25 à température ambiante pour conduire après lavage par un solvant organique approprié, alcalinisation et extraction par un solvant organique approprié à un dérivé de formule (II/2a) :

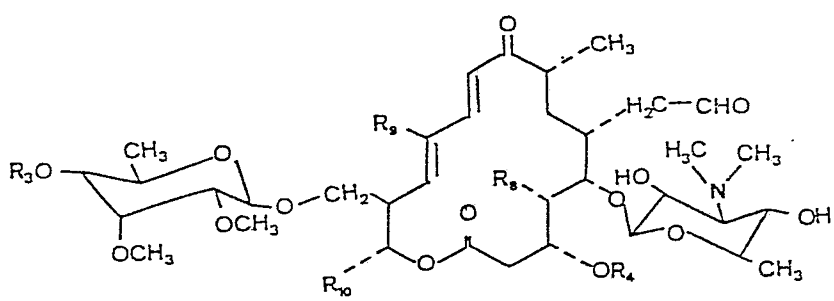

(II/2a)

dans laquelle $R_3$, $R_4$ et $R_{10}$ ont la même signification que dans la formule (I), et $R_8$ et $R_9$ la même signification que dans la formule (II/2),
que l'on fait réagir avec l'acide chlorhydrique dilué de normalité comprise entre 0,25 et 0,75 ; préférentiellement entre 0,3 et 0,7 ; préférentiellement entre 0,4 et 0,6 ; à une température comprise préférentiellement entre 30 et 100 ° C ; préférentiellement comprise entre 50 et 90 ° C ; préférentiellement entre 70 et 80 ° C, pour conduire, après lavage par un solvant organique approprié, alcalinisation et extraction par un solvant organique approprié à un dérivé de formule (II/2b) :

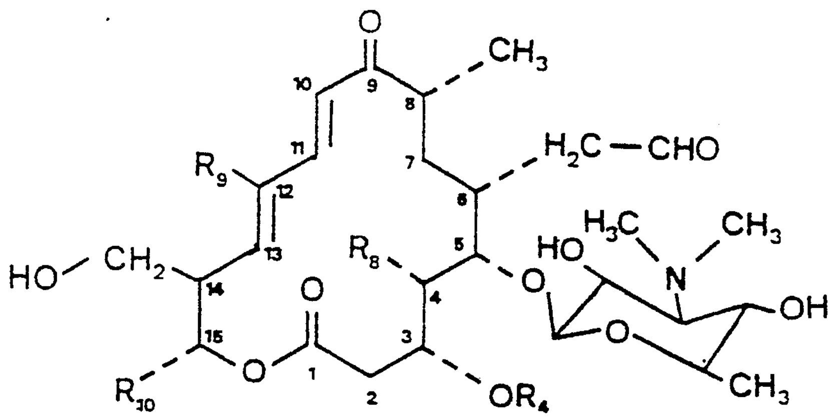

(II/2b)

dans laquelle $R_4$ et $R_{10}$ ont la même signification que dans la formule (I), et $R_8$ et $R_9$ la même signification que dans la formule (II/2)
que l'on purifie éventuellement par chromatographie sur colonne de silice,
le dérivé alors choisi de formule générale (II/1a), (II/2), ou (II/2b) selon le composé de formule (I) que l'on souhaite obtenir, étant alors condensé avec un alcool de formule Z - OH, dans lequel Z signifie un groupement alkoyle inférieur, en présence d'un agent catalyseur acide comme l'acide paratoluène sulfonique ou préférentiellement l'acide difluoroacétique lorsque le composé de formule (I) que l'on souhaite obtenir a un radical $R_2$ différent H,
le milieu réactionnel étant ensuite soumis à l'action d'un agent basique, en particulier une amine comme la triéthylamine, pour obtenir, après extraction par un solvant organique préférentiellement choisi parmi éther diéthylique, chloroforme, chlorure de méthylène et purification par chromatographie sur colonne de silice, un dérivé de formule (III) :

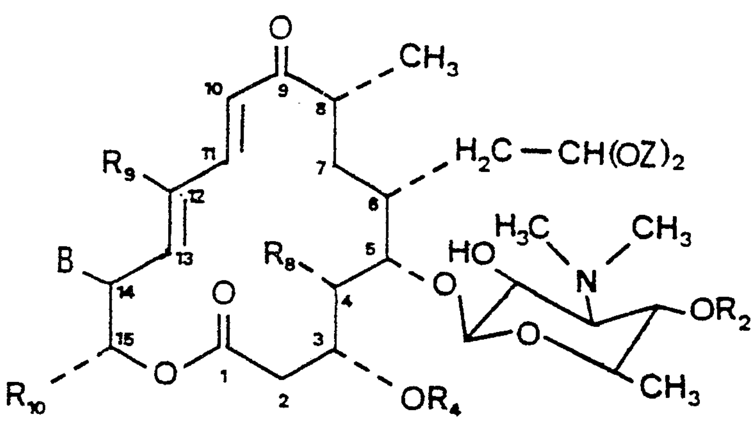

(III)

dans lequel B, $R_2$, $R_4$, $R_8$, $R_9$ et $R_{10}$ ont la même signification que dans la formule de dérivé choisi (II/1a), (II/2) ou (II/2b) et Z la même définition que précédemment,
que l'on condense en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium,

- soit avec un dérivé de formule (IV) :

**$H_2N$ - O - X - $R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V) :

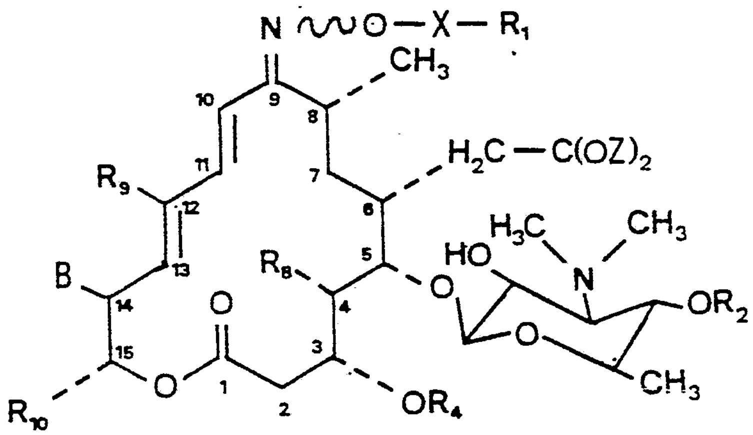

(V)

dans lequel le signe ∿ , X, $R_1$, $R_2$, $R_4$, $R_8$, $R_9$, $R_{10}$ et B ont la même signification que dans la formule (I) et Z la même définition que précédemment,

- soit avec un sel d'acide fort de l'hydroxylamine pour obtenir un dérivé de formule (V/a) :

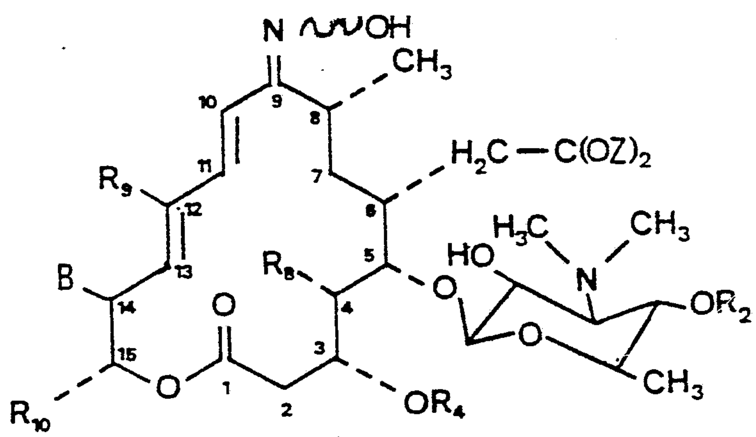

(V/a)

cas particulier de dérivés de formule (V), dans lequel le signe ∿ , B, $R_2$, $R_4$, $R_8$, $R_9$, $R_{10}$ et Z ont la même signification que dans la formule (V), X - $R_1$ significant ici un atome d'hydrogène, que l'on traite le cas échéant en présence d'une base, comme par exemple la triéthylamine ou la pyridine ou un sel de métal alcalin comme le carbonate de sodium ou le carbonate de potassium ou le carbonate de calcium ou l'hydrogéno carbonate de sodium ou l'hydrogéno carbonate de potassium, ou un hydrure de métal alcalin tel que l'hydrure de sodium dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V), dans lequel le signe ∿ , X, $R_1$, $R_2$, $R_4$, $R_8$, $R_9$, $R_{10}$ et B ont la même signification que dans la formule (I) et Z la même signification que dans la formule (III),

qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,01N et 0,5N, préférentiellement comprise entre 0,02N et 0,2N, préférentiellement comprise entre 0,05N et 0,5N, préférentiellement comprise entre 0,075N et 0,25N à température ambiante,

ou préférentiellement l'acide difluoroacétique en solution dans un mélange de solvants tel que le mélange acétonitrile - eau (1/1, v/v) lorsque le composé de formule (I) que le composé de formule (I) que l'on désire obtenir, possède un radical $R_2$ différent de l'atome d'hydrogène,

pour obtenir un composé de formule (I/a), cas particulier des composés de formule (I) :

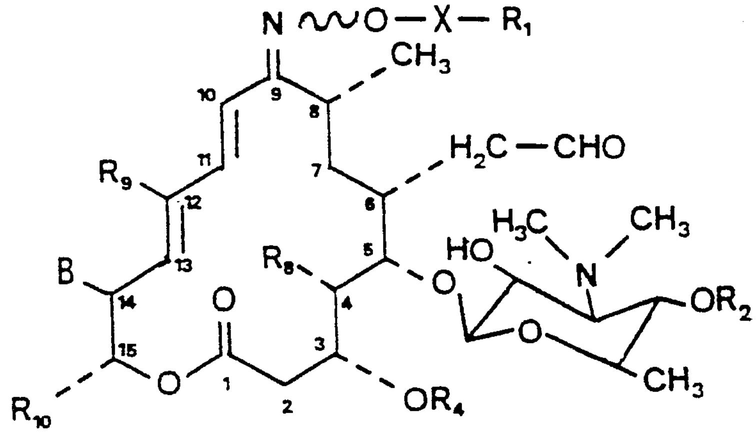

(I/a)

dans lequel le signe ∿∿ , X, $R_1$, $R_2$, $R_4$, $R_8$, $R_9$, $R_{10}$ et B ont la même définition que dans la formule (I), A représentant ici un atome d'oxygène,

lequel, dans le cas où dans la matière première utiisée $R_2$ ne signifie pas un atome d'hydrogène, peut au cours du processus de synthèse, en fonction des conditions opératoires sélectionnées ainsi que des valeurs des groupements X, $R_1$, $R_8$, $R_9$, $R_{10}$ et B, avoir été partiellement hydrolysé en un dérivé (I/a) démycarosylé pour lequel $R_2$ = H, les deux dérivés ainsi obtenus (c'est-à-dire celui pour lequel $R_2$ = H et celui pour lequel $R_2$ correspond à un résidu mycarosé) étant alors aisément séparés par une technique classique de séparation comme la chromatographie sur colonne de silice,

ce dérivé de formule (I/a) peut, le cas échéant, être traiter en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium,

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I),

- soit par un sel d'acide fort de l'hydroxylamine pour obtenir un produit de formule (I/b),

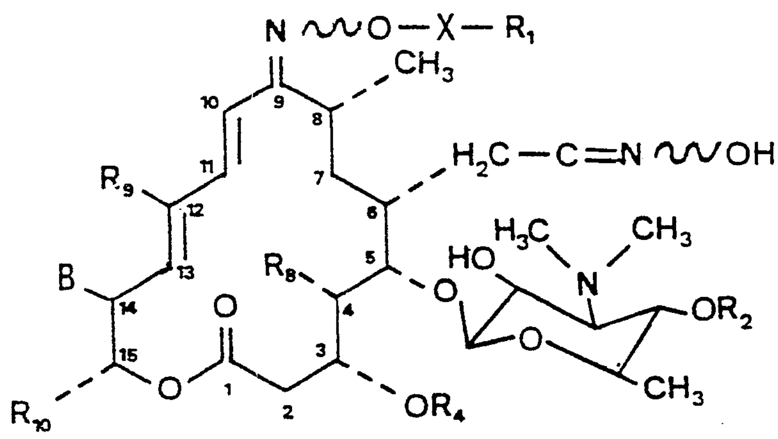

(I/b)

cas particulier de dérivés de formule (I) dans lequel le signe ∿∿ , X, $R_1$, $R_2$, $R_4$, $R_8$, $R_9$, $R_{10}$ et B ont la même signification que dans la formule (I), A représentant ici un groupement N ∿∿ O - Y - $R_5$ dans lequel Y - $R_5$ représente un atome d'hydrogène,

qui est traité le cas échéant, en présence d'une base, comme par exemple la triéthylamine, ou la pyridine, ou d'un sel de métal alcalin comme le carbonate de sodium ou de carbonate de potasium, ou l'hydrogéno carbonate de sodium ou l'hydrogéno carbonate de potasium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T′ (VIII)**

dans laquelle T′ représente un atome d'halogène et $R_5$ et Y ont la même signification que dans la formule

(I), à l'exclusion du cas où $R_5$ - Y représentent ensemble un atome d'hydrogène, pour obtenir un composé de formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

2/ Procédé de préparation selon la revendication 1 des dérivés de formule (I) dans lesquels Y - $R_5$ a la même signification que X - $R_1$ ou dérivés (IX), caractérisé en ce que l'on condense un dérivé de formule (II/1a) ou (II/2) ou (II/2a) ou (II/2b) selon la formule du composé de formule (I) que l'on souhaite obtenir :

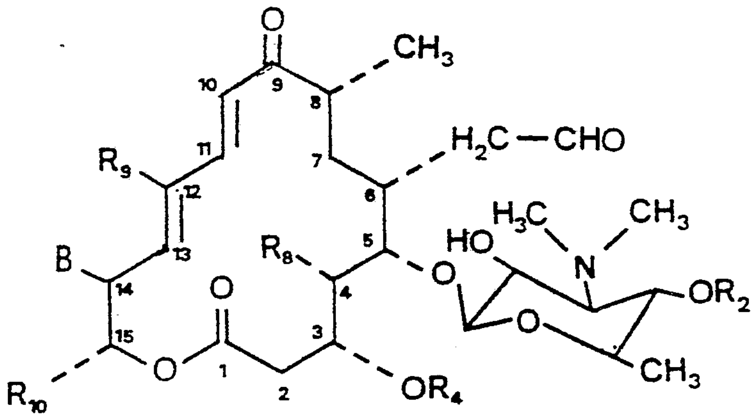

(II)

dans laquelle $R_2$, $R_4$, $R_8$, $R_9$, $R_{10}$ et B ont la même définition que celle correspondant à chacune des formules (II/1a), (II/2) ou (II/2b), dans la formule (I),en présence d'une base comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N$ - O - X - $R_1$ (IV)**

dans lequel X - $R_1$ a la même signification que dans la formule (I) à l'exception du cas où X - $R_1$ représente un atome d'hydrogène.

pour obtenir un produit de formule (IX) :

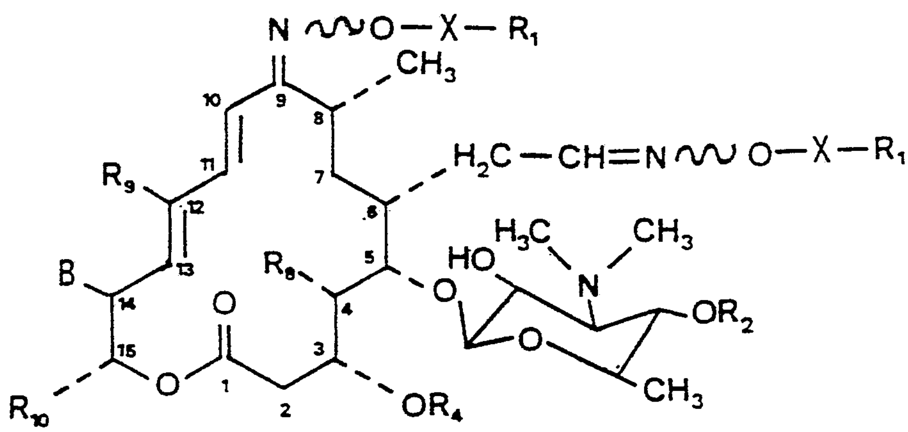

(IX)

cas particulier des dérivés de formule (I) dans laquelle le signe ∿ , X, $R_1$, $R_2$, $R_4$, $R_8$, $R_9$, $R_{10}$ et B ont la même signification que dans la formule (I),

- soit avec un sel d'acide fort de l'hydroxylamine pour obtenir un dérivé de formule (IX/a) :

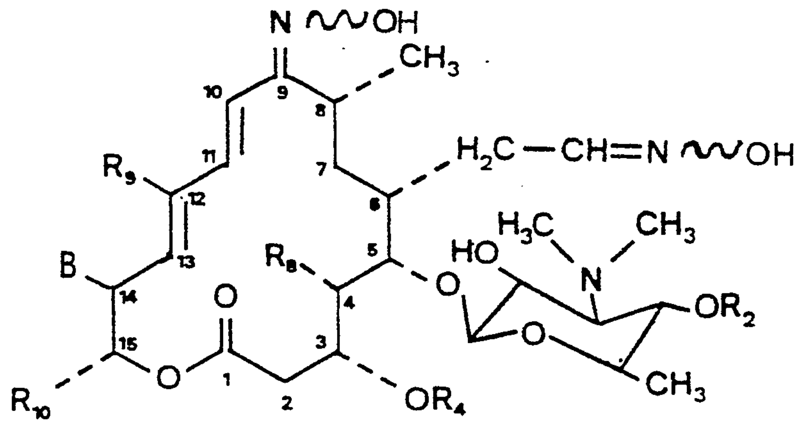

(IX/a)

cas particulier de dérivés de formule (I) et (IX), dans lequel le signe ∿ , $R_2$, $R_4$, $R_8$, $R_9$, $R_{10}$ et B ont la même signification que dans la formule I, X - $R_1$ et Y - $R_5$ représentant ici ensemble un atome d'hydrogène,
que l'on traite éventuellement en présence d'une base, comme par exemple la triéthylamine, la pyridine ou un sel de métal alcalin comme le carbonate de sodium ou le carbonate de potassium ou l'hydrogéno carbonate de sodium ou l'hydrogéno carbonate de potassium, ou un hydrure de métal alcalin tel que l'hydrure de sodium, dans un solvant préférentiellement choisi parmi l'acétone ou le diméthyl formamide, le dioxanne, l'acétontrile, le tétrahydrofuranne, l'éther diéthylique ou diisopropylique, par un dérivé de formule (X) :

**$R_1$ - X - T″ (X)**

dans lequel T″ représente un atome d'halogène et $R_1$ et X ont la même signification que dans la formule (I), à l'exclusion du cas où $R_1$ et X forment ensemble un atome d'hydrogène, pour obtenir un produit de formule (IX) cas particulier des dérivés de formule (I), dans lequel le signe ∿ , X, $R_1$, $R_2$, $R_4$, $R_8$, $R_9$, $R_{10}$ B et X ont la même signification que dans la formule (I),
que l'on purifie éventuellement par chromatographie sur colonne de silice à l'aide d'un mélange solvant approprié comme par exemple un mélange chlorure de méthylène / méthanol,
et que l'on peut, so on le désire :

- soit salifier par un acide pharmaceutiquement acceptable,
- soit séparer en ses isomères puis, si nécessaire, salifier par un acide pharmaceutiquement acceptable.

3/ Procédé de préparation selon la revendication 1 de composés de formule (I/f), cas particulier des dérivés de formule (I) dans lesquels :

B représente un groupement - $CH_2$ - O - B′ où B′ représente un groupement de formule :

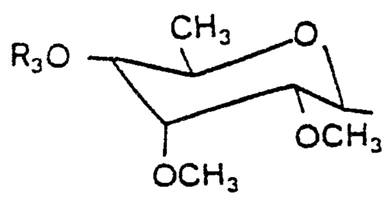

$R_2$ un groupe de formule :

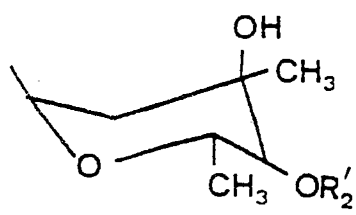

cas particulier où $R'_2$ et $R_3$ représentent un atome d'hydrogène, et A, X, R, $R_4$, $R_8$, $R_9$ et $R_{10}$ ont la même signification que dans la formule (I),

caractérisé en ce que l'on fait agir sur la tylosine un alcool aliphatique inférieur Z - OH, préférentiellement le méthanol, en présence d'un agent acide comme par exemple l'acide difluoroacétique,
le milieu réactionnel étant ensuite soumis à l'action d'un agent basique, en particulier une amine organique appropriée (préférentiellement le dichlorométhane) et purification par chromatographie sur colonne de silice un dérivé de formule (III/b) :

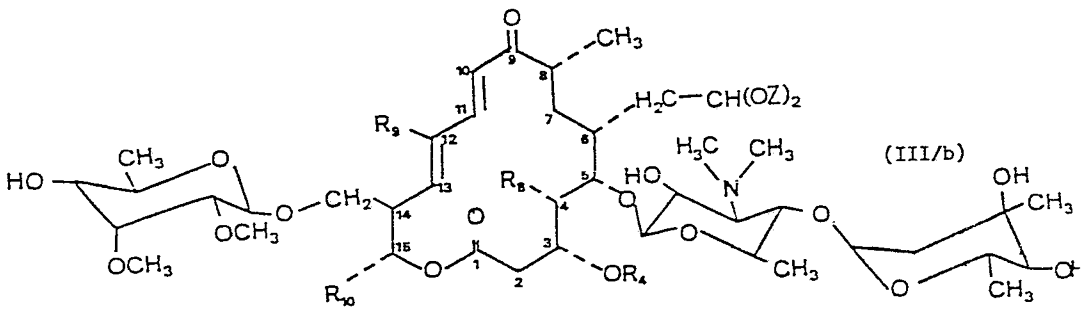

(III/b)

dans lequel $R_4$, $R_8$, $R_9$ et $R_{10}$ ont la même signification que dans la formule (I),
et Z représente un groupement alkyl inférieur,
que l'on condense en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium,

- soit avec un dérivé de formule (IV) :

**$H_2N$ - O - X - $R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V/b) :

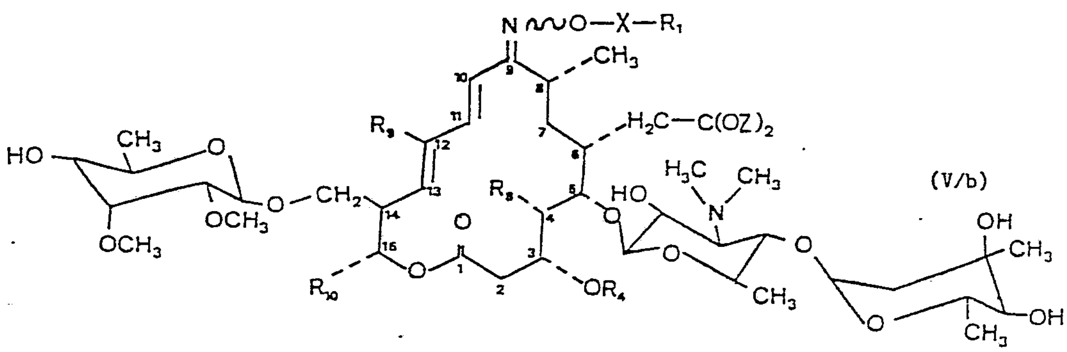

(V/b)

dans lequel le signe ∿∿ , X, $R_1$, $R_4$, $R_8$, $R_9$ et $R_{10}$ ont la même signification que dans la formule (I) et Z la même définition que précédemment,

- soit avec un sel d'acide fort de l'hydroxylamine pour obtenir un dérivé de formule (V/c) :

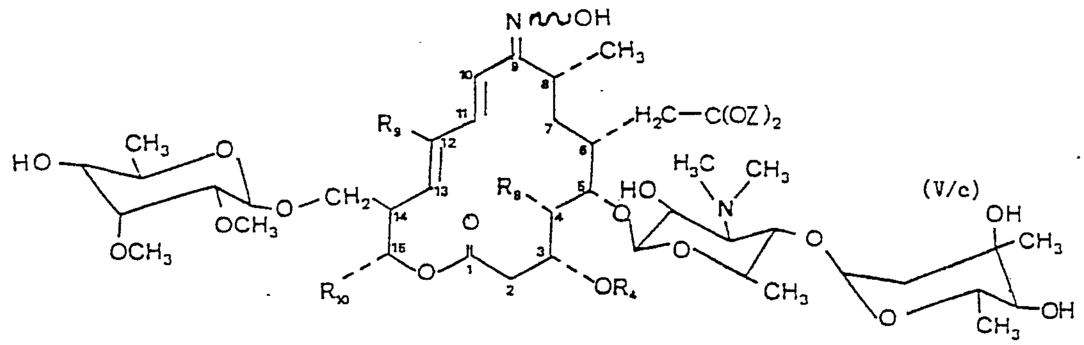

(V/c)

cas particulier de dérivés de formule (V/b), dans lequel le signe ∿ , $R_4$, $R_8$, $R_9$, $R_{10}$ et Z ont la même signification que dans la formule (V/b), X - R1 signifiant ici un atome d'hydrogène,
que l'on traite le cas échéant en présence d'une base, comme par exemple la triéthylamine ou la pyridine ou un sel de métal alcalin comme le carbonate de sodium ou le carbonate de potassium ou le carbonate de calcium ou l'hydrogéno carbonate de sodium ou l'hydrogéno carbonate de potassium, ou un hydrure de métal alcalin tel que l'hydrure de sodium dans un solvant préférentiellement choisi parmi la pyridine, l'acétone ou le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V), dans lequel le signe ∿ , X, $R_1$, $R_4$, $R_8$, $R_9$ et $R_{10}$ ont la même signification que dans la formula (I) et Z la même signification que dans la formule (III/b),
qui, quel que soit le procédé selon lequel il a été obtenu, est soumis à un procédé de déprotection habituel de la fonction aldéhydique portée par le carbone hors cycle en β du carbone 6, comme par exemple, l'action d'une solution acide préférentiellement l'acide difluoroacétique en solution dans un mélange de solvants tel que le mélange acétonitrile - eau (1/1, v/v) à température ambiante pour obtenir un composé de formule (I/g), cas particulier des composés de formule (I) :

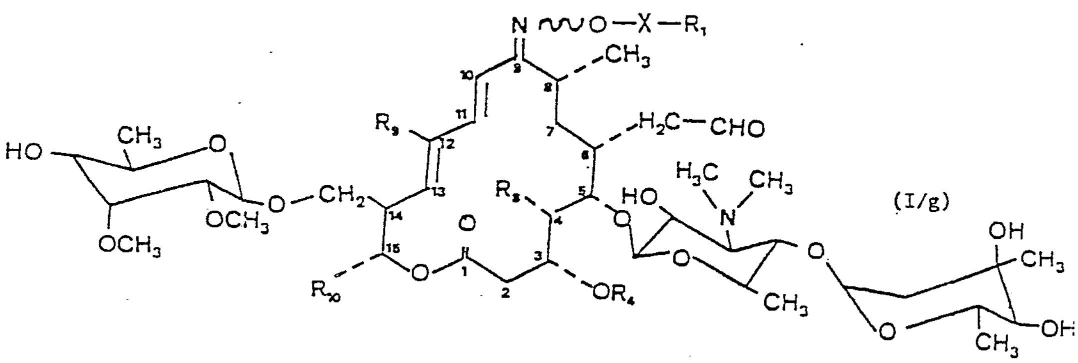

(I/g)

dans lequel le signe ∿ , X, $R_1$, $R_4$, $R_8$, $R_9$ et $R_{10}$ ont la même définition que dans la formule (I), A représentant ici un atome d'oxygène,
lequel, puisque $R_2$ ne signifie pas un atome d'hydorgène, peut au cours du processus de synthèse, en fonction des conditions opératoires sélectionnées ainsi que des valeurs des groupements X et $R_1$, avoir été partiellement hydrolysé en un dérivé (I/g) démycarosylé pour lequel $R_2$ = H, les deux dérivés ainsi obtenus (c'est-à-dire celui pour lequel $R_2$ = H et celui pour lequel $R_2$ correspond à un résidu mycarosé) étant alors aisément sépaés par une technique classique de séparation classique de séparation comme la

chromatographie sur colonne de silice,
dérivé de formule (I/g),
que l'on peut, le cas échéant, traiter en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium

- soit par un dérivé de formule (VII) :

**$H_2N - O - Y - R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I),

- soit par un sel d'acide fort de l'hydroxylamine pour obtenir un produit de formule (I/h) :

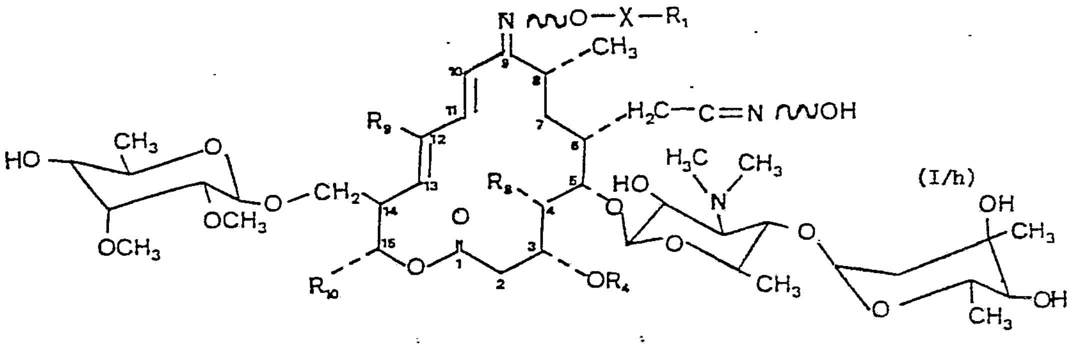
(I/h)

cas particulier de dérivés de formule (I) dans lequel le signe ∿ , X, $R_1$, $R_4$, $R_8$, $R_9$ et $R_{10}$ ont la même signification que dans la formule (I), A représentant ici un groupement N ∿ O - Y - $R_5$ dans lequel Y -$R_5$ représente un atome d'hydrogène,
qui est traité le cas échéant, en présence d'une base, comme par exemple la triéthylamine ou la pyridine ou d'un sel de métal alcalin comme le carbonate de sodium ou de carbonate de potassium ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5 - Y - T'$ (VIII)**

dans laquelle T' représente un atome d'halogène et $R_5$ et Y ont la même signification que dans la formule (I), à l'exclusion du cas où $R_5$ - Y représentent ensemble un atome d'hydrogène, pour obtenir un composé de formule (I/f),

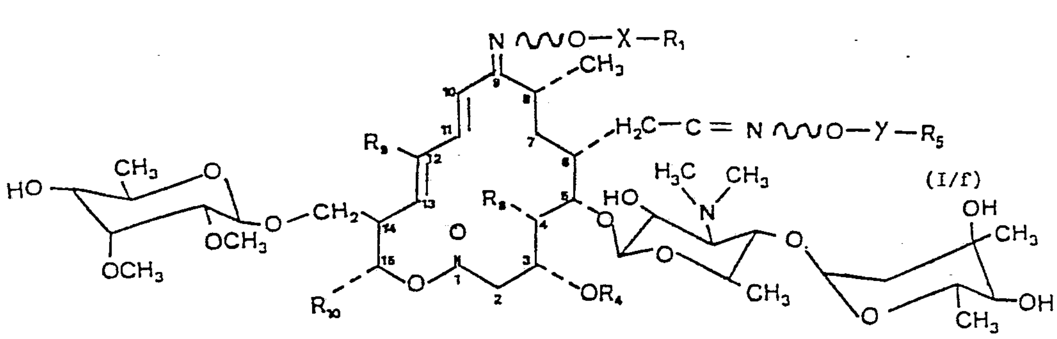
(I/f)

cas particulier de dérivés de formule (I) dans lequel le signe ∿ , X, $R_1$, $R_4$, $R_8$, $R_9$ et $R_{10}$ ont la même

signification que dans la formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable.
- soit séparer en ses isomères puis, si nécessaire, salifier par un acide pharmaceutiquement acceptable.

4/ Procédé de préparation selon la revendication 1, de composés de formule (I), appelés dérivés (I/k) dans lesquelles B représente un atome d'hydrogène, caractérisé en ce que l'on utilise comme matière première un dérivé de formule (II/1) :

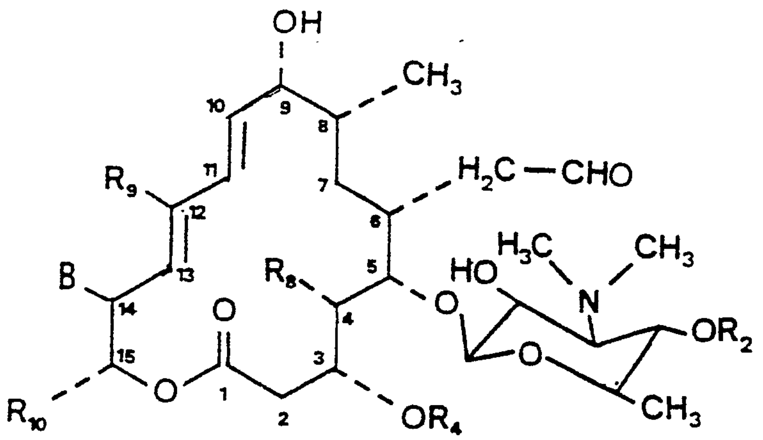

(II/1)

dans laquelle $R_2$ et $R_4$ ont la même définition que dans la formule (I), B et $R_9$ représentent simultanément chacun un atome d'hydrogène, $R_8$ représente un radical alkyloxy inférieur, et $R_{10}$ un groupement alkyle inférieur,

que l'on soumet à un agent d'oxydation comme par exemple le complexe trioxyde de soufre-pyridine en présence d'un agent basique comme une amine organique,

en milieu organique, préférentiellement choisi parmi diméthylsulfoxide, diméthylformamide, pyridine, pour obtenir un dérivé de formule (II/1a) :

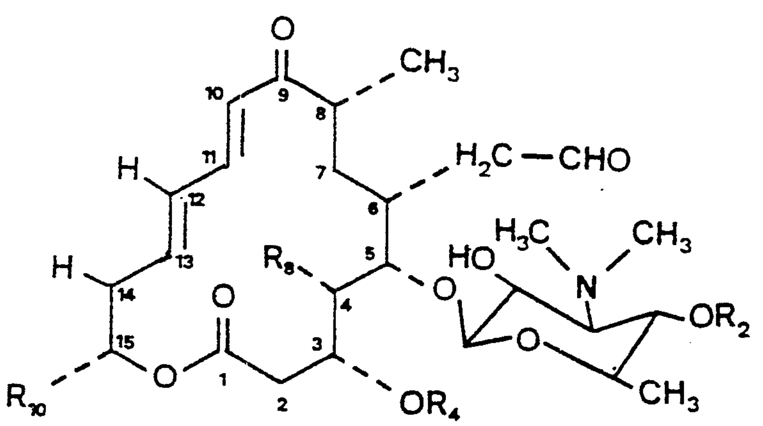

(II/1a)

dans laquelle $R_2$ et R4 ont la même signification que dans la formule (I), et $R_9$ et $R_{10}$ la même signification que dans la formule (II/1),

qui est alors condensé avec un alcool de formule Z - OH, dans lequel Z signifie un groupement alkyle inférieur, en présence d'un agent catalyseur acide comme l'acide paratoluène sulfonique, ou préférentiellement l'acide difluoroacétique lorsque le composé de formule (I/k) que l'on souhaite obtenir a un radical $R_2$ différent de H,

le milieu réactionnel étant ensuite soumis à l'action d'un agent basique, en particulier une amine comme la triéthylamine, pour obtenir, après extraction par un solvant organique, préférentiellement choisi parmi éther diéthylique, chloroforme, chlorure de méthylène et purification par chromatographie sur colonne de silice, un dérivé de formule (III/k) :

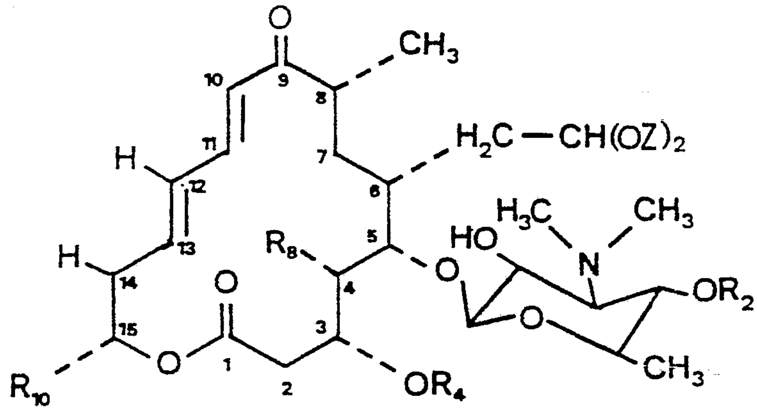

(III/k)

dans lequel $R_2$, $R_4$, $R_8$ et $R_{10}$ ont la même signification que dans la formule du dérivé (II/1a), et Z la même définition que précédemment,
que l'on condense en présence d'une base, comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N$ - O - X - $R_1$  (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V/k) :

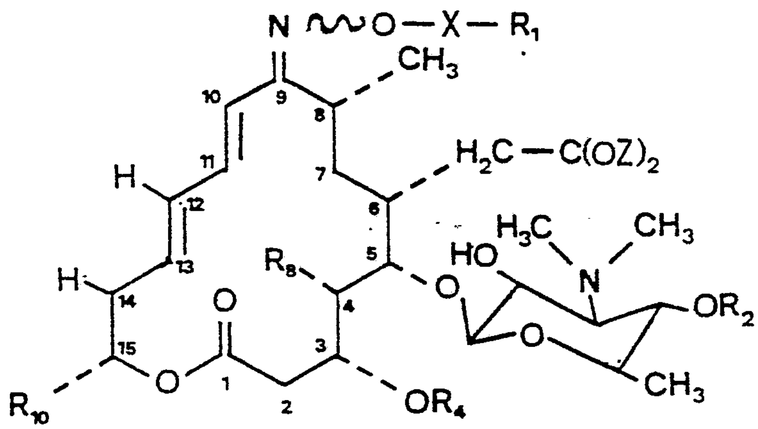

(V/k)

dans lequel le signe ∿ , X, $R_1$, $R_2$, $R_4$, $R_8$ et $R_{10}$ ont la même signification que dans la formule (I) et Z la même définition que précédemment,

- soit avec un sel d'acide fort de l'hydroxylamine pour obtenir un dérivé de formule (V/ak) :

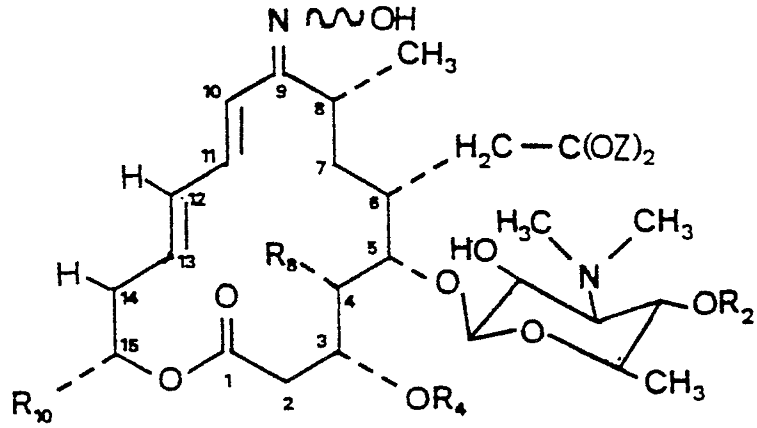

(V/ak)

cas particulier de dérivés de formule (V/k), dans lequel le signe ∿∿ , $R_2$, $R_4$, $R_8$, $R_{10}$ et Z ont la même signification que dans la formule (V/k), X - $R_1$ signifiant ici un atome d'hydrogène,
que l'on traite le cas échéant en présence d'une base, comme par exemple la triéthylamine, ou la pyridine, ou un sel de métal alcalin comme le carbonate de sodium, ou le carbonate de potassium, ou le carbonate de calcium, ou l'hydrogéno carbonate de sodium, ou l'hydrogéno carbonate de potassium, ou un hydrure de métal alcalin tel que l'hydrure de sodium, dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V/k), dans lequel le signe ∿∿ , X, $R_1$, $R_2$, $R_4$, $R_8$ et $R_{10}$ ont la même signification que dans la formule (I) et Z la même signification que dans la formule (III/k),
qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,1 N et 0,5 N, préférentiellement comprise entre 0,02 N et 0,2 N, préférentiellement comprise entre 0,05 N et 0,5 N, préférentiellement comprise 0,75 N et 0,25 N et à température ambiante, ou préférentiellement l'acide difluoroacétique en solution dans un mélange de solvants tel que le mélange acétonitrile - eau (1/1, v/v) lorsque le composé de formule (I) que l'on désire obtenir possède un radical $R_2$ différent de l'atome d'hydrogène,
pour obtenir un composé de formule (I/ak) :

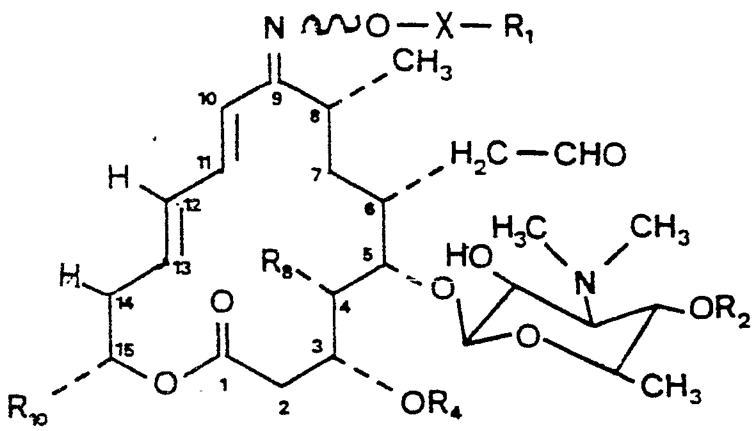

(I/ak)

cas particulier des composés de formule (I) dans laquelle le signe ∿∿ , X, $R_1$, $R_2$, $R_4$, $R_8$ et $R_{10}$ ont la même définition que dans la formule (I), A représentant ici un atome d'oxygène,
lequel, dans le cas où dans la matière première utilisée $R_2$ ne signifie pas un atome d'hydrogène, peut au cours du processus de synthèse, en fonction des conditions opératoires sélectionnées ainsi que des valeurs des groupements X, $R_1$, $R_8$, $R_9$ et $R_{10}$, avoir été partiellement hydrolysé en un dérivé (I/ak) démycarosylé pour lequel $R_2$ = H, les deux dérivés ainsi obtenus (c'est-à-dire celui pour lequel $R_2$ = H

et celui pour lequel $R_2$ correspond à un résidu mycarosylé) étant alors aisément séparés par une technique classique de séparation comme la chromatographie sur colonne de silice,
ce dérivé de formule (I/ak) pouvant alors, le cas échéant, être traité en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium,

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I/k),

- soit par un sel d'acide fort de l'hydroxylamine pour obtenir un produit de formule (I/bk),

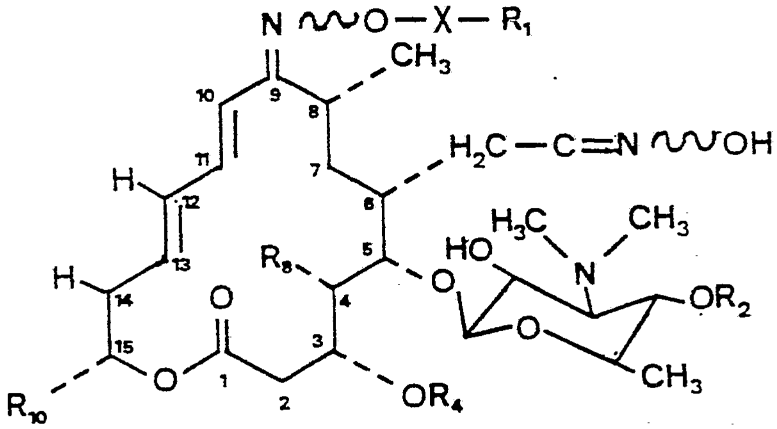

(I/bk)

cas particulier de dérivés de formule (I/k) dans lequel le signe ∿ , X, $R_1$, $R_2$, $R_4$, $R_8$ et $R_{10}$ ont la même signification que dans la formule (I), A représentant ici un groupement N ∿ O - Y - $R_5$ dans lequel Y -$R_5$ représente un atome d'hydrogène,
qui est traité le cas échéant, en présence d'une base, comme par exemple la triéthylamine, ou la pyridine, ou d'un sel de métal alcalin comme le carbonate de sodium ou de carbonate de potassium, ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T' (VIII)**

dans laquelle T' représente un atome d'halogène et $R_5$ et Y ont la même signification que dans la formule (I), à l'exclusion du cas où $R_5$ - Y représentent ensemble un atome d'hydrogène, pour obtenir un composé de formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

5/ Procédé de préparation selon l'une des revendications 1, 3 ou 4 de composés de formule (I) dans lesquels A représente un atome d'oxygène, leurs isomères, (Z) ou (E) isolés ou sous forme de mélange ainsi que leurs sels d'addition à un acide pharmaceutiquement acceptable.

6/ Procédé de préparation selon l'une des revendications 1, 3 ou 4 de composés de formule (I), caractérisés en ce que A représente un groupement de formule N ∿ O - Y - $R_5$ leurs isomères (Z) ou (E) isolés ou sous formes de mélange ainsi que leurs sels d'addition à un acide pharmaceutiquement acceptable.

7/ Procédé de préparation selon l'une quelconque des revendications 1, 3 ou 5 de composés caractérisé en ce que :

- A représente un atome d'oxygène,
- X est un radical alcoyle inférieur linéaire ou ramifié,
- $R_1$ est . un atome d'hydrogène,

. un radical alkyle,
. un atome aryle éventuellement substitué par un groupement nitro,
. un radical alkényloxy,

. un radical alkyloxy éventuellement substitué par un radical alkyloxy inférieur,
. un radical dialkylamino,
. un hétérocycle azoté comprenant éventuellement un autre hétéroatome,
. un radical aryloxy ou aralkyle,
ou bien X - $R_1$ représente un atome d'hydrogène, leurs isomères (Z) ou (E) isolés ou sous forme de mélange ainsi que leurs sels d'addition à un acide pharmaceutiquement acceptable.

8/ Procédé de préparation selon la revendication 1, de composés de formule (I) caractérisés en ce que :

- Y est un radical alkyle inférieur,
- $R_5$ est un atome d'hydrogène ou radical alkyle ou un radical alkyloxy éventuellement substitué par un radical alkyloxy inférieur, ou Y - $R_5$ représente un atome d'hydrogène, leurs isomères (Z) ou (E) isolés ou sous forme de mélange ainsi que leurs sels d'addition à un acide pharmaceutiquement acceptable.

9/ Procédé de préparation selon la revendication 1 de composés de formule (I) dans laquelle B′ et $R_2$ représentent simultanément un atome d'hydrogène, leurs isomères (Z) ou (E) isolés ou sous forme de mélange ainsi que leurs sels d'addition à un acide pharmaceutiquement acceptable.

10/ Procédé de préparation selon l'une des revendications 1 et 4 de composés de formule (I) dans laquelle B et $R_9$ représentent simultanément un atome d'hydrogène, $R_8$ un groupement méthoxy et $R_{10}$ un groupement méthyle, leurs isomères (Z) ou (E) isolés ou sous forme de mélange ainsi que leurs sels d'addition à un acide pharmaceutiquement acceptable.

11/ Procédé de préparation selon l'une des revendications 1 ou 3 de composés de formule (I) dans laquelle B représente un groupe de formule

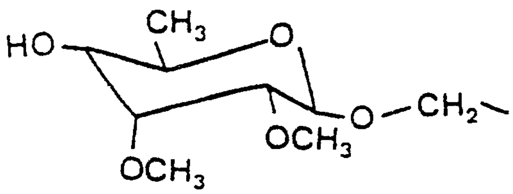

et $R_8$ et $R_9$ représentent simultanément un groupement méthyle et $R_{10}$ un groupement éthyle, leurs isomères (Z) ou (E) isolés ou sous forme de mélange ainsi que leurs sels d'addition à un acide pharmaceutiquement acceptable.

12/ Procédé de préparation selon l'une des revendications 1 ou 3 de composé de formule (I) qui est l'oxime - 9 de la tylosine, ses isomères (Z) et (E) isolés ou sous forme de mélange ainsi que ses sels d'addition à un acide pharmaceutiquement acceptable.

13/ Procédé de préparation selon l'une des revendications 1 ou 3 d'un composé de formule (I) qui est l'O - méthyloxime - 9 de la tylosine, ses isomères (Z) et (E) isolés ou sous forme de mélange ainsi que ses sels d'addition à un acide pharmaceutiquement acceptable.

14/ Procédé de préparation selon l'une des revendications 1 ou 3 d'un composé de formule (I) qui est l'O benzyloxime - 9 de la tylosine, ses isomères (Z) et (E) isolés ou sous forme de mélange ainsi que ses sels d'addition à un acide pharmaceutiquement acceptable.

15/ Procédé de préparation selon l'une des revendications 1 ou 3 d'un composé de formule (I) qui est l'O [(méthoxy - 2 éthoxy) - méthyl] oxime - 9 de la tylosine, ses isomères (Z) et (E) isolés ou sous forme de mélange ainsi que ses sels d'addition à un acide pharmaceutiquement acceptable.

16/ Procédé de préparation selon l'une des revendications 1 ou 4 d'un composé de formule (I) qui est l'hydroxyimino (E + Z) - 9 désoxy - 9 carbomycine B, ses isomères (Z) et (E) isolés ou sous forme de mélange ainsi que ses sels d'addition à un acide pharmaceutiquement acceptable.

17/ Procédé de préparation selon l'une des revendications 1 ou 4 d'un composé de formule (I) qui est la méthoxyimino (E + Z) - 9 désoxy - 9 carbomycine B, ses isomères (Z) et (E) isolés ou sous forme de mélange ainsi que ses sels d'addition à un acide pharmaceutiquement acceptable.

18/ Procédé de préparation selon l'une des revendications 1 ou 4 d'un composé de formule (I) qui est la benzyloxyimino (E + Z) - 9 désoxy -9 carbomycine B, ses isomères (Z) et (E) isolés ou sous forme de mélange ainsi que ses sels d'addition à un acide pharmaceutiquement acceptable.

19/ Procédé de préparation selon l'une des revendications 1 ou 4 d'un composé de formule (I) qui est la paranitrobenzyloxyimino (E + Z) - 9 désoxy - 9 carbomycine B, ses isomères (Z) et (E) isolés ou sous forme de mélange ainsi que ses sels d'addition à un acide pharmaceutiquement acceptable.

20/ Procédé de préparation selon l'une des revendications 1 ou 4 d'un composé de formule (I) qui est 1'0 [(méthoxy - 2 éthoxy) - méthyl] oxyimino - 9 désoxy - 9 carbomycine B, ses isomères (Z) et (E) isolés ou sous forme de mélange ains que ses sels d'addition à un acide pharmaceutiquement acceptable.

**Revendications pour l'Etat contractant suivant: GR**

1/ Procédé de préparation des composés de formule générale :

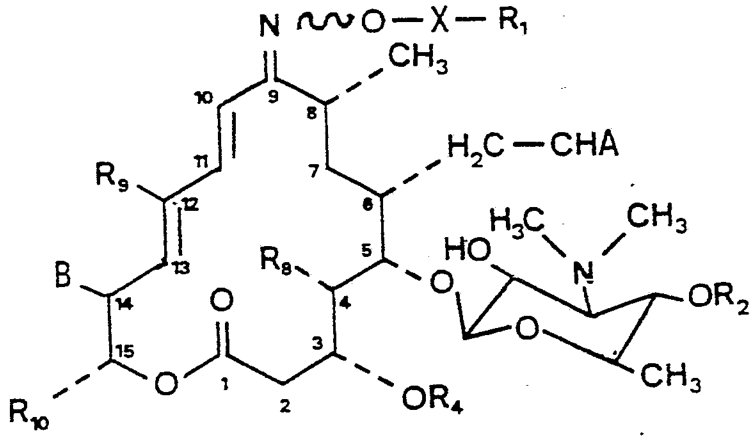

(I)

dans laquelle :

- A représente . soit un atome d'oxygène,

. soit un groupement de formule N ∿∿ O - Y - $R_5$, le signe ∿∿ existant sur le substituant du carbone 9 ainsi que dans la définition de A signifiant que l'une ou l'autre indépendamment des fonctions oxime ou éther d'oxime peut chacune se trouver sous la forme syn ou anti ou sous forme du mélange syn et anti,

- X et Y identiques ou différents représentent soit un radical alkyle comprenant au plus dix atomes de carbone, linéaire ou ramifié, soit un radical alkényle comprenant au plus dix atomes de carbone, linéaire ou ramifié, soit un radical alkynyle comprenant au plus dix atomes de carbone, linéaire ou ramifié, chacun des substituants alkyle, alkényle ou alkynyle pouvant être éventuellement substitué par un ou plusieurs groupements choisis parmi hydroxy, alkyle inférieur linéaire ou ramifié, alkényle inférieur linéaire ou ramifié, alkynyle inférieur linéaire ou ramifié, alkényloxy inférieur linéaire ou ramifié, alkénylthio inférieur linéaire ou ramifié, alkynyloxy inférieur linéaire ou ramifié, alkynylthio inférieur linéaire ou ramifié, fluoro, chloro, bromo, iodo, amino, dialkylamino inférieur,

- $R_1$ et $R_5$ identiques ou différents représentent :

- soit un atome d'hydrogène,

- soit un radical alkyloxy linéaire ou ramifié comprenant au plus dix atomes de carbone, un radical alkylthio linéaire ou ramifié comprenant au plus dix atomes de carbone, un radical alkényloxy linéaire ou ramifié comprenant au plus dix atomes de carbone, un radical alkénylthio linéaire ou ramifié comprenant au plus dix atomes de carbone, un radical alkynyloxy linéaire ou ramifié comprenant au plus dix atomes de carbone, un radical alkynylthio linéaire ou ramifié comprenant au plus dix atomes de carbone, un radical aryle (citons à titre d'exemple phényle, pyridyle, thiényle, furyle, benzothiényle, benzofuryle, indolyle, thiazolyle, oxazolyle, pyrrolyle, imidazolyle, pyrazolyle, isothiazolyle, isoxazolyle, benzimidazolyle), un radical aryloxy, arylthio, aralkyloxy, aralkylthio, chacun des radicaux alkyloxy, alkylthio, alkényloxy, aklénylthio, alkynyloxy, alkynylthio, aryle, aryloxy, arylthio, aralkyloxy, aralkylthio étant éventuellement substitué par un ou plusieurs des radicaux choisis parmi hydroxy, alkyle inférieur, alkényle inférieur, aklynyle inférieur, alkyloxy inférieur, alkényloxy inférieur, alkynyloxy inférieur, alkylthio inférieur, aklénylthio inférieur, alkynylthio inférieur, fluoro, chloro, bromo, iodo, nitro, amino, dialkkylamino inférieur, ou un radical N $R_6$ $R_7$ ci-dessous défini,

- soit un radical

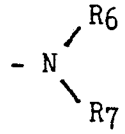

dans lequel $R_6$ et $R_7$ identiques ou différents représentent un atome d'hydrogène, un radical alkyle inférieur, ou alkényle inférieur ou alkynyle inférieur, ou forment avec l'azote un système hétérocyclique saturé ou non, comprenant éventuellement un autre hétéroatome et éventuellement substitué par un atome d'halogène ou un radical alkyle inférieur,

- un atome de chlore, de brome, d'iode ou de fluor,

- un groupement carboxylique libre ou salifié par une base minérale (KOH, NaOH, $Ca(OH)_2$) ou organique (triéthylamine, diéthylamine...) ou estérifié par un alcool aliphatique de formule $R_8OH$ où $R_8$ est un groupement alcoyle inférieur,
- ou X - $R_1$ et Y - $R_5$ indépendamment l'un de l'autre représentent chacun un atome d'hydrogène,
- $R_2$ représente . soit un atome d'hydrogène,
. soit un radical de formule :

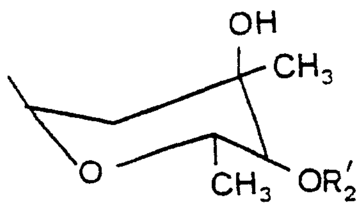

dans lequel $R'_2$ représente un atome d'hydrogène, un radical alkyle ou un radical acyle inférieur, linéaire ou ramifié,
- B représente . soit un atome d'hydrogène,
. soit un radical de formule : - $CH_2$ - O - B′, où B′ représente :
. soit un atome d'hydrogène,
. soit un radical de formule :

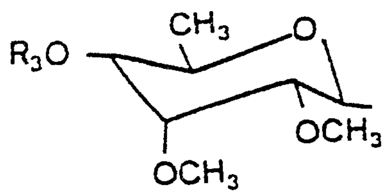

dans lequel $R_3$ représente un atome d'hydrogène ou un radical alkyle inférieur linéaire ou ramifié,
- $R_4$ représente . soit un atome d'hydrogène,
. soit un radical alkyle inférieur linéaire ou ramifié,
. soit un radical acyle inférieur linéaire ou ramifié,
- $R_8$ représente . soit un radical alkyle inférieur préférentiellement méthyle lorsque B représente un groupement $CH_2$ - O - B′,
. soit un radical alkyloxy inférieur préférentiellement méthoxy lorsque B représente un atome d'hydrogène,
- $R_9$ représente . soit un atome d'hydrogène lorsque B représente un atome d'hydrogène,
. soit un radical alkyle inférieur préférentiellement méthyle lorsque B représente un radical $CH_2$ - O - B′,
- $R_{10}$ représente un radical alkyle inférieur,
le terme inférieur concernant les radicaux alkyle inférieur, alkyloxy inférieur, alkylthio inférieur, alkényle inférieur, alkényloxy inférieur, alkénylthio inférieur, alkynyle inférieur, alkynyloxy inférieur, aklynylthio inférieur, signifiant qu'il s'agit de groupements comprenant entre 1 et 6 atomes de carbone,
et leurs sels d'addition avec un acide,
caractérisé en ce que l'on utilise comme matière première :
- soit lorsque B représente un atome d'hydrogène, un dérivé de formule (II/1) :

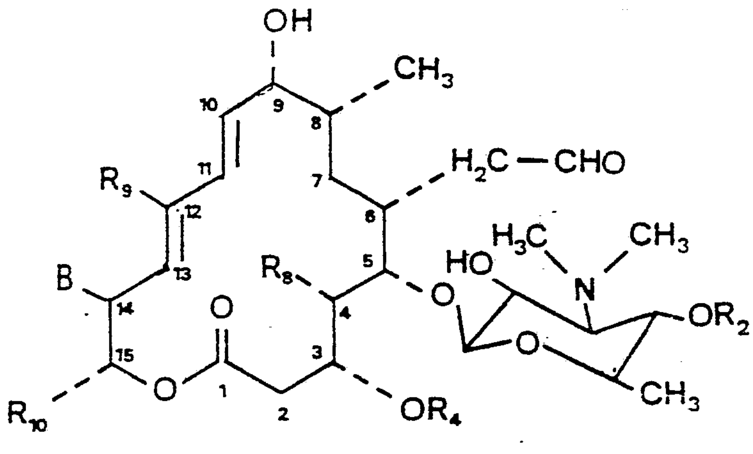

(II/1)

dans laquelle $R_2$, $R_4$, $R_8$ et $R_{10}$ ont la même signification que dans la formule (I), B et $R_9$ représentent simultanément un atome d'hydrogène,
que l'on soumet à un agent d'oxydation comme par exemple le complexe trioxyde de soufre-pyridine en présence d'un agent basique comme une amine organique,
en milieu organique, préférentiellement choisi parmi diméthylsulfoxide, diméthylformamide, pyridine,
pour obtenir un dérivé de formule (II/1a) :

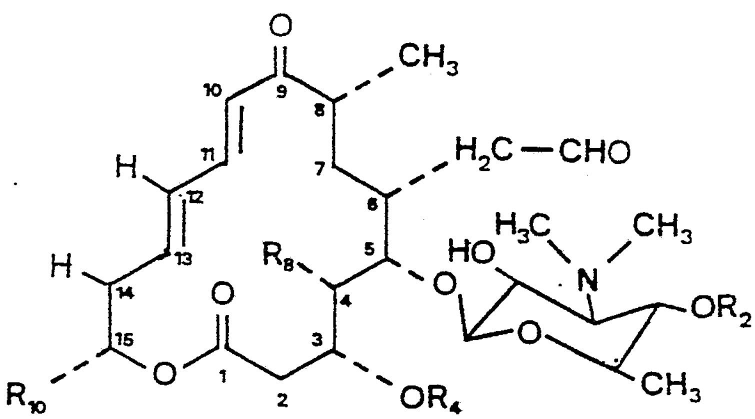

(II/1a)

dans laquelle $R_2$, $R_4$, $R_8$ et $R_{10}$ ont la même signification que dans la formule (I),
soit, lorsque dans le composé de formule (I) que l'on souhaite obtenir, B et $R_9$ ne représentent pas simultanément chacun un atome d'hydrogène,
un dérivé de formule (II/2) :

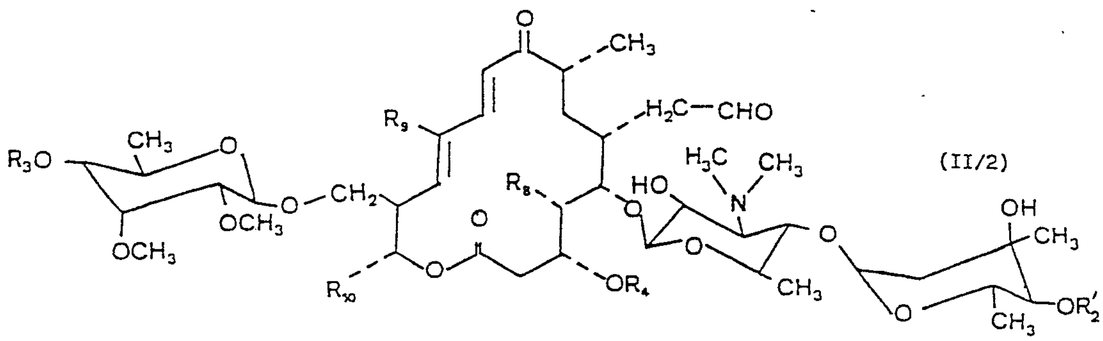

(II/2)

dans laquelle R'2, R3, R4 et R10 ont la même dfinition que dans la formule (I), et R8 et R9 signifient un radical alkyle inférieur préférentiellement méthyle,
qui, lorsque dans le composé de formule (I) que l'on souhaite obtenir B' et R2 représentent simultanément un atome d'hydrogène est tout d'abord soumis à l'action de l'acide chlorhydrique dilué de normalité comprise entr 0,05 et 0,4 préférentiellement comprise entre 0,1 et 0,30, préférentiellement comprise entre 0,15 et 0,25 à température ambiante pour conduire après lavage par un solvant organique approprié, alcalinisation et extraction par un solvant organique approprié à un dérivé de formule (II/2a) :

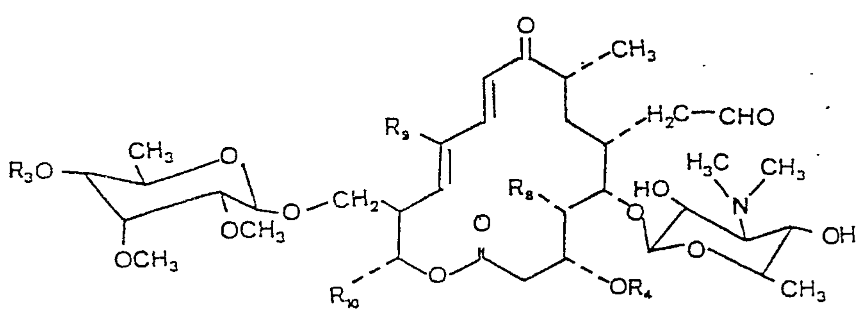

(II/2a)

dans laquelle R3, R4 et R10 ont la même signification que dans la formule (I), et R8 et R9 la même signification que dans la formule (II/2),
que l'on fait réagir avec l'acide chlorhydrique dilué de normalité comprise entre 0,25 et 0,75 ; préférentiellement entre 0,3 et 0,7 ; préférentiellement entre 0,4 et 0,6 ; à une température comprise préférentiellement entre 30 et 100 °C ; préférentiellement comprise entre 50 et 90 °C ; préférentiellement entre 70 et 80 °C, pour conduire, après lavage par un solvant organique approprié, alcalinisation et extraction par un solvant organique approprié à un dérivé de formule (II/2b) :

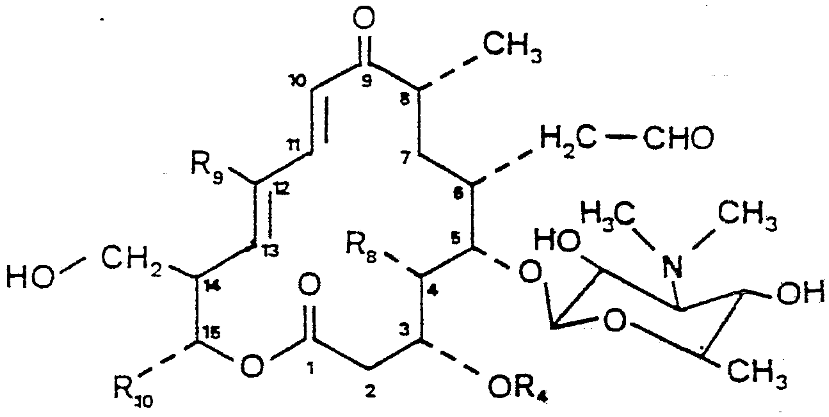

(II/2b)

dans laquelle $R_4$ et $R_{10}$ ont la même signification que dans la formule (I), et $R_8$ et $R_9$ la même signification que dans la formule (II/2)
que l'on purifie éventuellement par chromatographie sur colonne de silice,
le dérivé alors choisi de formule générale (II/1a), (II/2), ou (II/2b) selon le composé de formule (I) que l'on souhaite obtenir, étant alors condensé avec un alcool de formule Z - OH, dans lequel Z signifie un groupement alkoyle inférieur, en présence d'un agent catalyseur acide comme l'acide paratoluène sulfonique ou préférentiellement l'acide difluoroacétique lorsque le composé de formule (I) que l'on souhaite obtenir a un radical $R_2$ différent H,
le milieu réactionnel étant ensuite soumis à l'action d'un agent basique, en particulier une amine comme la triéthylamine, pour obtenir, après extraction par un solvant organique préférentiellement choisi parmi éther diéthylique, chloroforme, chlorure de méthylène et purification par chromatographie sur colonne de silice, un dérivé de formule (III)

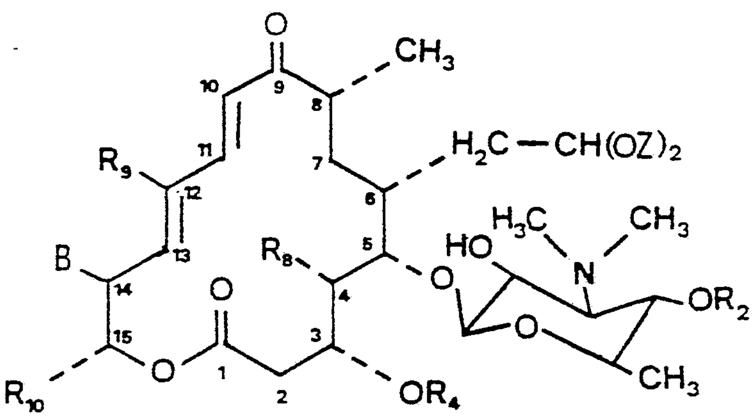

(III)

dans lequel B, $R_2$, $R_4$, $R_8$, $R_9$ et $R_{10}$ ont la même signification que dans la formule de dérivé choisi (II/1a), (II/2) ou (II/2b) et Z la même définition que précédemment,
que l'on condense en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium,

- soit avec un dérivé de formule (IV) :

**$H_2N - O - X - R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V) :

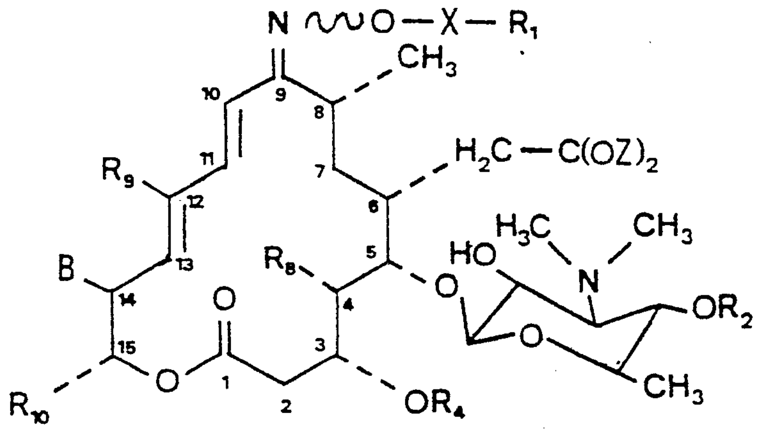

(V)

dans lequel le signe ∿ , X, $R_1$, $R_2$, $R_4$, $R_8$, $R_9$, $R_{10}$ et B ont la même signification que dans la formule (I) et Z la même définition que précédemment,

- soit avec un sel d'acide fort de l'hydroxylamine pour obtenir un dérivé de formule (V/a) :

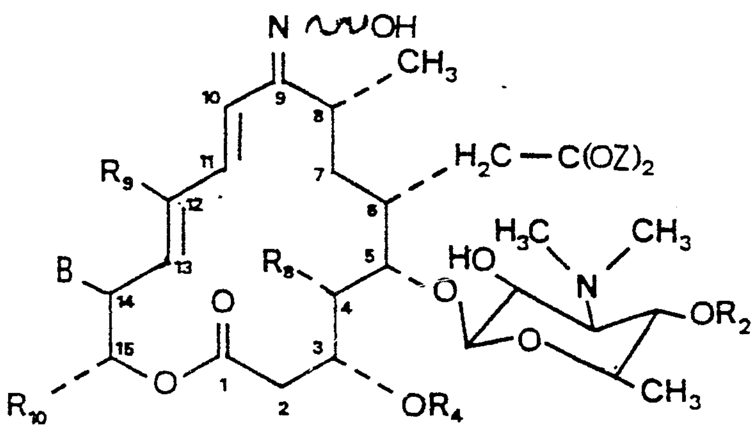

(V/a)

cas particulier de dérivés de formule (V), dans lequel le signe ∿ , B, $R_2$, $R_4$, $R_8$, $R_9$, $R_{10}$ et Z ont la même signification que dans la formule (V), X - R1 signifiant ici un atome d'hydrogène,
que l'on traite le cas échéant en présence d'une base, comme par exemple la triéthylamine ou la pyridine ou un sel de métal alcalin comme le carbonate de sodium ou le carbonate de potassium ou le carbonate de calcium ou l'hydrogéno carbonate de sodium ou l'hydrogéno carbonate de potassium, ou un hydrure de métal alcalin tel que l'hydrure de sodium dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V), dans lequel le signe ∿ , X, $R_1$, $R_2$, $R_4$, $R_8$, $R_9$, $R_{10}$ et B ont la même signification que dans la formule (I) et Z la même signification que dans la formule (III),
qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,01N et 0,5N, préférentiellement comprise entre 0,02N et 0,2N, préférentiellement comprise entre 0,05N et 0,5N, préférentiellement comprise entre 0,075N et 0,25N à température ambiante,
ou préférentiellement l'acide difluoroacétique en solution dans un mélange de solvants tel que le mélange acétonitrile - eau (1/1, v/v) lorsque le composé de formule (I) que le composé de formule (I) que l'on désire obtenir, possède un radical $R_2$ différent de l'atome d'hydrogène,
pour obtenir un composé de formule (I/a), cas particulier des composés de formule (I) :

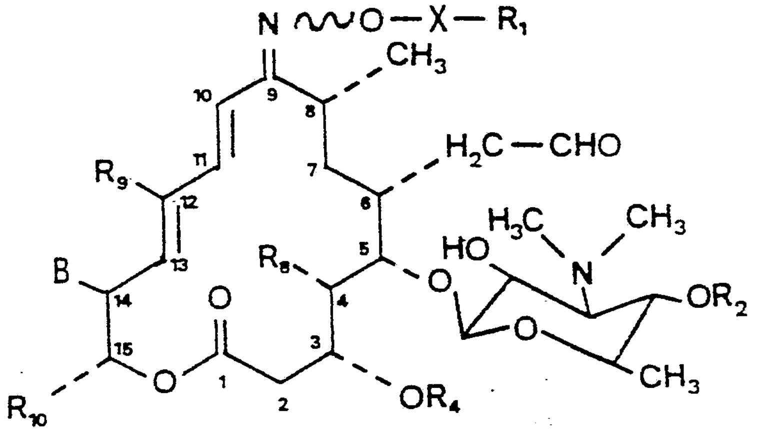

(I/a)

dans lequel le signe ∿ , X, $R_1$, $R_2$, $R_4$, $R_8$, $R_9$, $R_{10}$ et B ont la même définition que dans la formule (I), A représentant ici un atome d'oxygène,

lequel, dans le cas où dans la matière première utiisée $R_2$ ne signifie pas un atome d'hydrogène, peut au cours du processus de synthèse, en fonction des conditions opératoires sélectionnées ainsi que des valeurs des groupements X, $R_1$, $R_8$, $R_9$, $R_{10}$ et B, avoir été partiellement hydrolysé en un dérivé (I/a) démycarosylé pour lequel $R_2$ = H, les deux dérivés ainsi obtenus (c'est-à-dire celui pour lequel $R_2$ = H et celui pour lequel $R_2$ correspond à un résidu mycarosé) étant alors aisément séparés par une technique classique de séparation comme la chromatographie sur colonne de silice,

ce dérivé de formule (I/a) peut, le cas échéant, être traiter en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium,

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I),

- soit par un sel d'acide fort de l'hydroxylamine pour obtenir un produit de formule (I/b),

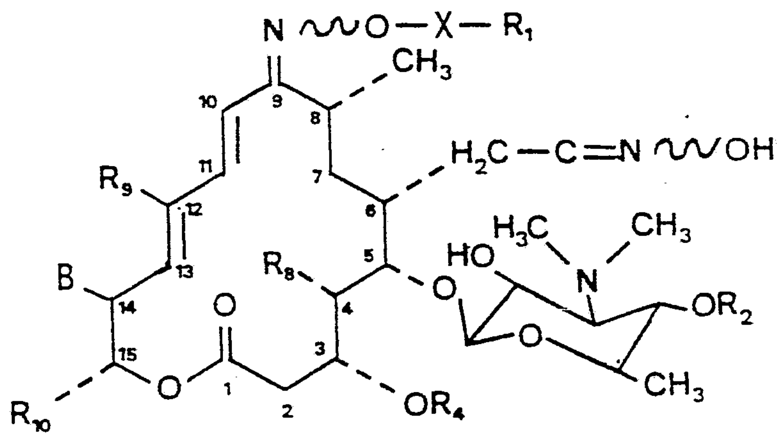

(I/b)

cas particulier de dérivés de formule (I) dans lequel le signe ∿ , X, $R_1$, $R_2$, $R_4$, $R_8$, $R_9$, $R_{10}$ et B ont la même signification que dans la formule (I), A représentant ici un groupement N ∿ O - Y - $R_5$ dans lequel Y - $R_5$ représente un atome d'hydrogène,

qui est traité le cas échéant, en présence d'une base, comme par exemple la triéthylamine, ou la pyridine, ou d'un sel de métal alcalin comme le carbonate de sodium ou de carbonate de potassium, ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T' (VIII)**

dans laquelle T' représente un atome d'halogène et $R_5$ et Y ont la même signification que dans la formule

(I), à l'exclusion du cas où $R_5$ - Y représentent ensemble un atome d'hydrogène, pour obtenir un composé de formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

2/ Procédé de préparation selon la revendication 1 des dérivés de formule (I) dans lesquels Y - $R_5$ a la même signification que X - $R_1$ ou dérivés (IX), caractérisé en ce que l'on condense un dérivé de formule (II/1a) ou (II/2) ou (II/2a) ou (II/2b) selon la formule du composé de formule (I) que l'on souhaite obtenir :

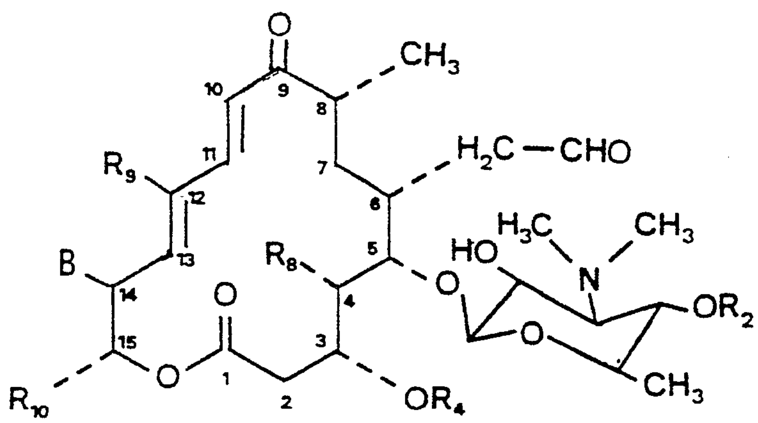

(II)

dans laquelle $R_2$, $R_4$, $R_8$, $R_9$, $R_{10}$ et B ont la même définition que celle correspondant à chacune des formules (II/1a), (II/2) ou (II/2b), dans la formule (I),en présence d'une base comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N$ - O - X - $R_1$ (IV)**

dans lequel X - $R_1$ a la même signification que dans la formule (I) à l'exception du cas où X - $R_1$ représente un atome d'hydrogène.

pour obtenir un produit de formule (IX) :

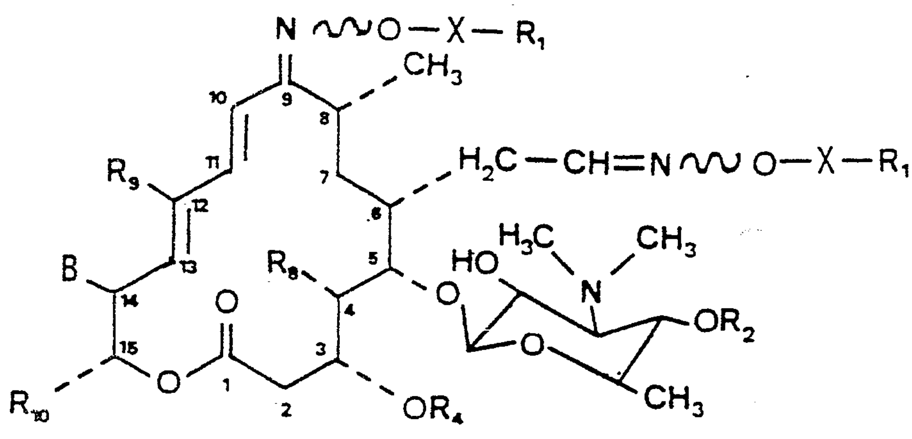

(IX)

cas particulier des dérivés de formule (I) dans laquelle le signe ∿ , X, $R_1$, $R_2$, $R_4$, $R_8$, $R_9$, $R_{10}$ et B ont la même signification que dans la formule (I),

- soit avec un sel d'acide fort de l'hydroxylamine pour obtenir un dérivé de formule (IX/a) :

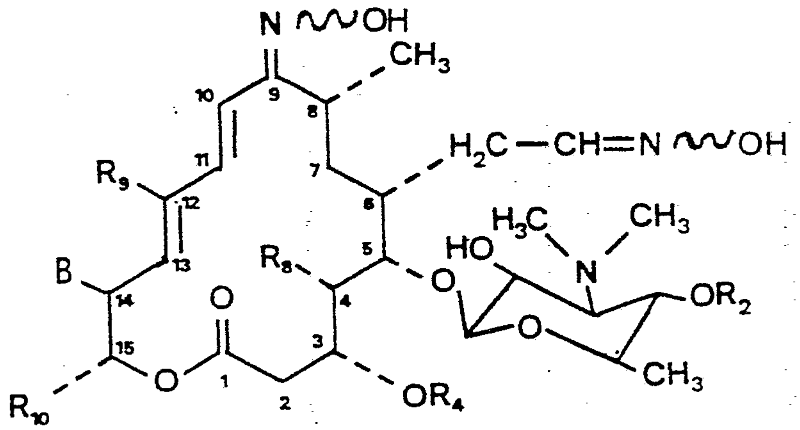

(IX/a)

cas particulier de dérivés de formule (I) et (IX), dans lequel le signe ~ , $R_2$, $R_4$, $R_8$, $R_9$, $R_{10}$ et B ont la même signification que dans la formule I, X - $R_1$ et Y - $R_5$ représentant ici ensemble un atome d'hydrogène, que l'on traite éventuellement en présence d'une base, comme par exemple la triéthylamine, la pyridine ou un sel de métal alcalin comme le carbonate de sodium ou le carbonate de potassium ou l'hydrogéno carbonate de sodium ou l'hydrogéno carbonate de potassium, ou un hydrure de métal alcalin tel que l'hydrure de sodium, dans un solvant préférentiellement choisi parmi l'acétone ou le diméthyl formamide, le dioxanne, l'acétonitrile, le tétrahydrofuranne, l'éther diéthylique ou diisopropylique, par un dérivé de formule (X) :

**$R_1$ - X - T″ (X)**

dans lequel T″ représente un atome d'halogène et $R_1$ et X ont la même signification que dans la formule (I), à l'exclusion du cas où $R_1$ et X forment ensemble un atome d'hydrogène, pour obtenir un produit de formule (IX) cas particulier des dérivés de formule (I), dans lequel le signe , $R_1$, $R_2$, $R_4$, $R_8$, $R_9$, $R_{10}$ B et X ont la même signification que dans la formule (I), que l'on purifie éventuellement par chromatographie sur colonne de silice à l'aide d'un mélange solvant approprié comme par exemple un mélange chlorure de méthylène / méthanol, et que l'on peut, si on le désire :

- soit salifier par un acide pharmaceutiquement acceptable,
- soit séparer en ses isomères puis, si nécessaire, salifier par un acide pharmaceutiquement acceptable.

3/ Procédé de préparation selon la revendication 1 de composés de formule (I/f), cas particulier des dérivés de formule (I) dans lesquels :

B représente un groupement - $CH_2$ - O - B′ où B′ représente un groupement de formule :

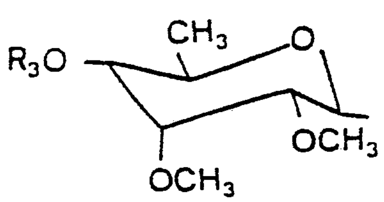

$R_2$ un groupe de formule :

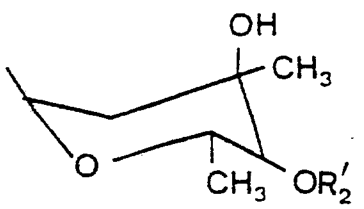

cas particulier où $R'_2$ et $R_3$ représentent un atome d'hydrogène, et A, X, R, $R_4$, $R_8$, $R_9$ et $R_{10}$ ont la même signification que dans la formule (I),

caractérisé en ce que l'on fait agir sur la tylosine un alcool aliphatique inférieur Z - OH, préférentiellement le méthanol, en présence d'un agent acide comme par exemple l'acide difluoroacétique, le milieu réactionnel étant ensuite soumis à l'action d'un agent basique, en particulier une amine organique appropriée (préférentiellement le dichlorométhane) et purification par chromatographie sur colonne de silice un dérivé de formule (III/b) :

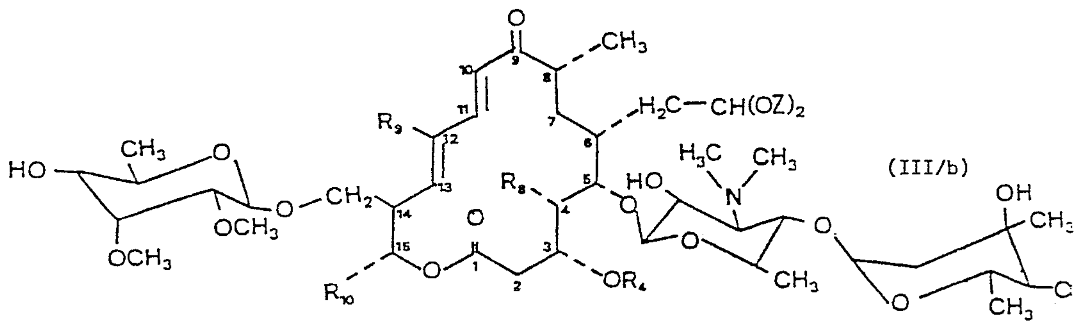

(III/b)

dans lequel $R_4$, $R_8$, $R_9$, et $R_{10}$ ont la même signification que dans la formule (I), et Z représente un groupement alkyl inférieur, que l'on condense en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium,

- soit avec un dérivé de formule (IV) :

**$H_2N$ - O - X - $R_1$ (IV)**

dans lequel X et $R_1$ ont la même signification que dans la formule (I) à l'exclusion du cas où X - $R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V/b) :

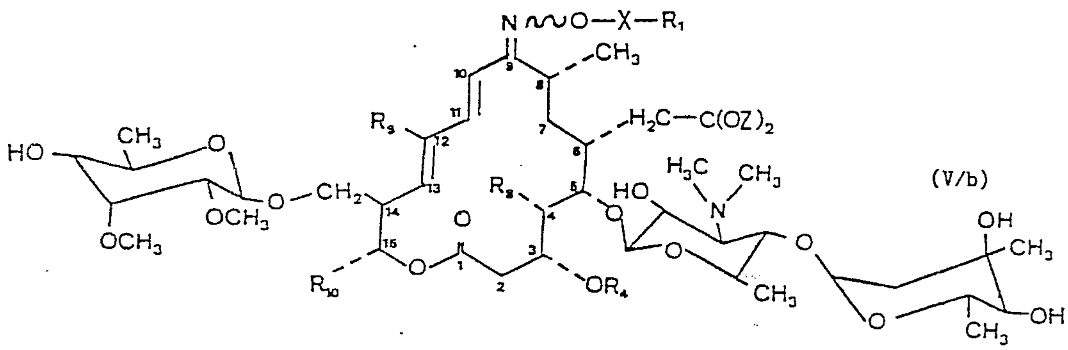

(V/b)

dans lequel le signe ∿∿ , X, $R_1$, $R_4$, $R_8$, $R_9$ et $R_{10}$ ont la même signification que dans la formule (I) et Z la même définition que précédemment,

- soit avec un sel d'acide fort de l'hydroxylamine pour obtenir un dérivé de formule (V/c) :

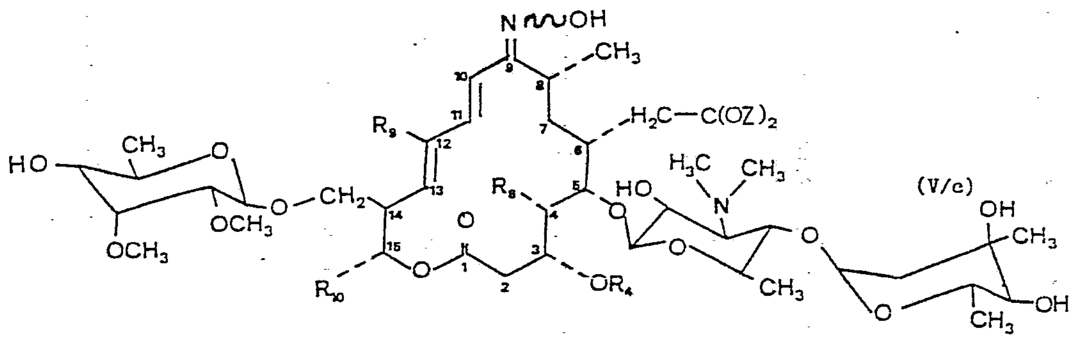

(V/c)

cas particulier de dérivés de formule (V/b), dans lequel le signe ∿ , $R_4$, $R_8$, $R_9$, $R_{10}$ et Z ont la même signification que dans la formule (V/b), X - R1 signifiant ici un atome d'hydrogène,

que l'on traite le cas échéant en présence d'un base, comme par exemple la triéthylamine ou la pyridine ou un sel de métal alcalin comme le carbonate de sodium ou le carbonate de potassium ou le carbonate de calcium ou l'hydrogéno carbonate de sodium ou l'hydrogéno carbonate de potassium, ou un hydrure de métal alcalin tel que l'hydrure de sodium dans un solvant préférentiellement choisi parmi la pyridine, l'acétone ou le diméthylformamide, le dioxanne, l'acétonitrile, le tétrahydrofuranne, l'éther diéthlique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V), dans lequel le signe ∿ , X, $R_1$, $R_4$, $R_8$, $R_9$ et $R_{10}$ ont la même signification que dans la formule (I) et Z la même signification que dans la formule (III/b),

qui, quel que soit le procédé selon lequel il a été obtenu, est soumis à un procédé de déprotection habituel de la fonction aldéhydique portée par le carbone hors cycle en β du carbone 6, comme par exemple, l'action d'une solution acide préférentiellement l'acide difluoroacétique en solution dans un mélange de solvants tel que le mélange acétonitrile - eau (1/1, v/v) à température ambiante pour obtenir un composé de formule (I/g), cas particulier des composés de formule (I) :

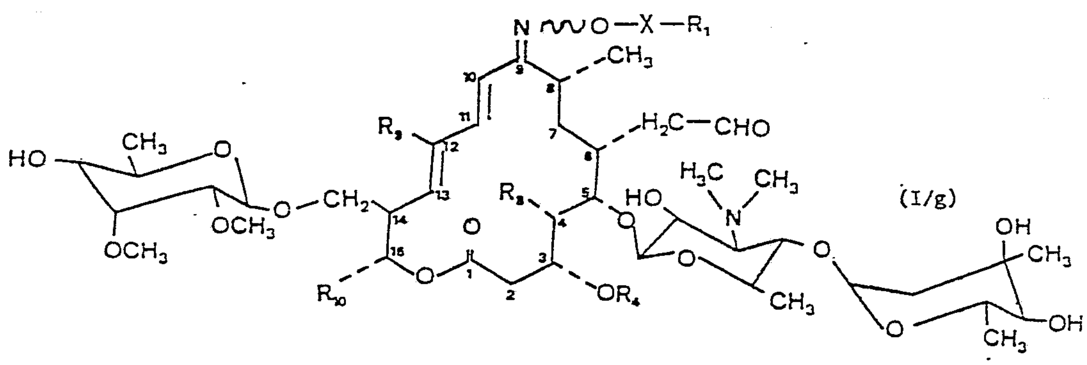

(I/g)

dans lequel le signe ∿ , X, $R_1$, $R_4$, $R_8$, $R_9$ et $R_{10}$ ont la même définition que dans la formule (I), A représentant ici un atome d'oxygène,

lequel, puisque $R_2$ ne signifie pas un atome d'hydorgène, peut au cours du processus de synthèse, en fonction des conditions opératoires sélectionnées ainsi que des valeurs des groupements X et $R_1$, avoir été partiellement hydrolysé en un dérivé (I/g) démycarosylé pour lequel $R_2$ = H, les deux dérivés ainsi obtenus (c'est-à-dire celui pour lequel $R_2$ = H et celui pour lequel $R_2$ correspond à un résidu mycarosé) étant alors aisément sépaés par une technique classique de séparation classique de séparation comme la

chromatographie sur colonne de silice,
dérivé de formule (I/g),
que l'on peut, le cas échéant, traiter en présence d'un base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I),

- soit par un sel d'acide fort de l'hydroxylamine pour obtenir un produit de formule (I/h) :

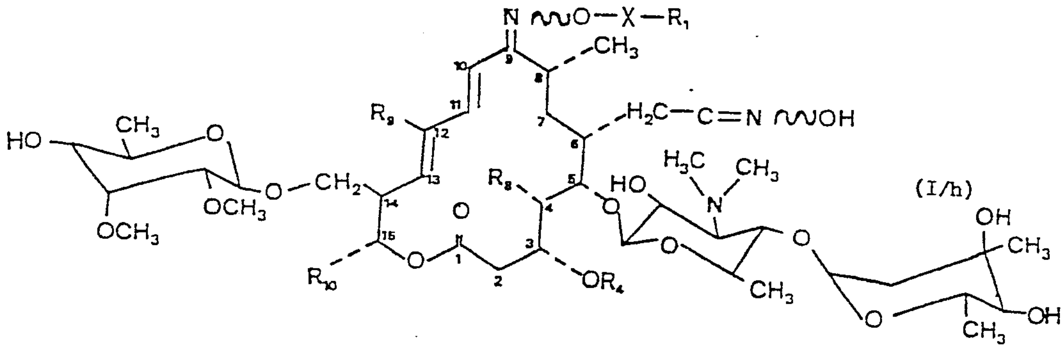

(I/h)

cas particulier de dérivés de formule (I) dans lequel le signe ∿ , X, $R_1$, $R_4$, $R_8$, $R_9$ et $R_{10}$ ont la même signification que dans la formule (I), A représentant ici un groupement N ∿ O - Y - $R_5$ dans lequel Y -$R_5$ représente un atome d'hydrogène,
qui est traité le cas échéant, en présence d'une base, comme par exemple la triéthylamine ou la pyridine ou d'un sel de métal alcalin comme le carbonate de sodium ou de carbonate de potassium ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T' (VIII)**

dans laquelle T' représente un atome d'halogène et $R_5$ et Y ont la même signification que dans la formule (I), à l'exclusion du cas où $R_5$ - Y représentent ensemble un atome d'hydrogène, pour obtenir un composé de formule (I/f),

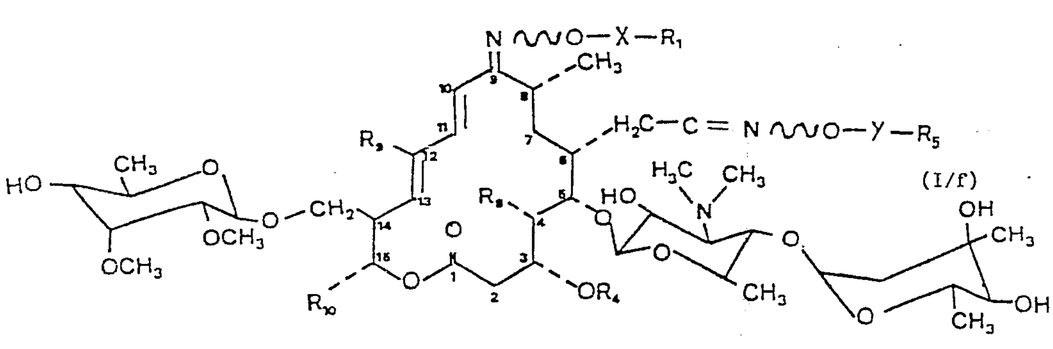

(I/f)

cas particulier de dérivés de formule (I) dans lequel le signe ∿ , X, $R_1$, $R_4$, $R_8$, $R_9$ et $R_{10}$ ont la même

signification que dans la formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

4/ Procédé de préparation selon la revendication 1, de composés de formule (I), appelés dérivés (I/k) dans lesquelles B représente un atome d'hydrogène, caractérisé en ce que l'on utilise comme matière première un dérivé de formule (II/1):

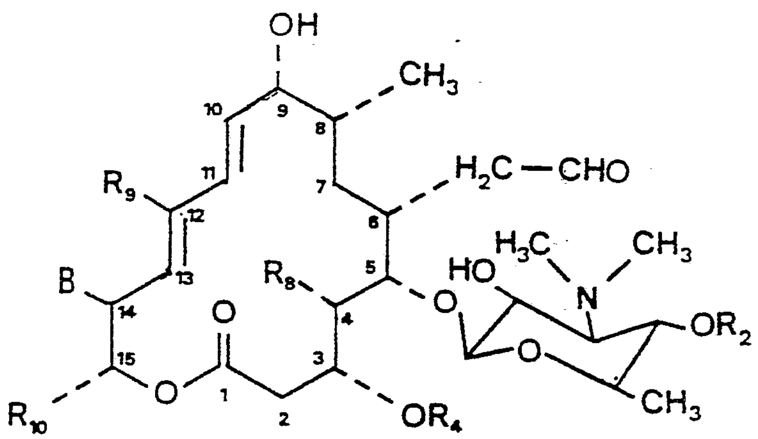

(II/1)

dans laquelle $R_2$ et $R_4$ ont la même définition que dans la formule (I), B et $R_9$ représentent simultanément chacun un atome d'hydrogène, $R_8$ représente un radical alkyloxy inférieur, et $R_{10}$ un groupement alkyle inférieur,
que l'on soumet à un agent d'oxydation comme par exemple le complexe trioxyde de soufre-pyridine en présence d'un agent basique comme une amine organique,
en milieu organique, préférentiellement choisi parmi diméthylsulfoxide, diméthylformamide, pyridine,
pour obtenir un dérivé de formule (II/1a) :

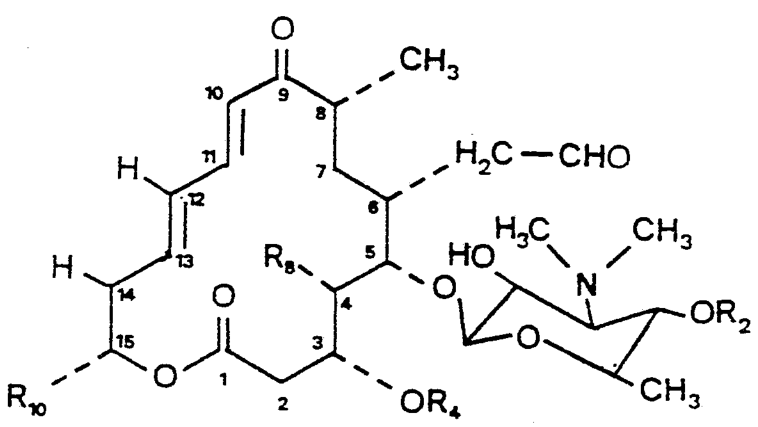

(II/1a)

dans laquelle $R_2$ et $R_4$ ont la même signification que dans la formule (I), et $R_9$ et $R_{10}$ la même signification que dans la formule (II/1),
qui est alors condensé avec un alcool de formule Z - OH, dans lequel Z signifie un groupement alkyle inférieur, en présence d'un agent catalyseur acide comme l'acide paratoluène sulfonique, ou préférentiellement l'acide difluoroacétique lorsque le composé de formule (I/k) que l'on souhaite obtenir a un radical $R_2$ différent de H,
le milieu réactionnel étant ensuite soumis à l'action d'un agent basique, en particulier une amine comme la triéthylamine, pour obtenir, après extraction par un solvant organique, préférentiellement choisi parmi éther diéthylique, chloroforme, chlorure de méthylène et purification par chromatographie sur colonne de silice, un dérivé de formule (III/k) :

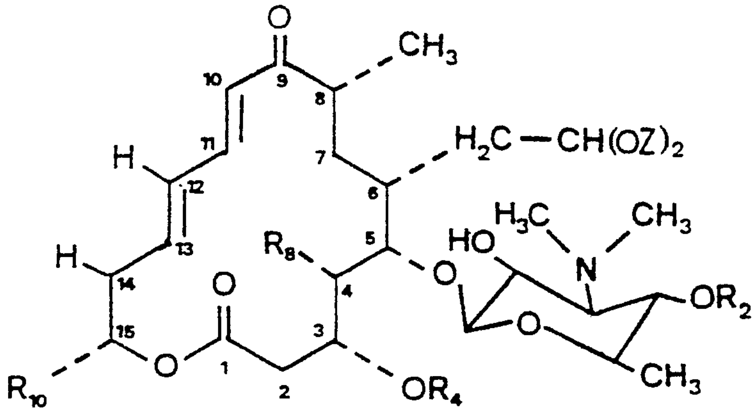

(III/k)

dans lequel $R_2$, $R_4$, $R_8$ et $R_{10}$ ont la même siginification que dans la formule du dérivé (II/1a), et Z la même définition que précédemment,

que l'on condense en présence d'une base, comme par exemple la pyridine, la triéthylamine, ou un sel de métal alcalin tel que l'acétate de sodium, ou le carbonate acide de sodium, ou le carbonate acide de potassium, ou le carbonate de sodium, ou le carbonate de potassium, ou le carbonate de calcium,

- soit avec un dérivé de formule (IV) :

**$H_2N - O - X - R_1$ (IV)**

dans lequel X et $R_1$ ont la même signification que dans la formule (I) à l'exclusion du cas où $X - R_1$ représente un atome d'hydrogène ou bien préférentiellement avec un sel d'acide fort (chlorhydrate, bromhydrate...) d'un tel produit pour obtenir après purification éventuelle par chromatographie sur colonne de silice un dérivé de formule (V/k) :

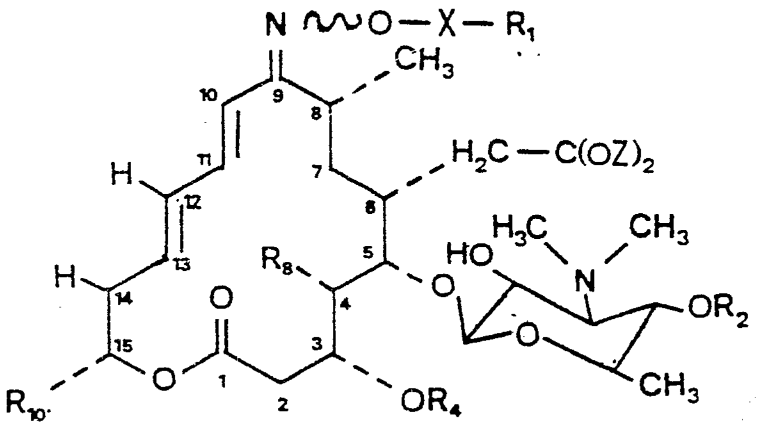

(V/k)

dans lequel le signe ∿∿ , X, $R_1$, $R_2$, $R_4$, $R_8$, et $R_{10}$ ont la même signification que dans la formule (I) et Z la même définition que précédemment,

- soit avec un sel d'acide fort de l'hydroxylamine pour obtenir un dérivé de formule (V/ak) :

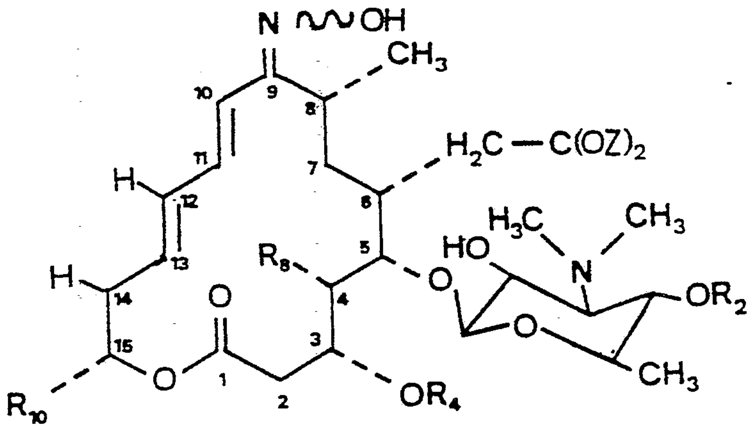

(V/ak)

cas particulier de dérivés de formule (V/k), dans lequel le signe ∿ , $R_2$, $R_4$, $R_8$, $R_{10}$ et Z ont la même signification que dans la formule (V/k), X - $R_1$ signifiant ici un atome d'hydrogène,
que l'on traite le cas échéant en présence d'un base, comme par exemple la triéthylamine, ou la pyridine, ou un sel de métal alcalin comme le carbonate de sodium, ou le carbonate de potassium, ou le carbonate de calcium, ou l'hydrogéno carbonate de sodium, ou l'hydrogéno carbonate de potassium, ou un hydrure de métal alcalin tel que l'hydrure de sodium, dans un solvant préférentiellement choisi parmi la pyridine, l'acétone, le diméthylformamide, le dioxanne, l'acétonitrile, le tétrahydrofuranne, l'éther diéthylique ou diisopropylique, par un dérivé de formule (VI) :

**$R_1$ - X - T (VI)**

dans lequel T représente un atome d'halogène et $R_1$ et X ont la même signification que dans la formule (I), à l'exclusion du cas où $R_1$ et X forment ensemble un atome d'hydrogène pour obtenir après purification éventuelle par chromatographie sur colonne de silice un produit de formule (V/k), dans lequel le signe ∿ , X, $R_1$, $R_2$, $R_4$, $R_8$ et $R_{10}$ ont la même signification que dans la formule (I) et Z la même signification que dans la formule (III/k),
qui, quel que soit le procédé selon lequel il a été obtenu, est soumis à un procédé de déprotection habituel de la fonction aldéhydique portée par le carbone hors cycle en β du carbone 6, comme, par exemple, l'action d'une solution aqueuse d'acide chlorhydrique de normalité comprise entre 0,1 N et 0,5 N, préférentiellement comprise entre 0,02 N et 0,2 N, préférentiellement comprise entre 0,05 N et 0,5 N, préférentiellement comprise 0,75 N et 0,25 N et à température ambiante, ou préférentiellement l'acide difluoroacétique en solution dans un mélange de solvants tel que le mélange acétonitrile - eau (1/1, v/v) lorsque le composé de formule (I) que l'on désire obtenir possède un radical $R_2$ différent de l'atome d'hydrogène,
pour obtenir un composé de formule (I/ak) :

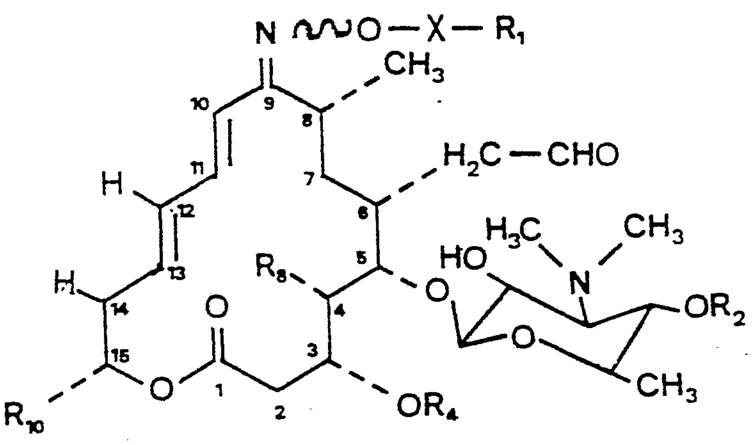

(I/ak)

cas particulier des composés de formule (I) dans laquelle le signe ∿ , X, $R_1$, $R_2$, $R_4$, $R_8$, et $R_{10}$ ont la même définition que dans la formule (I), A représentant ici un atome d'oxygène,
lequel, dans le cas où dans la matière première utilisée $R_2$ ne signifie pas un atome d'hydrogène, peut au cours du processus de synthèse, en fonction des conditions opératoires sélectionnées ainsi que des valeurs des groupements X, $R_1$, $R_8$, $R_9$ et $R_{10}$, avoir été partiellement hydrolysé en un dérivé (I/ak) démycarosylé pour lequel $R_2$ = H, les deux dérivés ainsi obtenus (c'est-à-dire celui pour lequel $R_2$ = H

et celui pour lequel $R_2$ correspond à un résidu mycarosylé) étant alors aisément séparés par une technique classique de séparation comme la chromatographie sur colonne de silice,
ce dérivé de formule (I/ak) pouvant alors, le cas échéant, être traité en présence d'une base comme par exemple la pyridine, la triéthylamine ou un sel de métal alcalin tel que l'acétate de sodium, le carbonate acide de potassium ou le carbonate acide de sodium, le carbonate de sodium ou le carbonate de potassium ou le carbonate de calcium,

- soit par un dérivé de formule (VII) :

**$H_2N$ - O - Y - $R_5$ (VII)**

dans laquelle Y et $R_5$ ont la même définition que dans la formule (I), à l'exclusion du cas où Y - $R_5$ représente un atome d'hydrogène, ou par un sel d'acide fort d'un tel produit, pour obtenir après purification éventuelle par chromatographie sur colonne de silice un dérivé de formule (I/k),

- soit par un sel d'acide fort de l'hydroxylamine pour obtenir un produit de formule (I/bk),

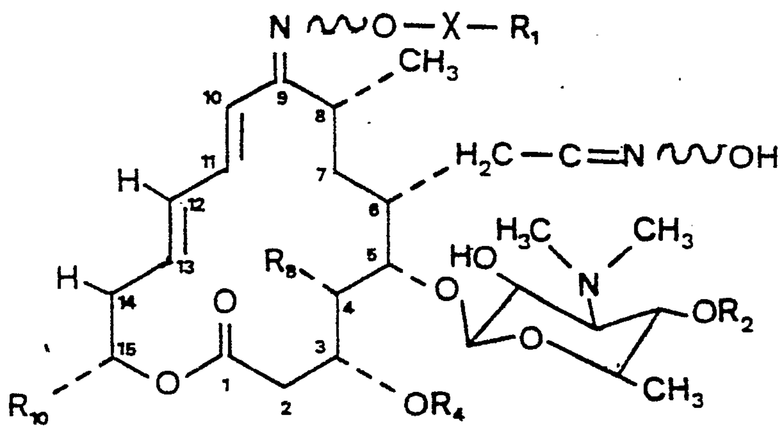

(I/bk)

cas particulier de dérivés de formule (I/k) dans lequel le signe ∿∿ , X, $R_1$, $R_2$, $R_4$, $R_8$, et $R_{10}$ ont la même signification que dans la formule (I), A représentant ici un groupement N ∿∿ O - Y - $R_5$ dans lequel Y -$R_5$ représente un atome d'hydrogène,
qui est traité le cas échéant, en présence d'une base, comme par exemple la triéthylamine, ou la pyridine, ou d'un sel de métal alcalin comme le carbonate de sodium ou de carbonate de potassium, ou l'hydrogéno carbonate de sodium ou l'hydrogéno carbonate de potassium, ou bien un hydrure de métal alcalin tel que l'hydrure de sodium dans un solvant préférentiellement choisi parmi l'acétone, le diméthyl formamide, le dioxanne, l'acétonitrile, le tétrahydrofuranne, l'éther diéthylique ou diisopropylique par un produit de formule (VIII) :

**$R_5$ - Y - T' (VIII)**

dans laquelle T' représente un atome d'halogène et $R_5$ et Y ont la même signification que dans la formule (I), à l'exclusion du cas où $R_5$ - Y représentent ensemble un atome d'hydrogène, pour obtenir un composé de formule (I),

- que l'on purifie éventuellement par chromatographie sur colonne de silice à l'aide d'un mélange solvant approprié comme par exemple un mélange chlorure de méthylène / méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

5/ Procédé de préparation selon l'une des revendications 1, 3 ou 4 de composés de formule (I) dans lesquels A représente un atome d'oxygène, leurs isomères, (Z) ou (E) isolés ou sous forme de mélange ainsi que leurs sels d'addition à un acide pharmaceutiquement acceptable.

6/ Procédé de préparation selon l'une des revendications 1, 3 ou 4 de composés de formule (I), caractérisés en ce que A représente un groupement de formule N ∿∿ O - Y - $R_5$ leurs isomères (Z) ou (E) isolés ou sous forme de mélange ainsi que leurs sels d'addition à un acide pharmaceutiquement acceptable.

7/ Procédé de préparation selon l'une quelconque des revendications 1, 3 ou 5 de composés caractérisé en ce que :

- A représente un atome d'oxygène,
- X est un radical alcoyle inférieur linéaire ou ramifié,
- $R_1$ est . un atome d'hydrogène,

. un radical alkyle,
. un atome aryle éventuellement substitué par un groupement nitro,
. un radical alkényloxy,

. un radical alkyloxy éventuellement substitué par un radical alkyloxy inférieur,
. un radical dialkylamino,
. un hétérocycle azoté comprenant éventuellement un autre hétéroatome,
. un radical aryloxy ou aralkyle,
ou bien X - $R_1$ représente un atome d'hydrogène, leurs isomères (Z) ou (E) isolés ou sous forme de mélange ainsi que leurs sels d'addition à un acide pharmaceutiquement acceptable.

8/ Procédé de préparation selon la revendication 1, de composés de formule (I) caractérisés en ce que :

- Y est un radical alkyle inférieur,
- $R_5$ est un atome d'hydrogène ou radical aklyle ou un radical aklyloxy éventuellement substitué par un radical alkyloxy inférieur, ou Y - $R_5$ représente un atome d'hydrogène, leurs isomères (Z) ou (E) isolés ou sous forme de mélange ainsi que leurs sels d'addition à un acide pharmaceutiquement acceptable.

9/ Procédé de préparation selon la revendication 1 de composés de formule (I) dans laquelle B′ et $R_2$ représentent simultanément un atome d'hydrogène, leurs isomères (Z) ou (E) isolés ou sous forme de mélange ainsi que leurs sels d'addition à un acide pharmaceutiquement acceptable.

10/ Procédé de préparation selon l'une des revendications 1 et 4 de composés de formule (I) dans laquelle B et $R_9$ représentent simultanément un atome d'hydrogène, $R_8$ un groupement méthoxy et $R_{10}$ un groupement méthyle, leurs isomères (Z) ou (E) isolés ou sous forme de mélange ainsi que leurs sels d'addition à un acide pharmaceutiquement acceptable.

11/ Procédé de préparation selon l'une des revendications 1 ou 3 de composés de formule (I) dans laquelle B représente un groupe de formule

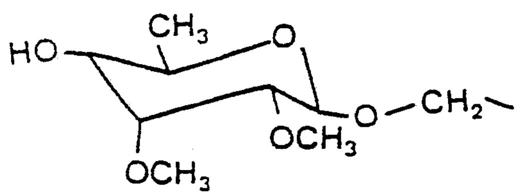

et $R_8$ et $R_9$ représentent simultanément un groupement méthyle et $R_{10}$ un groupement éthyle, leurs isomères (Z) ou (E) isolés ou sous forme de mélange ainsi que leurs sels d'addition à un acide pharmaceutiquement acceptable.

12/ Procédé de préparation selon l'une des revendications 1 ou 3 de composé de formule (I) qui est l'oxime - 9 de la tylosine, ses isomères (Z) et (E) isolés ou sous forme de mélange ainsi que ses sels d'addition à un acide pharmaceutiquement acceptable.

13/ Procédé de préparation selon l'une des revendications 1 ou 3 d'un composé de formule (I) qui est l'O - méthyloxime - 9 de la tylosine, ses isomères (Z) et (E) isolés ou sous forme de mélange ainsi que ses sels d'addition à un acide pharmaceutiquement acceptable.

14/ Procédé de préparation selon l'une des revendications 1 ou 3 d'un composé de formule (I) qui est l'O benzyloxime - 9 de la tylosine, ses isomères (Z) et (E) isolés ou sous forme de mélange ainsi que ses sels d'addition à un acide pharmaceutiquement acceptable.

15/ Procédé de préparation selon l'une des revendications 1 ou 3 d'un composé de formule (I) qui est l'O [(méthoxy - 2 éthoxy) - méthyl] oxime - 9 de la tylosine, ses isomères (Z) et (E) isolés ou sous forme de mélange ainsi que ses sels d'addition à un acide pharmaceutiquement acceptable.

16/ Procédé de préparation selon l'une des revendications 1 ou 4 d'un composé de formule (I) qui est l'hydroxyimino (E + Z) - 9 désoxy - 9 carbomycine B, ses isomères (Z) et (E) isolés ou sous forme de mélange ainsi que ses sels d'addition à un acide pharmaceutiquement acceptable.

17/ Procédé de préparation selon l'une des revendications 1 ou 4 d'un composé de formule (I) qui est la méthoxyimino (E + Z) - 9 désoxy - 9 carbomycine B, ses isomères (Z) et (E) isolés ou sous forme de mélange ainsi que ses sels d'addition à un acide pharmaceutiquement acceptable.

18/ Procédé de préparation selon l'une des revendications 1 ou 4 d'un composé de formule (I) qui est la benzyloxyimino (E + Z) - 9 désoxy -9 carbomycine B, ses isomères (Z) et (E) isolés ou sous forme de mélange ainsi que ses sels d'addition à un acide pharmaceutiquement acceptable.

19/ Procédé de préparation selon l'une des revendications 1 ou 4 d'un composé de formule (I) qui est la paranitrobenzyloxyimino (E + Z) - 9 désoxy - 9 carbomycine B, ses isomères (Z) et (E) isolés ou sous forme de mélange ainsi que ses sels d'addition à un acide pharmaceutiquement acceptable.

20/ Procédé de préparation selon l'une des revendications 1 ou 4 d'un composé de formule (I) qui est l'O [(méthoxy - 2 éthoxy) - méthyl] oxyimino - 9 désoxy - 9 carbomycine B, ses isomères (Z) et (E) isolés ou sous forme de mélange ains que ses sels d'addition à un acide pharmaceutiquement acceptable.